# EUROPEAN PATENT SPECIFICATION

(11) **EP 2 412 805 B1**
(45) Date of publication and mention of the grant of the patent: **30.04.2014**
(21) Application number: 11008446.4
(22) Date of filing: 08.06.2007
(51) Int. Cl.: C12N 15/00, A61K 31/7088, A61P 35/00, A61P 43/00, C07K 16/32, C12N 15/09, C12Q 1/68, G01N 33/15, G01N 33/50, G01N 33/574

(54) **Gene involved in immortalization of human cancer cell and use thereof**
An der Verewigung menschlicher Krebszellen beteiligtes Gen und Verwendung dafür
Gène impliqué dans l'immortalisation des cellules cancéreuses humaines et utilisation associée

(30) Priority: 09.06.2006 JP 2006161350
(43) Date of publication of application: 01.02.2012
(62) Divisional of application: 07767074.3
(73) Proprietor: Kabushiki Kaisha Yakult Honsha, Tokyo 105-8660 (JP)
(72) Inventor: Nishiyama, Masahiko, Hiroshima-shi, Hiroshima 7348553 (JP); Hiyama, Keiko, Hiroshima-shi, Hiroshima 7348553 (JP); Tanimoto, Keiji, Hiroshima-shi, Hiroshima 7348553 (JP); Masuko, Norio, Hanno-shi, Saitama 3578527 (JP)
(74) Representative: Müller-Boré & Partner Patentanwälte PartG mbB

(56) References cited:
- WO-A2-02/10436
- US-A1- 2005 250 137
- US-A1- 2005 255 487
- US-A1- 2006 074 565
- M. MONTE: "The Cell Cycle-regulated Protein Human GTSE-1 Controls DNA Damage-induced Apoptosis by Affecting p53 Function", JOURNAL OF BIOLOGICAL CHEMISTRY, vol. 278, no. 32, 1 January 2003 (2003-01-01), pages 30356-30364, XP55015251, ISSN: 0021-9258, DOI: 10.1074/jbc.M302902200
- COLLAVIN<A 1> L ET AL: "Cell-cycle regulation of the p53-inducible gene B99", FEBS LETTERS, ELSEVIER, AMSTERDAM, NL, vol. 481, no. 1, 8 September 2000 (2000-09-08), pages 57-62, XP004337556, ISSN: 0014-5793, DOI: 10.1016/S0014-5793(00)01969-4
- HIYAMA KEIKO ET AL: "Differentially expressed genes involved in cellular immortalization in cooperation with telomerase", PROCEEDINGS OF THE ANNUAL MEETING OF THE AMERICAN ASSOCIATION FOR CANCER RESEARCH; 95TH ANNUAL MEETING OF THE AMERICAN-ASSOCIATION-FOR- CANCER-RESEARCH; ORLANDO, FL, USA; MARCH 27 -31, 2004, AMERICAN ASSOCIATION FOR CANCER RESEARCH, US, vol. 45, 1 March 2004 (2004-03-01), page 223, XP008123952, ISSN: 0197-016X
- HIYAMA K ET AL: "Differentially expressed genes throughout the cellular immortalization processes are quite different between normal human fibroblasts and endothelial cells", INTERNATIONAL JOURNAL OF ONCOLOGY, DEMETRIOS A. SPANDIDOS ED. & PUB, GR, vol. 27, 1 January 2005 (2005-01-01), pages 87-95, XP003020161, ISSN: 1019-6439
- HIYAMA KEIKO ET AL: "Differential regulation of cellular proliferation between transformed and non-transformed immortal cells.", PROCEEDINGS OF THE AMERICAN ASSOCIATION FOR CANCER RESEARCH ANNUAL MEETING, vol. 47, April 2006 (2006-04), page 1175, XP8146739, & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- LANGFORD L A ET AL: "Telomerase activity in human brain tumours.", LANCET 11 NOV 1995 LNKD- PUBMED:7475720, vol. 346, no. 8985, 11 November 1995 (1995-11-11), pages 1267-1268, XP002587745, ISSN: 0140-6736
- WHITFIELD MICHAEL L ET AL: "Common markers of proliferation", NATURE REVIEWS. CANCER, NATUR PUBLISHING GROUP, LONDON, GB LNKD- DOI:10.1038/NRC1802, vol. 6, no. 2, 1 February 2006 (2006-02-01), pages 99-106, XP002494575, ISSN: 1474-175X

### SEQUENCE LISTING

<110> HIROSHIMA UNIVERSITY
<110> TAIHO PHARMACEUTICAL CO., LTD.
<120> GENE INVOLVED IN IMMORTALIZATION OF HUMAN CANCER CELL AND USE THEREOF
<130> P07-68
<150> JP 2006-161350
   <151> 2006-06-09
<160> 76
<170> PatentIn version 3.1
<210> 1
   <211> 890
   <212> DNA
   <213> Homo sapiens
<400> 1
   ggcggaccga agaacgcagg aagggggccg gggggacccg cccccggccg gccgcagcca      60
   tgaactccaa cgtggagaac ctacccccgc acatcatccg cctggtgtac aaggaggtga     120
   cgacactgac cgcagaccca cccgatggca tcaaggtctt tcccaacgag gaggacctca     180
   ccgacctcca ggtcaccatc gagggccctg aggggacccc atatgctgga ggtctgttcc     240
   gcatgaaact cctgctgggg aaggacttcc ctgcctcccc acccaagggc tacttcctga     300
   ccaagatctt ccacccgaac gtgggcgcca atggcgagat ctgcgtcaac gtgctcaaga     360
   gggactggac ggctgagctg ggcatccgac acgtactgct gaccatcaag tgcctgctga     420
   tccaccctaa ccccgagtct gcactcaacg aggaggcggg ccgcctgctc ttggagaact     480
   acgaggagta tgcggctcgg gcccgtctgc tcacagagat ccacgggggc gccggcgggc     540
   ccagcggcag ggccgaagcc ggtcgggccc tggccagtgg cactgaagct tcctccaccg     600
   accctggggc cccagggggc ccgggagggg ctgagggtcc catggccaag aagcatgctg     660
   gcgagcgcga taagaagctg gcggccaaga aaaagacgga caagaagcgg gcgctgcggg     720
   cgctgcggcg gctgtagtgg gctctcttcc tccttccacc gtgaccccaa cctctcctgt     780
   cccctccctc caactctgtc tctaagttat ttaaattatg gctggggtcg gggagggtac     840
   agggggcact gggacctgga tttgtttttc taaataaagt tggaaaagca              890
<210> 2
   <211> 1427
   <212> DNA
   <213> Homo sapiens
<400> 3
<210> 3
   <211> 2120
   <212> DNA
   <213> Homo sapiens
<400> 3
   ggagggcaga gagaaccgca acacctggtg ccgggtcggg tcgtttccgg ggctttcagt      60
   ggccggaagt cgcggcgcct gtactgactc taggaagggc tggagttgtt ttgaatgggc     120
   gcccgtaaga gaggtgggca agtacgtgtt acagacggcc acgccgccct ttaggcggtc     180
   aaggtggggc gagcagacgt tcgcccccct gcagtcggcc gggtcactac ccaagagcct     240
   ttggaggcgg aagcatggaa cggtctgcaa acgttcccga gcgggcctct gcggctctgg     300
   cgggcgtttc gaacttgggc gccgggcaca cgcccagtcc cgagagcgct gagggttccc     360
   ttagcgtcgc cctcaccccg gccaacccgc ggggcgccag agtcctggcc ctttaaacgc     420
   cgcgcgtgcc tcggcgtctt cgtttcgcgc gcccgcccgc ggcgccggcg gagcgaacat     480
   ggacccggct gcgcgggtgg tgcgggcgct gtggcctggt gggtgcgcct tggcctggag     540
   gctgggaggc cgcccccagc cgctgctacc cacgcagagc cgggctggct tcgcgggggc     600
   ggcgggcggc ccgagccccg tggctgcagc tcgtaagggg agcccgcggc tgctgggagc     660
   tgcggcgctg gccctggggg gagccctggg gctgtaccac acggcgcggt ggcacctgcg     720
   cgcccaggac ctccacgcag agcgctcagc cgcgcagctc tccctgtcca gccgcctgca     780
   gctgaccctg taccagtaca agacgtgtcc cttctgcagc aaggtccgag ccttcctcga     840
   cttccatgcc ctgccctacc aggtggtgga ggtgaaccct gtgcgcaggg ctgagatcaa     900
   gttctcctcc tacagaaagg tgcccatcct ggtggcccag gaaggagaaa gctcgcaaca     960
   actaaatgac tcctctgtca tcatcagcgc cctcaagacc tacctggtgt cggggcagcc    1020
   cctggaagag atcatcacct actacccagc catgaaggct gtgaacgagc agggcaagga    1080
   ggtgaccgag ttcggcaata agtactggct catgctcaac gagaaggagg cccagcaagt    1140
   gtatggtggg aaggaggcca ggacggagga gatgaagtgg cggcagtggg cggacgactg    1200
   gctggtgcac ctgatctccc ccaatgtgta ccgcacgccc accgaggctc tggcgtcctt    1260
   tgactacatt gtccgcgagg gcaagttcgg agccgtggag ggtgccgtgg ccaagtacat    1320
   gggtgcagcg gccatgtacc tcatcagcaa gcgactcaag agcaggcacc gcctccagga    1380
   caacgtgcgc gaggacctct atgaggctgc tgacaagtgg gtggctgctg tgggcaagga    1440
   ccggcccttc atggggggcc agaagccgaa tctcgctgat ttggcggtgt atggcgtgct    1500
   gcgtgtgatg gaggggctgg atgcattcga tgacctgatg cagcacacgc acatccagcc    1560
   ctggtacctg cgggtggaga gggccatcac cgaggcctcc ccagcgcact gaatgtcccc    1620
   gcgcagagca gagggaaggc agcggaagac gccagctgcc agggcctggg gccactgggc    1680
   cagcgcctgg cgatactggt tgggggcagg atcattctgc cccttgtcca cgcaccccca    1740
   ccagccctct cgcttctaac acagggcacc tgctggggct cagggatgtt agggacgagt    1800
   tccagccctg ccactgccct ggggcgaccc ctccctgtcc ctgcctccct gctctgccgc    1860
   ccctcttcct ggaccctcag tggctgtccc atggctacat cctgtgggtg ggggccctcg    1920
   acaggacagc aggacggttt gttttcagtg gaatcccatc cctgggttcc cctggttccc    1980
   actcttccca agcctcccgg gactgggaca tgtttgcaat aaaggaaagg tttgtggcgc    2040
   ctgtcatggc aggcatctca tggaaaaaaa aaaaaaaaaa aaaaaaaaaa aaaaaaaaaa    2100
   aaaaaaaaaa aaaaaaaaaa                                                2120
<210> 4
   <211> 1766
   <212> DNA
   <213> Homo sapiens
<400> 4
   acccgatggc tattcgagct ctcgatctcg gagactggag cgggccattc agaggcgcgg       60
   ggccgggccc ggcggacgct tgttgttgtc cggccggggg aggcggaggt cgctcgctcg      120
   ctcgctcggc tcgctgactc gccggagcgc tctgtggcgg tcggcggcag gtcggtcgcg      180
   agagcgggct ctgtggaagg gggcgaggct atgtcgcggt ggcagcccgg atgggccggc      240
   agggccggga gtaacgggac gtcgccgcgg agcttcttcc cccggataca gtgcggcccg      300
   agcggaggcc gcggcgccgc cctccgatct tgaagagccc gcgctgcgcg gagcccgccc      360
   ccgcctgcgc accggcaccg acgcggagcg acccagccca gccagacccg gcccggcgcg      420
   gcctgatcta acccagccag gcaggcaata ctagcccctc tggagcacgg agctccttcc      480
   ccaaagacat gaagctattg gagaactcga gctttgaagc catcaactca cagctgactg      540
   tggagaccgg agatgcccac atcattggca ggattgagag ctactcatgt aagatggcag      600
   gagacgacaa acacatgttc aagcagttct gccaggaggg ccagccccac gtgctggagg      660
   cactttctcc accccagact tcaggactga gccccagcag actcagcaaa agccaaggcg      720
   gtgaggagga gggccccctc agtgacaagt gcagccgcaa gaccctcttc tacctgattg      780
   ccacgctcaa tgagtccttc aggcctgact atgacttcag cacagcccgc agccatgagt      840
   tcagccggga gcccagcctt agctgggtgg tgaatgcagt caactgcagt ctgttctcag      900
   ctgtgcggga ggacttcaag gatctgaaac cacagctgtg gaacgcggtg gacgaggaga      960
   tctgcctggc tgaatgtgac atctacagct ataacccaga cttggactca gatcccttcg     1020
   gggaggatgg tagcctctgg tccttcaact acttcttcta caacaagcgg ctcaagcgaa     1080
   tcgtcttctt tagctgccgt tccatcagtg gctccaccta cacaccctca gaggcaggca     1140
   acgagctgga catggagctg ggggaggagg aggtggagga agaaagcaga agcaggggca      1200
   gtggggccga ggagaccagc accatggagg aggacagggt cccagtgatc tgtatttgat     1260
   gaggaggagc cgaggcccca gcttcatcca gcttcaacca atgcctggac ctgtccacct     1320
   gagaggcccc tggggcctcc ccagctgctg gccagaccct ggcgctgcca cagtcctggc     1380
   actgcccaag gccatacctg cctagccctt tggctccatc ctgtggatgc ccactcaccc     1440
   ctcagactcc tgctgcccat gctgtggccg gacttgtcag cagggggcct ggtgggagga      1500
   gcgactgccc tgcccaaatg aactgccaca gcagggacag ctggaccgca gagtttattt     1560
   ttgtatttct actgggcctg cacactccag cccaaagggt ctgtggccgg aggccccacg     1620
   agcaggcccc agcagtcacc ggctctggtc ttgggccggc cccggtgccc acctgtaccc     1680
   ccacctcgcc catttggccg cgtgcactga gtgtcacttt gctgcagctc gtttctttcc     1740
   aataaaagtt tctgtgactt agaaaa                                          1766
<210> 5
   <211> 1584
   <212> DNA
   <213> Homo sapiens
<400> 5
   gaacatgacg gcgccctggg tggccctcgc cctcctctgg ggatcgctgt ggcccggctc       60
   tgggcgtggg gaggctgaga cacgggagtg catctactac aacgccaact gggagctgga      120
   gcgcaccaac cagagcggcc tggagcgctg cgaaggcgag caggacaagc ggctgcactg      180
   ctacgcctcc tgggccaaca gctctggcac catcgagctc gtgaagaagg gctgctggct      240
   agatgacttc aactgctacg ataggcagga gtgtgtggcc actgaggaga acccccaggt      300
   gtacttctgc tgctgtgaag gcaacttctg caacgagcgc ttcactcatt tgccagaggc      360
   tgggggcccg gaagtcacgt acgagccacc cccgacagcc cccaccctgc tcacggtgct      420
   ggcctactca ctgctgccca tcgggggcct ttccctcatc gtcctgctgg ccttttggat      480
   gtaccggcat cgcaagcccc cctacggtca tgtggacatc catgaggacc ctgggcctcc      540
   accaccatcc cctctggtgg gcctgaagcc actgcagctg ctggagatca aggctcgggg      600
   gcgctttggc tgtgtctgga aggcccagct catgaatgac tttgtagctg tcaagatctt      660
   cccactccag gacaagcagt cgtggcagag tgaacgggag atcttcagca cacctggcat      720
   gaagcacgag aacctgctac agttcattgc tgccgagaag cgaggctcca acctcgaagt      780
   agagctgtgg ctcatcacgg ccttccatga caagggctcc ctcacggatt acctcaaggg      840
   gaacatcatc acatggaacg aactgtgtca tgtagcagag acgatgtcac gaggcctctc      900
   atacctgcat gaggatgtgc cctggtgccg tggcgagggc cacaagccgt ctattgccca      960
   cagggacttt aaaagtaaga atgtattgct gaagagcgac ctcacagccg tgctggctga     1020
   ctttggcttg gctgttcgat ttgagccagg gaaacctcca ggggacaccc acggacaggt     1080
   aggcacgaga cggtacatgg ctcctgaggt gctcgaggga gccatcaact tccagagaga     1140
   tgccttcctg cgcattgaca tgtatgccat ggggttggtg ctgtgggagc ttgtgtctcg     1200
   ctgcaaggct gcagacggac ccgtggatga gtacatgctg ccctttgagg aagagattgg     1260
   ccagcaccct tcgttggagg agctgcagga ggtggtggtg cacaagaaga tgaggcccac     1320
   cattaaagat cactggttga aacacccggg cctggcccag ctttgtgtga ccatcgagga     1380
   gtgctgggac catgatgcag aggctcgctt gtccgcgggc tgtgtggagg agcgggtgtc     1440
   cctgattcgg aggtcggtca acggcactac ctcggactgt ctcgtttccc tggtgacctc     1500
   tgtcaccaat gtggacctgc cccctaaaga gtcaagcatc taagcccagg acatgagtgt     1560
   ctctccagac tcagtggatc tgaa                                            1584
<210> 6
   <211> 1770
   <212> DNA
   <213> Homo sapiens
<400> 6
   ccacgcgtcc gcacttccag ggtcggggag acggaactgc ggcgaccatg tatttctggt 60
   ttatcaaacc gctaacaccc agtctaaggg caggttctgt cccattgtta tcactatcga 120
   agcagccgat ggaggagggg aggtctgagc agagggcggg gtgcaggcgg aatggccctc 180
   gtgccctatg aggagaccac ggaatttggg ttgcagaaat tccacaagcc tcttgcaact 240
   ttttcctttg caaaccacac gatccagatc cggcaggact ggagacacct gggagtcgca 300
   gcggtggttt gggatgcggc catcgttctt tccacatacc tggagatggg agctgtggag 360
   ctcaggggcc gctctgccgt ggagctgggt gctggcacgg ggctggtggg catagtggct 420
   gccctgctgg gtgctcatgt gactatcacg gatcgaaaag tagcattaga atttcttaaa 480
   tcaaacgttc aagccaactt acctcctcat atccaaacta aaactgttgt taaggagctg 540
   acttggggac aaaatttggg gagtttttct cctggagaat ttgacctgat acttggtgct 600
   gatatcatat atttagaaga aacattcaca gatcttcttc aaacactgga acatctctgt 660
   agcaatcact ctgtgattct tttagcatgc cgaattcgct atgaacggga taacaacttc 720
   ttagcaatgc tggagaggca atttattgtg agaaaggttc actacgatcc tgaaaaagat 780
   gtacatattt acgaagcaca gaagagaaac cagaaggagg acttataatt ggctataatt 840
   tataagaatg ttgtcattga gtgtgtcact taaggtctta gactgcaaat ctaaccatat 900
   ttaatgaaat gtcttactgt acaaaaagtc taagccaaag gttctcaggg gagaaagcac 960
   atgtgcagtt ttaaaacaaa gcagtgcttt gtcccattgc tgtgattttt agtcagactt 1020
   tactcagtct gaaatgcaat taacattaaa ggattaagtg tgagatttcg atttatgcta 1080
   tttgtgtatc ccatactcct cccttttaat aaacagtttc cactgatgat atgaagggcc 1140
   ggtataaaga agtctttaaa tgagtaagct ttcttggtaa gattaaatct tacaaattat 1200
   ttttaaaacc ttgtgatata tacaatgttt agctgagttt tctaattttc tggatgtaaa 1260
   acaaaaggtt taacctatac attccttgag ctgttagtgc tatttaaatc ttttgccctg 1320
   tttaggtcct aaacactttt agttgagtag gatatgagct tttttgggtc tcatatcatg 1380
   ctttttgcct taatttcagg tatatatata tataagtaaa ggaattaagt aaaaataaaa 1440
   tttcagttac tttttaaaag cacctgaaat ctggccggat gcggtggctc atgcctgtaa 1500
   tcccaccact ttgggaggcc gaggcgggca gatcacctga ggtcgggagt tcaagaccag 1560
   cctggccaac atggtgaaac cccatctcta ctaaaaatac aaaaattagc cgggcgtggt 1620
   gtcgggcgcc tgtagtccca gctgctcggg aggctgaggc aggggaatcg cttgaacctg 1680
   ggaggcggag gttgcagtga gctgagattg cgccattgta ctccagcctg ggggacagga 1740
   gcgagactcc atctcaaaaa aaaaaaaaaa 1770
<210> 7
   <211> 1257
   <212> DNA
   <213> Homo sapiens
<400> 7
   gtcccgacgc ctcccgcccc cgcagttcct tggagagctt ggagccgcgc gccggaggga 60
   ataggaaagc ttggttacaa cccgggacac ccggagcttc aggatggttc gtactaagac 120
   atggaccctg aagaagcact ttgttggcta tcctactaat agtgactttg agttgaagac 180
   atctgagctc ccacccttaa aaaatggaga ggtcctgctt gaagctttgt tcctcaccgt 240
   ggatccctac atgagagtgg cagccaaaag attgaaggaa ggtgatacaa tgatggggca 300
   gcaagtggcc aaagttgtgg aaagtaaaaa tgtagcccta ccaaaaggaa ctattgtact 360
   ggcttctcca ggctggacaa cgcactccat ttctgatggg aaagatctgg aaaagctgct 420
   gacagagtgg ccagacacaa taccactgtc tttggctctg gggacagttg gcatgccagg 480
   cctgactgcc tactttggcc tacttgaaat ctgtggtgtg aagggtggag aaacagtgat 540
   ggttaatgca gcagctggag ctgtgggctc agtcgtgggg cagattgcaa agctcaaggg 600
   ctgcaaagtt gttggagcag tagggtctga tgaaaaggtt gcctaccttc aaaagcttgg 660
   atttgatgtc gtctttaact acaagacggt agagtctttg gaagaaacct tgaagaaagc 720
   gtctcctgat ggttatgatt gttattttga taatgtaggt ggagagtttt caaacactgt 780
   tatcggccag atgaagaaat ttggaaggat tgccatatgt ggagccatct ctacatataa 840
   cagaaccggc ccacttcccc caggcccacc cccagagatt gttatctatc aggagcttcg 900
   catggaagct tttgtcgtct accgctggca aggagatgcc cgccaaaaag ctctgaagga 960
   cttgctgaaa tgggtcttag agggtaaaat ccagtacaag gaatatatca ttgaaggatt 1020
   tgaaaacatg ccagccgcat ttatgggaat gctgaaagga gataatttgg ggaagacaat 1080
   agtgaaagca tgaaaaagag gacacatgga atctggaggc catttagatg attagttaat 1140
   ttgtttttca ccatttagca aaaatgtata ctaccttaaa tgtcttaaga aatagtactc 1200
   ataatgagtt tgagctactt aataaaatac atttaagtgg taaaaaaaaa aaaaaaa 1257
<210> 8
   <211> 910
   <212> DNA
   <213> Homo sapiens
<400> 8
   gtggcttgcg gctcgggtgg ctgagcgcgc ggggaaatgg ccacggggac agaccaggtg      60
   gtgggactcg gcctcgtcgc cgttagcctg atcatcttca cctactacac cgcctgggtg     120
   attctcttgc cattcatcga cagtcagcat gtcatccaca agtatttcct gccccgagcc     180
   tatgctgtcg ccatcccact ggctgcaggc ctcctgctgc tcctgtttgt gggactgttc     240
   atctcctatg tgatgctgaa gaccaagaga gtgaccaaga aggctcagtg aaggtcccgc     300
   agggatgagg ctgccagccc cttctctgct tcccctccag cacagggacc aagtggggga     360
   gcctgcagaa cctgtccagg cacagtggct cctcaagcct gcctgtcctg cagagtcccc     420
   atggcatgga gcttacacct gactgactgg agccccctcc ccgactccca cttccagaag     480
   ctaggaggga gggatacctg gaagactccg gtcacctcct tcttgctcag ggcctaaaag     540
   atgctggtcc tcccaacctc actctcagac tccctgccac cttttcccct gggttctgcc     600
   gtcttgcctc acttcccctc ctgtcacatg ctgacgttgg acttagcagg ttctaaggcc     660
   acatgtgtga cctctctgac ttctcttcct ccaccaaggc agctttcctt accctgacac     720
   agccccagac cccacaaagc cttctggacc tggaaagcct ggggaaggac tgacagaccc     780
   caggaccagc cctggggctc agggcagcca ccccgggccg ctgaccgact gacctctcct     840
   cacggaggcc cagccccaaa gccccagggc tggcccgttt gggacagctg accaataaac     900
   actgatggtg                                                            910
<210> 9
   <211> 1386
   <212> DNA
   <213> Homo sapiens
<400> 9
   ggaagccggg attcgccctc cggggagcga ttggtcctcg ggaggggcgg ggaggtggac      60
   gcgggtaccg gcggtcgtcg ggtcggcagc ctttggtcag ttggcagcgg caagcgcgct     120
   gcggttccgg tggcgccatg tcgttctgca gcttcttcgg gggcgaggtt ttccagaatc     180
   actttgaacc tggcgtttac gtgtgtgcca agtgtggcta tgagctgttc tccagccgct     240
   cgaagtatgc acactcgtct ccatggccgg cgttcaccga gaccattcac gccgacagcg     300
   tggccaagcg tccggagcac aatagatctg aagccttgaa ggtgtcctgt ggcaagtgtg     360
   gcaatgggtt gggccacgag ttcctgaacg acggccccaa gccggggcag tcccgattct     420
   gaatattcag cagctcgctg aagtttgtcc ctaaaggcaa agaaacttct gcctcccagg     480
   gtcactaggc gggcagccca cacccacccc agacggccac cacactgagg ccacacgttg     540
   gccattccac cttggagttg gaaccctggg cgtcgagaca ggaaggcagg gcgcagtggt     600
   tgaaacatca ggacactccc aaggccccgg ctctgaacaa gaccttttcg tttcttggaa     660
   aagagactca tttgctgatg gttcatgcct tctgctggga caggcctggg ctgtgcagcc     720
   acactgtcgg ctgacttagc cccctgctca ctctaggtgc ctccaggagg tgagccctgg     780
   gtgcagctgg tctctgaatg acgttacacc ctcaccttct tttcctggcc ctgtctctgg     840
   actctcccct gtgaggccca attccaagac agactctcgt cctcaccgaa gcttaggccc     900
   acatctccca ggctgcttag gagacagaat ggaaacggag gccgcccctg ccagccgccc     960
   tggccctggt cactgcatga tccgctctgg tcaaaccctt ccaggccagc cagagtgggg    1020
   atggtctgtg acctgctggg aaggcaggct gatggggcac acccttggcc tctcgtccac    1080
   gaggggagaa acctaaaccc tgtttcacaa tctgtgcgga agtagcttgc ctcacttctg    1140
   cttaggaaag cggctgttgc tccataactc taaccagcac agggctgagg cctgcagtgc    1200
   acacctgcag ggaggccctt cccaaggtgt ggtgactgtg ccttactgta catgctcgga    1260
   ggcctggcca tataggaggg tgggtgatgc tgaaatcacc ccccatctta agtaattact    1320
   ttctggagta atcaggtgga aatccataga caaatgaaac attcagaaaa aaaaaaaaaa    1380
   aaaaaa                                                               1386
<210> 10
   <211> 949
   <212> DNA
   <213> Homo sapiens
<400> 10
   gcgctccggg cctggaatcc ctacgcgtcc ctttgggttt agcacgatga gctcaatcgg 60
   cactgggtat gacctgtcag cctctacatt ctctcctgac ggaagagttt ttcaagttga 120
   atatgctatg aaggctgtgg aaaatagtag tacagctatt ggaatcagat gcaaagatgg 180
   tgttgtcttt ggggtagaaa aattagtcct ttctaaactt tatgaagaag gttccaacaa 240
   aagacttttt aatgttgatc ggcatgttgg aatggcagta gcaggtttgt tggcagatgc 300
   tcgttcttta gcagacatag caagagaaga agcttccaac ttcagatcta actttggcta 360
   caacattcca ctaaaacatc ttgcagacag agtggccatg tatgtgcatg catatacact 420
   ctacagtgct gttagacctt ttggctgcag tttcatgtta gggtcttaca gtgtgaatga 480
   cggtgcgcaa ctctacatga ttgacccatc aggtgtttca tacggttatt ggggctgtgc 540
   catcggcaaa gccaggcaag ctgcaaagac ggaaatagag aagcttcaga tgaaagaaat 600
   gacctgccgt gatatcgtta aagaagttgc aaaaataatt tacatagtac atgacgaagt 660
   taaggataaa gcttttgaac tagaactcag ctgggttggt gaattaacta atggaagaca 720
   tgaaattgtt ccaaaagata taagagaaga agcagagaaa tatgctaagg aatctctgaa 780
   ggaagaagat gaatcagatg atgataatat gtaacattta ctccagcatc tattgtattt 840
   taaatttcta ctccagtcca atgtaactat ttagccctgg attatacata ctgtccaatt 900
   ttcattaaat ttttgtctta taactataaa aaaaaaaaaa aaaaaaaaa 949
<210> 11
   <211> 1623
   <212> DNA
   <213> Homo sapiens
<400> 11
   gctggtttct gggggtcgtg gccttgctcc cgctgtgcgg gaaaagaatc caggcccttc 60
   cacgcgcgtg tgggtgcggg ggccccgaag tgctcgtggt tccccgctag gtctccgctg 120
   gggcaggaac cggaatcatg ggtgggacca ccagcacccg ccgggtcacc ttcgaggcgg 180
   acgagaatga gaacatcacc gtggtgaagg gcatccggct ttcggaaaat gtgattgatc 240
   gaatgaagga atcctctcca tctggttcga agtctcagcg gtattctggt gcttatggtg 300
   cctcagtttc tgatgaagaa ttgaaaagaa gagtagctga ggagctggca ttggagcaag 360
   ccaagaaaga atccgaagat cagaaacgac taaagcaagc caaagagctg gaccgagaga 420
   gggctgctgc caatgagcag ttaaccagag ccatccttcg ggagaggata tgtagcgagg 480
   aggaacgcgc taaggcaaag cacctggcta ggcagctgga agagaaagac cgagtgctaa 540
   agaagcagga tgcattctac aaagaacagc tggctagact ggaggagagg agctcagagt 600
   tctacagagt caccactgaa caatatcaga aagctgctga agaggtggaa gcaaagttca 660
   agcgatatga gtctcatcca gtctgtgctg atctgcaggc caaaattctt cagtgttacc 720
   gtgagaacac ccaccagacc ctcaaatgct ccgctctggc cacccagtat atgcactgtg 780
   tcaatcatgc caaacagagc atgcttgaga agggaggata aaaactttca gaatgagcaa 840
   aacaccatca acgttaattc cagagatgga acattttttt tcctagtgag aaaacaaccc 900
   atttgaagag aagaccacta atgagaagac cactaaagag agacatcaag aatggattca 960
   gcagaatcat ttcacgtttt gaacagcagc agtttgaagg gccaaagcct tgatcaggga 1020
   tcagtcatta aaggacactc ttgagtatta gtaaaccctc ttatgatgat taaaagagaa 1080
   gggcagccct ctccaccttt tggtactttc tattcaactt gcactgacca taaaatgttt 1140
   ctcttctgaa caagccccat catttggtga acctccaccc taacaaagta ggatggggtt 1200
   gggggctaaa ttaattggag tggggcgagg agagagccag aaaacataga tccgagggca 1260
   gcagtgctgg gtggagagag ccagaaaaca gatctggagg cagcagtgct ggatggaatt 1320
   gtctaggctg tggcatgttg gttttgtctt tcttttctcc tttgattatg taagagctat 1380
   ttcattataa cttattatgg tgattataca ggcaagaaga caaaaaggag agaaaatgta 1440
   cctcttctac tggaataatg tttatgatta caagtgagat aaggtatttt tatcaatatg 1500
   aaggcaacct tggctgataa aacctctata gtgaatactc acatctttac ttcactcact 1560
   atcaataata aatatatttt ctgacaaaga ctggcaaaaa aaaaaaaaaa aaaaaaaaaa 1620
   aaa 1623
<210> 12
   <211> 579
   <212> DNA
   <213> Homo sapiens
<400> 12
   acgaggggaa agggcgcagg gtttgaaaca tggcggacga cgtagaccag caacaaacta      60
   ccaacactgt agaggagccc ctggatctta tcaggctcag cctagatgag cgaatttatg     120
   tgaaaatgag aaatgaccga gagcttcgag gcagattaca tgcttatgat caacatttaa     180
   atatgatctt gggagatgtg gaagaaactg tgactactat agaaattgat gaagaaacat     240
   atgaagagat atataaatca acgaaacgga atattccaat gctctttgtc cggggagatg     300
   gcgttgtcct ggttgcccct ccactgagag ttggctgaaa caaagaattt gtcctgtatg     360
   gaaaacggga gactttgtac agtggcctct ctaaaagtac aaaacattca taagagaaac     420
   ctgcatacat tttgatatta agaaataatt ccggggattc ttccactcct gaaatgagtt     480
   gatttgcaga taactcacaa cttcttaagc taaatggtat tttcattttt ctcaagctct     540
   ccaataaata tgaccaccaa aaaaaaaaaa aaaaaaaaa                            579
<210> 13
   <211> 4493
   <212> DNA
   <213> Homo sapiens
<400> 13
   atcgtctgcc gctttggtga cttctgacag ctctctccat ggaaggaggc ggcggccgcg      60
   atgagccttc agcctgccgg gcaggggacg tgaacatgga tgaccctaag aaggaagaca     120
   ttcttctttt ggccgatgaa aaatttgact tcgatctttc attgtcttct tcgagtgcaa     180
   atgaagatga tgaagtcttc ttcggaccct ttggacataa agaaagatgt attgctgcca     240
   gcttggaatt aaataatccg gttcccgaac agcctccgtt gcccacatct gagagtccct     300
   ttgcctggag ccctctggcc ggggagaagt tcgtggaggt gtacaaagaa gctcacttac     360
   tggctttaca cattgagagc agcagccgga accaggcagc ccaagctgcc aagcctgaag     420
   accctcggag ccagggcgtg gaaagattca tacaggagtc aaaattaaaa ataaacctct     480
   ttgagaaaga aaaggaaatg aagaaaagcc ccacgtctct taaaagggag acatactacc     540
   tgtcagacag ccccttgctg ggcccccctg tgggtgagcc tcggctcttg gcctcctccc     600
   cggccctgcc cagctctggt gcccaggccc gcctcacccg ggcgccgggg cctccgcact     660
   ctgctcatgc tttgcccagg gaatcatgca ctgctcatgc tgcaagtcag gcagcgactc     720
   agaggaagcc cgggaccaaa ttgctgctgc ctcgagcggc ctctgttaga ggaagaagca     780
   tccctggggc tgcggagaag cccaagaaag agattccagc tagtccttcc aggacaaaaa     840
   tcccagctga gaaggaatcc caccgggatg ttctccctga caaacctgcc ccgggtgctg     900
   tcaatgtgcc ggccgccgga agccacttgg gccagggcaa gcgggcgatc cctgttccaa     960
   acaagttggg gctgaagaag accctgttaa aagcacccgg ctctaccagc aatctcgcaa    1020
   ggaagtcctc ctcggggcct gtttggagcg gggcatccag tgcgtgcaca tccccagcag    1080
   tgggcaaagc taaatcaagt gaatttgcaa gtattcctgc aaatagctcc cggcctctgt    1140
   caaacatcag caagtcaggc agaatgggac ccgccatgct gcggccagct ctgcctgcag    1200
   gccctgtggg ggcatcctcc tggcaggcca agcgggtcga tgtttctgag ctggcagcgg    1260
   agcagctcac ggcacccccc tcagcatccc ccacccaacc ccagactccg gaaggtggcg     1320
   gccagtggct gaactccagt tgcgcttggt cagaatcttc tcaattgaat aagactagaa    1380
   gtatcagacg gcgagattcc tgtctaaatt ccaagacaaa ggttatgcct actcctacaa    1440
   atcaatttaa aattcctaag ttttctattg gtgactcccc ggacagctca acaccaaagc    1500
   tttcgcgggc acagcggccg cagtcgtgca cgtcagttgg cagggtcact gtccacagca    1560
   ccccggttag acgctcatct gggccagcac cacaaagcct gctgagcgca cggcgtgtgt    1620
   cagccttgcc cacacccgcc agccggcgct gctctggcct tccaccgatg acccccaaaa    1680
   cgatgcccag ggccgtgggc tctcccctgt gtgtgccagc tcggagacgt tcctctgagc    1740
   cccgcaagaa ctctgcaatg agaactgaac caacaaggga gagcaacaga aagacagatt    1800
   ccaggctggt ggatgtgtcc cctgacaggg gttctcctcc ttcccgtgtg cctcaggcac    1860
   ttaacttttc tccagaggaa agcgattcta ctttctccaa aagtactgcc acagaagtag    1920
   ctcgggagga agccaagccg ggtggagatg cagcccctag tgaggctctt cttgtagata    1980
   tcaaactgga accactcgcg gtcactccag atgctgcaag ccagcccctc attgaccttc    2040
   ctctcatcga cttctgcgat accccagaag cacacgtggc tgtaggatct gaaagcaggc    2100
   ctctgatcga cctcatgaca aacactccag acatgaataa aaatgtggcc aaaccttcac    2160
   cggtggtggg acagctcata gacctgagct cccctctgat ccagctgagc cctgaggctg    2220
   acaaggagaa cgtggattcc ccactcctca agttctaagc cgaaccaaat cctttgcctt    2280
   gaaagaacag ccctaaagtg gttttcaacc ctcagaaaca agctttaggc tggtcgcagt    2340
   ggcttacact tgtaacccta gaacttggga ggctgaggtg ggcggattac ttgagcccag    2400
   gagttcggga ccagcctggg aaatatagtg aaactcctgt ccctacaaaa aatacaaaaa    2460
   ttagccgggt gtggtagtgc atgcctgtag tcccagctac ttgggaggct gaagtgggag    2520
   gatggcctga gctcaaggag atgcaggctg cagtgggctg tgattgtgcc actgcactcc    2580
   agcctgggca ccaatgtgag aacctgtctt ggaaaaaaaa aaaaagaaac atgttttagt 2640
   agaagtttta tttgaaaaag aaaaataagc ataaatatat tcccagtgct ggagagggtg 2700
   ggctgaggga ctggggccag cacggaccac ccaaggcctc tgcttcccgc cgccaccctc 2760
   ctcgctgcca ttctctgggc tggaatgtga agcctcagtc actctaaatg aagaattttc 2820
   ttttgaatgt tttgtatgta aaatagcaag tggctatttt taaagttaag tttgtataaa 2880
   tagttagata ttctagattt acattaaatt gtaaaataaa tggacttatt gaagcatatc 2940
   ttgattttta agcttatctt gattttcaaa catgcatagc tatttttatc actctaatca 3000
   gtaaggctac tatctagact cgaatgcttt catacaagtg attttcaaaa attagtcaat 3060
   aaaaattgat gtcagtgcag gcccaggccc gcccccagat acactagttt ctaggtctgg 3120
   ggccagccta gtaattgtta ctaggcacac aggtgatgct gactcgatgg cctgagacac 3180
   accccttgag aagaagctgc tctggggaga cgagggtatg agtggaaaga ggatgggcga 3240
   ggaggccggg cttacctgga cactaaggtg atggacggcg tctgctgggc aggcctcggg 3300
   gaggacccct cagctttgct ctcagcaggg gcccgacaag ctcagtgggc agtggcagga 3360
   actgagtgcc actggaaagc ccattccctt tatttagaaa acgagctcca ggaagccgct 3420
   actttgtgtc catttctctt gaggaaactt accaccttgg ttgagcggct tcatggcaga 3480
   cagcagcgag ccagcggccg gactctgtat ttcggacccc actccagtgc tccctgggtc 3540
   ataccaggat ctgcctctgt ccacaaagat gagggaaaat gatgactgtg ctgtgctctc 3600
   tacttcctgc ggttactgcg tgattctaaa gctgccactc ggaacagcga gtcctctgcc 3660
   gtcgagagca gggaggggta gggtggacag gcgatgccgt ggaaagacgt ctcggtggaa 3720
   actgccatgg tggaaaggtg gcgcgcttct cacggctgag ttgctgcgcc tgcagacgga 3780
   agctccccac aggcagagct gcttggatgt gtgagtcatg aagccagaga agccccgctc 3840
   catgagcagt gactccccag gccctgtgac ctccctcctg tcttgcagct cctcctggca 3900
   ccagtcccca gggctctcct gttggtagtt cctgcttttc ttcttggaaa ttcctcgtgg 3960
   acctcgagat ctttacccta aaatagttct gttgaatttc accctggcaa tgtaaattga 4020
   tagcttatct tcacagatgc cagacaatgg acaactcacc atcagtcctc tgctcacctg 4080
   agacaaatgc atgtctgatt gcttcctctg ccctattgtt tatgtgaaaa tgcagattca 4140
   ctgagccaga ctaaggcatc agtgactgtt cctctacctg cctctcacat ggagattgtg 4200
   tattcagtga aaggctgatc aaagacccaa aggaatgcaa cagtttatct cttatctacc 4260
   tatgacctgc gagctgccca ccacccccag ttgttgcgcc tttccagaca gaaccagtgt 4320
   acatcttaca cgtattaatt gatgtcctgt gtctccctaa tatgtatcaa agcaagctgt 4380
   gcctcgacca ccttgggcac atatcctcag gacatcatcc tgaggctgtg tcactggcat 4440
   gtccttaacc ttggcaaaat aaactttcta aattgaaaaa aaaaaaaaaa aaa 4493
<210> 14
   <211> 225
   <212> PRT
   <213> Homo sapiens
<400> 14
   Met Asn Ser Asn Val Glu Asn Leu Pro Pro His Ile Ile Arg Leu Val
   1 5 10 15
   Tyr Lys Glu Val Thr Thr Leu Thr Ala Asp Pro Pro Asp Gly Ile Lys
   20 25 30
   Val Phe Pro Asn Glu Glu Asp Leu Thr Asp Leu Gln Val Thr Ile Glu
   35 40 45
   Gly Pro Glu Gly Thr Pro Tyr Ala Gly Gly Leu Phe Arg Met Lys Leu
   50 55 60
   Leu Leu Gly Lys Asp Phe Pro Ala Ser Pro Pro Lys Gly Tyr Phe Leu
   65 70 75 80
   Thr Lys Ile Phe His Pro Asn Val Gly Ala Asn Gly Glu Ile Cys Val
   85 90 95
   Asn Val Leu Lys Arg Asp Trp Thr Ala Glu Leu Gly Ile Arg His Val
   100 105 110
   Leu Leu Thr Ile Lys Cys Leu Leu Ile His Pro Asn Pro Glu Ser Ala
   115 120 125
   Leu Asn Glu Glu Ala Gly Arg Leu Leu Leu Glu Asn Tyr Glu Glu Tyr
   130 135 140
   Ala Ala Arg Ala Arg Leu Leu Thr Glu Ile His Gly Gly Ala Gly Gly
   145 150 155 160
   Pro Ser Gly Arg Ala Glu Ala Gly Arg Ala Leu Ala Ser Gly Thr Glu
   165 170 175
   Ala Ser Ser Thr Asp Pro Gly Ala Pro Gly Gly Pro Gly Gly Ala Glu
   180 185 190
   Gly Pro Met Ala Lys Lys His Ala Gly Glu Arg Asp Lys Lys Leu Ala
   195 200 205
   Ala Lys Lys Lys Thr Asp Lys Lys Arg Ala Leu Arg Ala Leu Arg Arg
   210 215 220
   Leu
   225
<210> 15
   <211> 363
   <212> PRT
   <213> Homo sapiens
<400> 15
   Met Gln Ala Phe Leu Lys Gly Thr Ser Ile Ser Thr Lys Pro Pro Leu
   1               5                   10                  15
   Thr Lys Asp Arg Gly Val Ala Ala Ser Ala Gly Ser Ser Gly Glu Asn
               20                  25                  30
   Lys Lys Ala Lys Pro Val Pro Trp Val Glu Lys Tyr Arg Pro Lys Cys
           35                  40                  45
   Val Asp Glu Val Ala Phe Gln Glu Glu Val Val Ala Val Leu Lys Lys
       50                  55                  60
   Ser Leu Glu Gly Ala Asp Leu Pro Asn Leu Leu Phe Tyr Gly Pro Pro
   65                  70                  75                  80
   Gly Thr Gly Lys Thr Ser Thr Ile Leu Ala Ala Ala Arg Glu Leu Phe
                   85                  90                  95
   Gly Pro Glu Leu Phe Arg Leu Arg Val Leu Glu Leu Asn Ala Ser Asp
               100                 105                 110
   Glu Arg Gly Ile Gln Val Val Arg Glu Lys Val Lys Asn Phe Ala Gln
           115                 120                 125
   Leu Thr Val Ser Gly Ser Arg Ser Asp Gly Lys Pro Cys Pro Pro Phe
       130                 135                 140
   Lys Ile Val Ile Leu Asp Glu Ala Asp Ser Met Thr Ser Ala Ala Gln
   145                 150                 155                 160
   Ala Ala Leu Arg Arg Thr Met Glu Lys Glu Ser Lys Thr Thr Arg Phe
                   165                 170                 175
   Cys Leu Ile Cys Asn Tyr Val Ser Arg Ile Ile Glu Pro Leu Thr Ser
               180                 185                 190
   Arg Cys Ser Lys Phe Arg Phe Lys Pro Leu Ser Asp Lys Ile Gln Gln
           195                 200                 205
   Gln Arg Leu Leu Asp Ile Ala Lys Lys Glu Asn Val Lys Ile Ser Asp
       210                 215                 220
   Glu Gly Ile Ala Tyr Leu Val Lys Val Ser Glu Gly Asp Leu Arg Lys
   225                 230                 235                 240
   Ala Ile Thr Phe Leu Gln Ser Ala Thr Arg Leu Thr Gly Gly Lys Glu
                   245                 250                 255
   Ile Thr Glu Lys Val Ile Thr Asp Ile Ala Gly Val Ile Pro Ala Glu
               260                 265                 270
   Lys Ile Asp Gly Val Phe Ala Ala Cys Gln Ser Gly Ser Phe Asp Lys
           275                 280                 285
   Leu Glu Ala Val Val Lys Asp Leu Ile Asp Glu Gly His Ala Ala Thr
       290                 295                 300
   Gln Leu Val Asn Gln Leu His Asp Val Val Val Glu Asn Asn Leu Ser
   305                 310                 315                 320
   Asp Lys Gln Lys Ser Ile Ile Thr Glu Lys Leu Ala Glu Val Asp Lys
                   325                 330                 335
   Cys Leu Ala Asp Gly Ala Asp Glu His Leu Gln Leu Ile Ser Leu Cys
               340                 345                 350
   Ala Thr Val Met Gln Gln Leu Ser Gln Asn Cys
           355                 360
<210> 16
   <211> 377
   <212> PRT
   <213> Homo sapiens
<400> 16
   Met Asp Pro Ala Ala Arg Val Val Arg Ala Leu Trp Pro Gly Gly Cys
   1               5                   10                  15
   Ala Leu Ala Trp Arg Leu Gly Gly Arg Pro Gln Pro Leu Leu Pro Thr
               20                  25                  30
   Gln Ser Arg Ala Gly Phe Ala Gly Ala Ala Gly Gly Pro Ser Pro Val
           35                  40                  45
   Ala Ala Ala Arg Lys Gly Ser Pro Arg Leu Leu Gly Ala Ala Ala Leu
       50                  55                  60
   Ala Leu Gly Gly Ala Leu Gly Leu Tyr His Thr Ala Arg Trp His Leu
   65                  70                  75                  80
   Arg Ala Gln Asp Leu His Ala Glu Arg Ser Ala Ala Gln Leu Ser Leu
                   85                  90                  95
   Ser Ser Arg Leu Gln Leu Thr Leu Tyr Gln Tyr Lys Thr Cys Pro Phe
               100                 105                 110
   Cys Ser Lys Val Arg Ala Phe Leu Asp Phe His Ala Leu Pro Tyr Gln
           115                 120                 125
   Val Val Glu Val Asn Pro Val Arg Arg Ala Glu Ile Lys Phe Ser Ser
       130                 135                 140
   Tyr Arg Lys Val Pro Ile Leu Val Ala Gln Glu Gly Glu Ser Ser Gln
   145                 150                 155                 160
   Gln Leu Asn Asp Ser Ser Val Ile Ile Ser Ala Leu Lys Thr Tyr Leu
                   165                 170                 175
   Val Ser Gly Gln Pro Leu Glu Glu Ile Ile Thr Tyr Tyr Pro Ala Met
               180                 185                 190
   Lys Ala Val Asn Glu Gln Gly Lys Glu Val Thr Glu Phe Gly Asn Lys
           195                 200                 205
   Tyr Trp Leu Met Leu Asn Glu Lys Glu Ala Gln Gln Val Tyr Gly Gly
       210                 215                 220
   Lys Glu Ala Arg Thr Glu Glu Met Lys Trp Arg Gln Trp Ala Asp Asp
   225                 230                 235                 240
   Trp Leu Val His Leu Ile Ser Pro Asn Val Tyr Arg Thr Pro Thr Glu
                   245                 250                 255
   Ala Leu Ala Ser Phe Asp Tyr Ile Val Arg Glu Gly Lys Phe Gly Ala
               260                 265                 270
   Val Glu Gly Ala Val Ala Lys Tyr Met Gly Ala Ala Ala Met Tyr Leu
           275                 280                 285
   Ile Ser Lys Arg Leu Lys Ser Arg His Arg Leu Gln Asp Asn Val Arg
       290                 295                 300
   Glu Asp Leu Tyr Glu Ala Ala Asp Lys Trp Val Ala Ala Val Gly Lys
   305                 310                 315                 320
   Asp Arg Pro Phe Met Gly Gly Gln Lys Pro Asn Leu Ala Asp Leu Ala
                   325                 330                 335
   Val Tyr Gly Val Leu Arg Val Met Glu Gly Leu Asp Ala Phe Asp Asp
               340                 345                 350
   Leu Met Gln His Thr His Ile Gln Pro Trp Tyr Leu Arg Val Glu Arg
           355                 360                 365
   Ala Ile Thr Glu Ala Ser Pro Ala His
       370                 375
<210> 17
   <211> 256
   <212> PRT
   <213> Homo sapiens
<400> 17
   Met Lys Leu Leu Glu Asn Ser Ser Phe Glu Ala Ile Asn Ser Gln Leu
   1               5                   10                  15
   Thr Val Glu Thr Gly Asp Ala His Ile Ile Gly Arg Ile Glu Ser Tyr
               20                  25                  30
   Ser Cys Lys Met Ala Gly Asp Asp Lys His Met Phe Lys Gln Phe Cys
           35                  40                  45
   Gln Glu Gly Gln Pro His Val Leu Glu Ala Leu Ser Pro Pro Gln Thr
       50                  55                  60
   Ser Gly Leu Ser Pro Ser Arg Leu Ser Lys Ser Gln Gly Gly Glu Glu
   65                  70                  75                  80
   Glu Gly Pro Leu Ser Asp Lys Cys Ser Arg Lys Thr Leu Phe Tyr Leu
                   85                  90                  95
   Ile Ala Thr Leu Asn Glu Ser Phe Arg Pro Asp Tyr Asp Phe Ser Thr
               100                 105                 110
   Ala Arg Ser His Glu Phe Ser Arg Glu Pro Ser Leu Ser Trp Val Val
           115                 120                 125
   Asn Ala Val Asn Cys Ser Leu Phe Ser Ala Val Arg Glu Asp Phe Lys
       130                 135                 140
   Asp Leu Lys Pro Gln Leu Trp Asn Ala Val Asp Glu Glu Ile Cys Leu
   145                 150                 155                 160
   Ala Glu Cys Asp Ile Tyr Ser Tyr Asn Pro Asp Leu Asp Ser Asp Pro
                   165                 170                 175
   Phe Gly Glu Asp Gly Ser Leu Trp Ser Phe Asn Tyr Phe Phe Tyr Asn
               180                 185                 190
   Lys Arg Leu Lys Arg Ile Val Phe Phe Ser Cys Arg Ser Ile Ser Gly
           195                 200                 205
   Ser Thr Tyr Thr Pro Ser Glu Ala Gly Asn Glu Leu Asp Met Glu Leu
       210                 215                 220
   Gly Glu Glu Glu Val Glu Glu Glu Ser Arg Ser Arg Gly Ser Gly Ala
   225                 230                 235                 240
   Glu Glu Thr Ser Thr Met Glu Glu Asp Arg Val Pro Val Ile Cys Ile
                   245                 250                 255
<210> 18
   <211> 512
   <212> PRT
   <213> Homo sapiens
<400> 18
   Met Thr Ala Pro Trp Val Ala Leu Ala Leu Leu Trp Gly Ser Leu Trp
   1               5                   10                  15
   Pro Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Ala
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr Leu Leu Thr Val Leu Ala Tyr Ser Leu Leu
       130                 135                 140
   Pro Ile Gly Gly Leu Ser Leu Ile Val Leu Leu Ala Phe Trp Met Tyr
   145                 150                 155                 160
   Arg His Arg Lys Pro Pro Tyr Gly His Val Asp Ile His Glu Asp Pro
                   165                 170                 175
   Gly Pro Pro Pro Pro Ser Pro Leu Val Gly Leu Lys Pro Leu Gln Leu
               180                 185                 190
   Leu Glu Ile Lys Ala Arg Gly Arg Phe Gly Cys Val Trp Lys Ala Gln
           195                 200                 205
   Leu Met Asn Asp Phe Val Ala Val Lys Ile Phe Pro Leu Gln Asp Lys
       210                 215                 220
   Gln Ser Trp Gln Ser Glu Arg Glu Ile Phe Ser Thr Pro Gly Met Lys
   225                 230                 235                 240
   His Glu Asn Leu Leu Gln Phe Ile Ala Ala Glu Lys Arg Gly Ser Asn
                   245                 250                 255
   Leu Glu Val Glu Leu Trp Leu Ile Thr Ala Phe His Asp Lys Gly Ser
               260                 265                 270
   Leu Thr Asp Tyr Leu Lys Gly Asn Ile Ile Thr Trp Asn Glu Leu Cys
           275                 280                 285
   His Val Ala Glu Thr Met Ser Arg Gly Leu Ser Tyr Leu His Glu Asp
       290                 295                 300
   Val Pro Trp Cys Arg Gly Glu Gly His Lys Pro Ser Ile Ala His Arg
   305                 310                 315                 320
   Asp Phe Lys Ser Lys Asn Val Leu Leu Lys Ser Asp Leu Thr Ala Val
                   325                 330                 335
   Leu Ala Asp Phe Gly Leu Ala Val Arg Phe Glu Pro Gly Lys Pro Pro
               340                 345                 350
   Gly Asp Thr His Gly Gln Val Gly Thr Arg Arg Tyr Met Ala Pro Glu
           355                 360                 365
   Val Leu Glu Gly Ala Ile Asn Phe Gln Arg Asp Ala Phe Leu Arg Ile
       370                 375                 380
   Asp Met Tyr Ala Met Gly Leu Val Leu Trp Glu Leu Val Ser Arg Cys
   385                 390                 395                 400
   Lys Ala Ala Asp Gly Pro Val Asp Glu Tyr Met Leu Pro Phe Glu Glu
                   405                 410                 415
   Glu Ile Gly Gln His Pro Ser Leu Glu Glu Leu Gln Glu Val Val Val
               420                 425                 430
   His Lys Lys Met Arg Pro Thr Ile Lys Asp His Trp Leu Lys His Pro
           435                 440                 445
   Gly Leu Ala Gln Leu Cys Val Thr Ile Glu Glu Cys Trp Asp His Asp
       450                 455                 460
   Ala Glu Ala Arg Leu Ser Ala Gly Cys Val Glu Glu Arg Val Ser Leu
   465                 470                 475                 480
   Ile Arg Arg Ser Val Asn Gly Thr Thr Ser Asp Cys Leu Val Ser Leu
                   485                 490                 495
   Val Thr Ser Val Thr Asn Val Asp Leu Pro Pro Lys Glu Ser Ser Ile
               500                 505                 510
<210> 19
   <211> 218
   <212> PRT
   <213> Homo sapiens
<400> 19
   Met Ala Leu Val Pro Tyr Glu Glu Thr Thr Glu Phe Gly Leu Gln Lys
   1               5                   10                  15
   Phe His Lys Pro Leu Ala Thr Phe Ser Phe Ala Asn His Thr Ile Gln
               20                  25                  30
   Ile Arg Gln Asp Trp Arg His Leu Gly Val Ala Ala Val Val Trp Asp
           35                  40                  45
   Ala Ala Ile Val Leu Ser Thr Tyr Leu Glu Met Gly Ala Val Glu Leu
       50                  55                  60
   Arg Gly Arg Ser Ala Val Glu Leu Gly Ala Gly Thr Gly Leu Val Gly
   65                  70                  75                  80
   Ile Val Ala Ala Leu Leu Gly Ala His Val Thr Ile Thr Asp Arg Lys
                   85                  90                  95
   Val Ala Leu Glu Phe Leu Lys Ser Asn Val Gln Ala Asn Leu Pro Pro
               100                 105                 110
   His Ile Gln Thr Lys Thr Val Val Lys Glu Leu Thr Trp Gly Gln Asn
           115                 120                 125
   Leu Gly Ser Phe Ser Pro Gly Glu Phe Asp Leu Ile Leu Gly Ala Asp
       130                 135                 140
   Ile Ile Tyr Leu Glu Glu Thr Phe Thr Asp Leu Leu Gln Thr Leu Glu
   145                 150                 155                 160
   His Leu Cys Ser Asn His Ser Val Ile Leu Leu Ala Cys Arg Ile Arg
                   165                 170                 175
   Tyr Glu Arg Asp Asn Asn Phe Leu Ala Met Leu Glu Arg Gln Phe Ile
               180                 185                 190
   Val Arg Lys Val His Tyr Asp Pro Glu Lys Asp Val His Ile Tyr Glu
           195                 200                 205
   Ala Gln Lys Arg Asn Gln Lys Glu Asp Leu
       210                 215
<210> 20
   <211> 329
   <212> PRT
   <213> Homo sapiens
<400> 20
   Met Val Arg Thr Lys Thr Trp Thr Leu Lys Lys His Phe Val Gly Tyr
   1               5                   10                  15
   Pro Thr Asn Ser Asp Phe Glu Leu Lys Thr Ala Glu Leu Pro Pro Leu
               20                  25                  30
   Lys Asn Gly Glu Val Leu Leu Glu Ala Leu Phe Leu Thr Val Asp Pro
           35                  40                  45
   Tyr Met Arg Val Ala Ala Lys Arg Leu Lys Glu Gly Asp Thr Met Met
       50                  55                  60
   Gly Gln Gln Val Ala Lys Val Val Glu Ser Lys Asn Val Ala Leu Pro
   65                  70                  75                  80
   Lys Gly Thr Ile Val Leu Ala Ser Pro Gly Trp Thr Thr His Ser Ile
                   85                  90                  95
   Ser Asp Gly Lys Asp Leu Glu Lys Leu Leu Thr Glu Trp Pro Asp Thr
               100                 105                 110
   Ile Pro Leu Ser Leu Ala Leu Gly Thr Val Gly Met Pro Gly Leu Thr
           115                 120                 125
   Ala Tyr Phe Gly Leu Leu Glu Ile Cys Gly Val Lys Gly Gly Glu Thr
       130                 135                 140
   Val Met Val Asn Ala Ala Ala Gly Ala Val Gly Ser Val Val Gly Gln
   145 150 155 160
   Ile Ala Lys Leu Lys Gly Cys Lys Val Val Gly Ala Val Gly Ser Asp
   165 170 175
   Glu Lys Val Ala Tyr Leu Gln Lys Leu Gly Phe Asp Val Val Phe Asn
   180 185 190
   Tyr Lys Thr Val Glu Ser Leu Glu Glu Thr Leu Lys Lys Ala Ser Pro
   195 200 205
   Asp Gly Tyr Asp Cys Tyr Phe Asp Asn Val Gly Gly Glu Phe Ser Asn
   210 215 220
   Thr Val Ile Gly Gln Met Lys Lys Phe Gly Arg Ile Ala Ile Cys Gly
   225 230 235 240
   Ala Ile Ser Thr Tyr Asn Arg Thr Gly Pro Leu Pro Pro Gly Pro Pro
   245 250 255
   Pro Glu Ile Val Ile Tyr Gln Glu Leu Arg Met Glu Ala Phe Val Val
   260 265 270
   Tyr Arg Trp Gln Gly Asp Ala Arg Gln Lys Ala Leu Lys Asp Leu Leu
   275 280 285
   Lys Trp Val Leu Glu Gly Lys Ile Gln Tyr Lys Glu Tyr Ile Ile Glu
   290 295 300
   Gly Phe Glu Asn Met Pro Ala Ala Phe Met Gly Met Leu Lys Gly Asp
   305 310 315 320
   Asn Leu Gly Lys Thr Ile Val Lys Ala
   325
<210> 21
   <211> 84
   <212> PRT
   <213> Homo sapiens
<400> 21
<210> 22
   <211> 116
   <212> PRT
   <213> Homo sapiens
<400> 22
   Met Ser Phe Cys Ser Phe Phe Gly Gly Glu Val Phe Gln Asn His Phe
   1 5 10 15
   Glu Pro Gly Val Tyr Val Cys Ala Lys Cys Gly Tyr Glu Leu Phe Ser
   20 25 30
   Ser Arg Ser Lys Tyr Ala His Ser Ser Pro Trp Pro Ala Phe Thr Glu
   35 40 45
   Thr Ile His Ala Asp Ser Val Ala Lys Arg Pro Glu His Asn Arg Ser
   50 55 60
   Glu Ala Leu Lys Val Ser Cys Gly Lys Cys Gly Asn Gly Leu Gly His
   65 70 75 80
   Glu Phe Leu Asn Asp Gly Pro Lys Pro Gly Gln Ser Arg Phe Ser Ile
   85 90 95
   Phe Ser Ser Ser Leu Lys Phe Val Pro Lys Gly Lys Glu Thr Ser Ala
   100 105 110
   Ser Gln Gly His
   115
<210> 23
   <211> 255
   <212> PRT
   <213> Homo sapiens
<400> 23
   Met Ser Ser Ile Gly Thr Gly Tyr Asp Leu Ser Ala Ser Thr Phe Ser
   1 5 10 15
   Pro Asp Gly Arg Val Phe Gln Val Glu Tyr Ala Met Lys Ala Val Glu
   20 25 30
   Asn Ser Ser Thr Ala Ile Gly Ile Arg Cys Lys Asp Gly Val Val Phe
   35 40 45
   Gly Val Glu Lys Leu Val Leu Ser Lys Leu Tyr Glu Glu Gly Ser Asn
   50 55 60
   Lys Arg Leu Phe Asn Val Asp Arg His Val Gly Met Ala Val Ala Gly
   65 70 75 80
   Leu Leu Ala Asp Ala Arg Ser Leu Ala Asp Ile Ala Arg Glu Glu Ala
   85 90 95
   Ser Asn Phe Arg Ser Asn Phe Gly Tyr Asn Ile Pro Leu Lys His Leu
   100 105 110
   Ala Asp Arg Val Ala Met Tyr Val His Ala Tyr Thr Leu Tyr Ser Ala
   115 120 125
   Val Arg Pro Phe Gly Cys Ser Phe Met Leu Gly Ser Tyr Ser Val Asn
   130 135 140
   Asp Gly Ala Gln Leu Tyr Met Ile Asp Pro Ser Gly Val Ser Tyr Gly
   145 150 155 160
   Tyr Trp Gly Cys Ala Ile Gly Lys Ala Arg Gln Ala Ala Lys Thr Glu
   165 170 175
   Ile Glu Lys Leu Gln Met Lys Glu Met Thr Cys Arg Asp Ile Val Lys
   180 185 190
   Glu Val Ala Lys Ile Ile Tyr Ile Val His Asp Glu Val Lys Asp Lys
   195 200 205
   Ala Phe Glu Leu Glu Leu Ser Trp Val Gly Glu Leu Thr Asn Gly Arg
   210 215 220
   His Glu Ile Val Pro Lys Asp Ile Arg Glu Glu Ala Glu Lys Tyr Ala
   225 230 235 240
   Lys Glu Ser Leu Lys Glu Glu Asp Glu Ser Asp Asp Asp Asn Met
   245 250 255
<210> 24
   <211> 227
   <212> PRT
   <213> Homo sapiens
<400> 24
   Met Gly Gly Thr Thr Ser Thr Arg Arg Val Thr Phe Glu Ala Asp Glu
   1               5                   10                  15
   Asn Glu Asn Ile Thr Val Val Lys Gly Ile Arg Leu Ser Glu Asn Val
               20                  25                  30
   Ile Asp Arg Met Lys Glu Ser Ser Pro Ser Gly Ser Lys Ser Gln Arg
           35                  40                  45
   Tyr Ser Gly Ala Tyr Gly Ala Ser Val Ser Asp Glu Glu Leu Lys Arg
       50                  55                  60
   Arg Val Ala Glu Glu Leu Ala Leu Glu Gln Ala Lys Lys Glu Ser Glu
   65                  70                  75                  80
   Asp Gln Lys Arg Leu Lys Gln Ala Lys Glu Leu Asp Arg Glu Arg Ala
                   85                  90                  95
   Ala Ala Asn Glu Gln Leu Thr Arg Ala Ile Leu Arg Glu Arg Ile Cys
               100                 105                 110
   Ser Glu Glu Glu Arg Ala Lys Ala Lys His Leu Ala Arg Gln Leu Glu
           115                 120                 125
   Glu Lys Asp Arg Val Leu Lys Lys Gln Asp Ala Phe Tyr Lys Glu Gln
       130                 135                 140
   Leu Ala Arg Leu Glu Glu Arg Ser Ser Glu Phe Tyr Arg Val Thr Thr
   145                 150                 155                 160
   Glu Gln Tyr Gln Lys Ala Ala Glu Glu Val Glu Ala Lys Phe Lys Arg
                   165                 170                 175
   Tyr Glu Ser His Pro Val Cys Ala Asp Leu Gln Ala Lys Ile Leu Gln
               180                 185                 190
   Cys Tyr Arg Glu Asn Thr His Gln Thr Leu Lys Cys Ser Ala Leu Ala
           195                 200                 205
   Thr Gln Tyr Met His Cys Val Asn His Ala Lys Gln Ser Met Leu Glu
       210                 215                 220
   Lys Gly Gly
   225
<210> 25
   <211> 102
   <212> PRT
   <213> Homo sapiens
<400> 25
   Met Ala Asp Asp Val Asp Gln Gln Gln Thr Thr Asn Thr Val Glu Glu
   1               5                   10                  15
   Pro Leu Asp Leu Ile Arg Leu Ser Leu Asp Glu Arg Ile Tyr Val Lys
               20                  25                  30
   Met Arg Asn Asp Arg Glu Leu Arg Gly Arg Leu His Ala Tyr Asp Gln
           35                  40                  45
   His Leu Asn Met Ile Leu Gly Asp Val Glu Glu Thr Val Thr Thr Ile
       50                  55                  60
   Glu Ile Asp Glu Glu Thr Tyr Glu Glu Ile Tyr Lys Ser Thr Lys Arg
   65                  70                  75                  80
   Asn Ile Pro Met Leu Phe Val Arg Gly Asp Gly Val Val Leu Val Ala
                   85                  90                  95
   Pro Pro Leu Arg Val Gly
               100
<210> 26
   <211> 720
   <212> PRT
   <213> Homo sapiens
<400> 26
   Met Asp Asp Pro Lys Lys Glu Asp Ile Leu Leu Leu Ala Asp Glu Lys
   1               5                   10                  15
   Phe Asp Phe Asp Leu Ser Leu Ser Ser Ser Ser Ala Asn Glu Asp Asp
               20                  25                  30
   Glu Val Phe Phe Gly Pro Phe Gly His Lys Glu Arg Cys Ile Ala Ala
           35                  40                  45
   Ser Leu Glu Leu Asn Asn Pro Val Pro Glu Gln Pro Pro Leu Pro Thr
       50                  55                  60
   Ser Glu Ser Pro Phe Ala Trp Ser Pro Leu Ala Gly Glu Lys Phe Val
   65                  70                  75                  80
   Glu Val Tyr Lys Glu Ala His Leu Leu Ala Leu His Ile Glu Ser Ser
                   85                  90                  95
   Ser Arg Asn Gln Ala Ala Gln Ala Ala Lys Pro Glu Asp Pro Arg Ser
               100                 105                 110
   Gln Gly Val Glu Arg Phe Ile Gln Glu Ser Lys Leu Lys Ile Asn Leu
           115                 120                 125
   Phe Glu Lys Glu Lys Glu Met Lys Lys Ser Pro Thr Ser Leu Lys Arg
       130                 135                 140
   Glu Thr Tyr Tyr Leu Ser Asp Ser Pro Leu Leu Gly Pro Pro Val Gly
   145                 150                 155                 160
   Glu Pro Arg Leu Leu Ala Ser Ser Pro Ala Leu Pro Ser Ser Gly Ala
                   165                 170                 175
   Gln Ala Arg Leu Thr Arg Ala Pro Gly Pro Pro His Ser Ala His Ala
               180                 185                 190
   Leu Pro Arg Glu Ser Cys Thr Ala His Ala Ala Ser Gln Ala Ala Thr
           195                 200                 205
   Gln Arg Lys Pro Gly Thr Lys Leu Leu Leu Pro Arg Ala Ala Ser Val
       210                 215                 220
   Arg Gly Arg Ser Ile Pro Gly Ala Ala Glu Lys Pro Lys Lys Glu Ile
   225                 230                 235                 240
   Pro Ala Ser Pro Ser Arg Thr Lys Ile Pro Ala Glu Lys Glu Ser His
                   245                 250                 255
   Arg Asp Val Leu Pro Asp Lys Pro Ala Pro Gly Ala Val Asn Val Pro
               260                 265                 270
   Ala Ala Gly Ser His Leu Gly Gln Gly Lys Arg Ala Ile Pro Val Pro
           275                 280                 285
   Asn Lys Leu Gly Leu Lys Lys Thr Leu Leu Lys Ala Pro Gly Ser Thr
       290                 295                 300
   Ser Asn Leu Ala Arg Lys Ser Ser Ser Gly Pro Val Trp Ser Gly Ala
   305                 310                 315                 320
   Ser Ser Ala Cys Thr Ser Pro Ala Val Gly Lys Ala Lys Ser Ser Glu
                   325                 330                 335
   Phe Ala Ser Ile Pro Ala Asn Ser Ser Arg Pro Leu Ser Asn Ile Ser
               340                 345                 350
   Lys Ser Gly Arg Met Gly Pro Ala Met Leu Arg Pro Ala Leu Pro Ala
           355                 360                 365
   Gly Pro Val Gly Ala Ser Ser Trp Gln Ala Lys Arg Val Asp Val Ser
       370                 375                 380
   Glu Leu Ala Ala Glu Gln Leu Thr Ala Pro Pro Ser Ala Ser Pro Thr
   385                 390                 395                 400
   Gln Pro Gln Thr Pro Glu Gly Gly Gly Gln Trp Leu Asn Ser Ser Cys
                   405                 410                 415
   Ala Trp Ser Glu Ser Ser Gln Leu Asn Lys Thr Arg Ser Ile Arg Arg
               420                 425                 430
   Arg Asp Ser Cys Leu Asn Ser Lys Thr Lys Val Met Pro Thr Pro Thr
           435                 440                 445
   Asn Gln Phe Lys Ile Pro Lys Phe Ser Ile Gly Asp Ser Pro Asp Ser
       450                 455                 460
   Ser Thr Pro Lys Leu Ser Arg Ala Gln Arg Pro Gln Ser Cys Thr Ser
   465                 470                 475                 480
   Val Gly Arg Val Thr Val His Ser Thr Pro Val Arg Arg Ser Ser Gly
                   485                 490                 495
   Pro Ala Pro Gln Ser Leu Leu Ser Ala Arg Arg Val Ser Ala Leu Pro
               500                 505                 510
   Thr Pro Ala Ser Arg Arg Cys Ser Gly Leu Pro Pro Met Thr Pro Lys
           515                 520                 525
   Thr Met Pro Arg Ala Val Gly Ser Pro Leu Cys Val Pro Ala Arg Arg
       530                 535                 540
   Arg Ser Ser Glu Pro Arg Lys Asn Ser Ala Met Arg Thr Glu Pro Thr
   545                 550                 555                 560
   Arg Glu Ser Asn Arg Lys Thr Asp Ser Arg Leu Val Asp Val Ser Pro
                   565                 570                 575
   Asp Arg Gly Ser Pro Pro Ser Arg Val Pro Gln Ala Leu Asn Phe Ser
               580                 585                 590
   Pro Glu Glu Ser Asp Ser Thr Phe Ser Lys Ser Thr Ala Thr Glu Val
           595                 600                 605
   Ala Arg Glu Glu Ala Lys Pro Gly Gly Asp Ala Ala Pro Ser Glu Ala
       610                 615                 620
   Leu Leu Val Asp Ile Lys Leu Glu Pro Leu Ala Val Thr Pro Asp Ala
   625                 630                 635                 640
   Ala Ser Gln Pro Leu Ile Asp Leu Pro Leu Ile Asp Phe Cys Asp Thr
                   645                 650                 655
   Pro Glu Ala His Val Ala Val Gly Ser Glu Ser Arg Pro Leu Ile Asp
               660                 665                 670
   Leu Met Thr Asn Thr Pro Asp Met Asn Lys Asn Val Ala Lys Pro Ser
           675                 680                 685
   Pro Val Val Gly Gln Leu Ile Asp Leu Ser Ser Pro Leu Ile Gln Leu
       690                 695                 700
   Ser Pro Glu Ala Asp Lys Glu Asn Val Asp Ser Pro Leu Leu Lys Phe
   705                 710                 715                 720
<210> 27
   <211> 21
   <212> RNA
   <213> Artificial sequence
<220>
   <223> SiRNA-1(44-64) oligonucleotide (sense) for human UBE2s mRNA
<400> 27
   ccggccggcc gcagccauga a 21
<210> 28
   <211> 21
   <212> RNA
   <213> Artificial Sequence
<220>
   <223> siRNA-1(146-166) oligonucleotide (sense) for human UBE2s mRNA
<400> 28
   uggcaucaag gucuuuccca **a** 21
<210> 29
   <211> 19
   <212> RNA
   <213> Artificial Sequence
<220>
   <223> siRNA-2 oligonucleotide (sense) for human UBE2S mRNA
<400> 29
   ggagaacuac gaggaguau 19
<210> 30
   <211> 19
   <212> RNA
   <213> Artificial Sequence
<220>
   <223> siRNA-3 oligonucleotide (sense) for human UBE2s mRNA
<400> 30
   uggcgagcgc gauaagaag 19
<210> 31
   <211> 21
   <212> RNA
   <213> Artificial Sequence
<220>
   <223> siRNA-1(897-917) oligonucleotide (sense) for human RFC4 mRNA
<400> 31
   agggaauagc uuaucuuguu a 21
<210> 32
   <211> 21
   <212> RNA
   <213> Artificial sequence
<220>
   <223> siRNA-1(189-209) oligonucleotide (sense) for human RFC4 mRNA
<400> 32
   cugcacgaga agccaggcua a 21
<210> 33
   <211> 19
   <212> RNA
   <213> Artificial Sequence
<220>
   <223> siRNA-2 oligonucleotide (sense) for human RFC4 mRNA
<400> 33
   ccgauucugu cuuaucugu 19
<210> 34
   <211> 19
   <212> RNA
   <213> Artificial Sequence
<220>
   <223> siRNA-3 oligonucleotide (sense) for human RFC4 mRNA
<400> 34
   ggguaauacc agcugagaa 19
<210> 35
   <211> 21
   <212> RNA
   <213> Artificial Sequence
<220>
   <223> siRNA-1(2003-2023) oligonucleotide (sense) for human PTGES2 mRNA
<400> 35
   cugggacaug uuugcaauaa a 21
<210> 36
   <211> 21
   <212> RNA
   <213> Artificial Sequence
<220>
   <223> siRNA-1(1105-1125) oligonucleotide (sense) for human PTGES2 mRNA
<400> 36
   cuggcucaug cucaacgaga a 21
<210> 37
   <211> 19
   <212> RNA
   <213> Artificial Sequence
<220>
   <223> siRNA-2 oligonucleotide (sense) for human PTGES2 mRNA
<400> 37
   aggagaaagc ucgcaacaa 19
<210> 38
   <211> 19
   <212> RNA
   <213> Artificial Sequence
<220>
   <223> siRNA-3 oligonucleotide (sense) for human PTGES2 mRNA
<400> 38
   ccgaguucgg caauaagua 19
<210> 39
   <211> 21
   <212> RNA
   <213> Artificial Sequence
<220>
   <223> siRNA-1(1538-1558) oiigonucleotide (sense) for human MAF1 mRNA
<400> 39
   cagcuggacc gcagaguuua u 21
<210> 40
   <211> 21
   <212> RNA
   <213> Artificial Sequence
<220>
   <223> siRNA-1(1031-1051) oligonucleotide (sense) for human MAF1 mRNA
<400> 40
   uagccucugg uccuucaacu a 21
<210> 41
   <211> 19
   <212> RNA
   <213> Artificial Sequence
<220>
   <223> siRNA-2 oligonucleotide (sense) for human MAF1 mRNA
<400> 41
   acgacaaaca cauguucaa 19
<210> 42
   <211> 19
   <212> RNA
   <213> Artificial Sequence
<220>
   <223> siRNA-3 oligonucleotide (sense) for human MAF1 mRNA
<400> 42
   gccuuagcug gguggugaa 19
<210> 43
   <211> 21
   <212> RNA
   <213> Artificial Sequence
<220>
   <223> siRNA-1(626-646) oligonucleotide (sense) for human ACVR2B mRNA
<400> 43
   cagcucauga augacuuugu a 21
<210> 44
   <211> 21
   <212> RNA
   <213> Artificial Sequence
<220>
   <223> siRNA-1(208-228) oligonucleotide (sense) for human ACVR2B mRNA
<400> 44
   caccaucgag cucgugaaga a 21
<210> 45
   <211> 19
   <212> RNA
   <213> Artificial Sequence
<220>
   <223> siRNA-2 oligonucleotide (sense) for human ACVR2B mRNA
<400> 45
   ggcagaguga acgggagau 19
<210> 46
   <211> 19
   <212> RNA
   <213> Artificial Sequence
<220>
   <223> siRNA-3 oligonucleotide (sense) for human ACVR2B mRNA
<400> 46
   ggaacaucau cacauggaa 19
<210> 47
   <211> 21
   <212> RNA
   <213> Artificial Sequence
<220>
   <223> siRNA-1(754-774) oligonucleotide (sense) for human LOC151194 mRNA
<400> 47
   aagguucacu acgauccuga a 21
<210> 48
   <211> 21
   <212> RNA
   <213> Artificial Sequence
<220>
   <223> siRNA-1(1068-1088) oligonucleotide (sense) for human LOC151194 mRNA
<400> 48
   ucgauuuaug cuauuugugu a 21
<210> 49
   <211> 19
   <212> RNA
   <213> Artificial Sequence
<220>
   <223> siRNA-2 oligonucleotide (sense) for human LOC151194 mRNA
<400> 49
   ggaauuuggg uugcagaaa 19
<210> 50
   <211> 19
   <212> RNA
   <213> Artificial Sequence
<220>
   <223> siRNA-3 oligonucleotide (sense) for human LOC151194 mRNA
<400> 50
   ccagaaggag gacuuauaa 19
<210> 51
   <211> 21
   <212> RNA
   <213> Artificial sequence
<220>
   <223> siRNA-1(705-725) oligonucleotide (sense) for human LTB4DH mRNA
<400> 51
   cacuguuauc ggccagauga a 21
<210> 52
   <211> 21
   <212> RNA
   <213> Artificial Sequence
<220>
   <223> siRNA-1(588-608) oligonucleotide (sense) for human LTB4DH mRNA
<400> 52
   uggauuugau gucgucuuua a 21
<210> 53
   <211> 19
   <212> RNA
   <213> Artificial Sequence
<220>
   <223> siRNA-2 oligonucleotide (sense) for human LTB4DH mRNA
<400> 53
   gccaaaagau ugaaggaag 19
<210> 54
   <211> 19
   <212> RNA
   <213> Artificial Sequence
<220>
   <223> siRNA-3 oligonucleotide (sense) for human LTB4DH mRNA
<400> 54
   ccugaugguu augauuguu 19
<210> 55
   <211> 21
   <212> RNA
   <213> Artificial Sequence
<220>
   <223> siRNA-1(145-165□joligonucleotide (sense) for human DPM2 mRNA
<400> 55
   cagcauguca uccacaagua u 21
<210> 56
   <211> 21
   <212> RNA
   <213> Artificial Sequence
<220>
   <223> siRNA-1(84-104□joligonucleotide (sense) for human DPM2 mRNA
<400> 56
   uagccugauc aucuucaccu a 21
<210> 57
   <211> 19
   <212> RNA
   <213> Artificial sequence
<220>
   <223> SiRNA-2 oligonucleotide (sense) for human DPM2 mRNA
<400> 57
   gggugauucu cuugccauu 19
<210> 58
   <211> 19
   <212> RNA
   <213> Artificial sequence
<220>
   <223> siRNA-3 oligonucleotide (sense) for human DPM2 mRNA
<400> 58
   uguuuguggg acuguucau 19
<210> 59
   <211> 21
   <212> RNA
   <213> Artificial Sequence
<220>
   <223> siRNA-1(870-890□joligonucleotide (sense) for human SEPX1 mRNA
<400> 59
   cagacucucg uccucaccga a 21
<210> 60
   <211> 21
   <212> RNA
   <213> Artificial Sequence
<220>
   <223> siRNA-1(794-814□joligonucleotide (sense) for human SEPX1 mRNA
<400> 60
   cugaaugacg uuacacccuc a 21
<210> 61
   <211> 19
   <212> RNA
   <213> Artificial sequence
<220>
   <223> siRNA-2 oligonucleotide (sense) for human SEPX1 mRNA
<400> 61
   gggcgagguu uuccagaau 19
<210> 62
   <211> 19
   <212> RNA
   <213> Artificial Sequence
<220>
   <223> siRNA-3oligonucleotide (sense) for human SEPx1 mRNA
<400> 62
   ucacuuugaa ccuggcguu 19
<210> 63
   <211> 21
   <212> RNA
   <213> Artificial Sequence
<220>
   <223> SiRNA-1(853-873□joligonucleotide (sense) for human PSMA3 mRNA
<400> 63
   ccaguccaau guaacuauuu a 21
<210> 64
   <211> 21
   <212> RNA
   <213> Artificial Sequence
<220>
   <223> siRNA-1(686-706□joligonucleotide (sense) for human PSMA3 mRNA
<400> 64
   cucagcuggg uuggugaauu a 21
<210> 65
   <211> 19
   <212> RNA
   <213> Artificial Sequence
<220>
   <223> siRNA-2 oligonucleotide (sense) for human PSMA3 mRNA
<400> 65
   uggcagaugc ucguucuuu 19
<210> 66
   <211> 19
   <212> RNA
   <213> Artificial Sequence
<220>
   <223> siRNA-3 oligonucleotide (sense) for human PSMA3 mRNA
<400> 66
   gguguuucau acgguuauu 19
<210> 67
   <211> 21
   <212> RNA
   <213> Artificial Sequence
<220>
   <223> siRNA-1(546-566□joligonucleotide (sense) for human CHCHD3 mRNA
<400> 67
   caggaugcau ucuacaaaga a 21
<210> 68
   <211> 21
   <212> RNA
   <213> Artificial Sequence
<220>
   <223> siRNA-1(1450-1470□joligonucleotide (sense) for human CHCHD3 mRNA
<400> 68
   cuggaauaau guuuaugauu a 21
<210> 69
   <211> 19
   <212> RNA
   <213> Artificial Sequence
<220>
   <223> siRNA-2 oligonucleotide (sense) for human CHCHD3 mRNA
<400> 69
   cgaagaucag aaacgacua 19
<210> 70
   <211> 19
   <212> RNA
   <213> Artificial Sequence
<220>
   <223> siRNA-3 oligonucleotide (sense) for human CHCHD3 mRNA
<400> 70
   gagaaagacc gagugcuaa 19
<210> 71
   <211> 21
   <212> RNA
   <213> Artificial Sequence
<220>
   <223> siRNA-1(540-560□joligonucleotide (sense) for human LSM3 mRNA
<400> 71
   uccaauaaau augaccacca a 21
<210> 72
   <211> 21
   <212> RNA
   <213> Artificial Sequence
<220>
   <223> siRNA-1(37-57□joligonucleotide (sense) for human LSM3 mRNA
<400> 72
   acgacguaga ccagcaacaa a 21
<210> 73
   <211> 19
   <212> RNA
   <213> Artificial Sequence
<220>
   <223> siRNA-2 oligonucleotide (sense) for human LSM3 mRNA
<400> 73
   gacguagacc agcaacaaa 19
<210> 74
   <211> 19
   <212> RNA
   <213> Artificial Sequence
<220>
   <223> siRNA-3 oligonucleotide (sense) for human LSM3 mRNA
<400> 74
   acgaaacgga auauuccaa 19
<210> 75
   <211> 19
   <212> RNA
   <213> Artificial sequence
<220>
   <223> siRNA-2 oligonucleotide (sense) for human GTSE1 mRNA
<400> 75
   gggcaaagcu aaaucaagu 19
<210> 76
   <211> 19
   <212> RNA
   <213> Artificial sequence
<220>
   <223> siRNA-3 oligonucleotide (sense) for human GTSE1 mRNA
<400> 76
   ugacaaacac uccagacau 19

## Description

The present invention relates to the use of a gene (immortalization-determining gene) involved in the immortalization of human cancer cells, and methods useful for determining an immortalized cancer cell in cancer cells. More specifically, the present invention relates to a method for discriminating an immortalized cancer cell using an immortalization-determining gene 11as an index, and the use of a reagent for the discrimination.

Patients are given a poor prognosis for human solid cancer unless radical surgery is performed. This mainly derives from the insufficient effect of the present chemotherapy. Since existing chemotherapy drugs basically influence the nucleic acid biosynthesis, DNA, microtubule, etc., as they inhibit the synthesis process from nucleic acid into protein, they inevitably damage, in addition to cancer cells, healthy cells that are in the process of cell growth. Recent research has identified a number of molecules that are specifically expressed in cancer cells, and inhibitors that target these specific molecules can have a minimal adverse reaction to healthy cells. This advantage allows for a greater dosage of the drug. However, in spite of its active development, such a molecule-targeted drug has the following major defect. The molecule-targeted drug does not necessarily promise a certain effect on a majority of cancer cases; the effect can greatly vary for each case. Moreover, it is usually difficult to precisely estimate the effect before the administration. Such a great variation in a molecule-targeted therapeutic drug in each case can derive from the fact that the drug is directed to the molecules of genes involved in carcinogenesis or metastasis. Carcinogenesis varies among different types of cancer and even within the same cancer; therefore, it is assumed that the effect of the targeted molecules on the cancer growth differs in each case. Conversely, the development of a molecule-targeted therapeutic drug that can exhibit a universal effect-on many cancers should become possible by identifying a molecule that has a universal and primary effect on various types of cancers.

In all vertebrates, including humans, each chromosome end contains a simple iteration sequence: "TTAGGG" (about 10kb in humans and several times greater in mice). This terminal sequence cannot be thoroughly reproduced at every DNA duplication upon cell division, and is gradually shortened (on the basis of about 200 bases in humans). This terminal portion is called a "telomere". When a telomere is shortened to a certain length (about 5kb in human noncancerous cells) by repeated cell divisions, the cell division eventually ceases. Cell division again becomes possible by cell canceration, but then, when the curtailment finally comes to the ultimate level (about 2kb), the cell dies. As disclosed in Non-Patent Document 1, reverse transcriptase "telomerase" serves to extend the shortened telomere to stabilize the telomere length, thereby prolonging the cell division limit. Because the telomerase is not expressed in a healthy cell, the cell division in a healthy cell is limited to several tens of times. However, the telomerase is highly expressed in both a productive cell and an immortalized cancer cell. All cancer cells used to be regarded as immortal; however, reports have revealed that some clinically evident cancers did not exhibit telomerase activities (see Non-Patent Documents 2, 3, etc.). Other reports have also revealed that the compulsory expression of telomerase enzyme component TERT in a noncancerous cell did not produce a cancer cell (see Non-Patent Document 4). Accordingly, at present, canceration and immortalization are recognized as different phenomena.
[Non-Patent Document 1] Harley CB, Mutation Res, 256: 271-82, 1991
[Non-Patent Document 2] Kim NW et al., Science 266: 2011-5, 1994
[Non-Patent Document 3] Hiyama K et al., J Natl Cancer Inst 87: 895-902, 1995
[Non-Patent Document 4] Morales CP et al., Nat Genet 21: 115-8, 1999
[Non-Patent Document 5] Shay JW, Bacchetti S, Eur J Cancer 33: 787-91, 1997
[Non-Patent Document 6] Hiyama K et al., J Immunol 155: 3711-5, 1995
[Non-Patent Document 7] Hiyama E et al., Int J Oncol 9: 453-8, 1996
[Non-Patent Document 8] Forsyth NR et al., Aging Cell 2: 235-43, 2003
[Non-Patent Document 9] Bodnar AG et al., Exp Cell Res 228: 58-64, 1996
[Non-Patent Document 10] Hiyama K et al., Int J Oncol 27: 87-95, 2005

In this context, Monte *et al.* (Monte, M. et al., The Journal of Biological Chemistry, 278(32), 2003, pp. 30356-30364) describes the effects of human GTSE-1 on p53 transcription function, protein levels and p53-dependent apoptosis. Further, US 2006/0074565 A1 describes methods, systems and compositions for predicting disease susceptibility in a patient. Furthermore, Collavin *et al.* (Collavin, L. et al., FEBS Letters, 481, 2000, pp. 57-62) describes B99-dependent cell cycle regulation in murine cells and the effect of p53 on said regulation. Finally, US 2005/0255487 A1 describes sequence specific gene silencing by the use of siRNA technology, as well as respective methods, compositions and kits.

The present invention relates to methods for determining an immortalized cancer cell in cancer cells, and the use of a reagent for the discrimination.

In order to develop a molecule-targeted therapeutic drug that is effective for cancer treatment, especially a drug that is expected to have a universal effect on many types of cancers, the inventors of the present invention focus their attention on another character of cancer, "immortalization", that is different from "canceration (malignant alteration)". Specifically, even if cancer cells are found, they will not spread or metastasize to kill the host if their limitless growth can be stopped. The suppression of the limitless growth also increases the possibility of the natural death of cancer cells given that the number of residual cancer cells is reduced by chemotherapy or nonradical surgery to some extent, because it is expected that the cancer cell division term would run out before the next cell growth begins. It has been reported that telomerase expression was seen in 80% or more of several thousands of clinical specimens (see Non-Patent Document 5); telomerase expression was confirmed in various types of cancers examined to date; and telomerase expression was seen in most of the cell strains that derive from human cancers (see Non-Patent Document 2). Based on these reports and the known fact that cancer cells containing telomerase expression will never naturally turn negative again, the immortalization phenomenon seems to exist universally and commonly in many cancer cells, in contrast to the wide variety of carcinogenesis. Accordingly, by targeting the molecule that regulates immortalization, it becomes possible to suppress infinite cancer growth with a universal effect on many types of cancers. Based on this principle, an anti-cancer strategy that directly focuses on telomerase has been tested.

However, the inventors of the present invention had already found that a precursor cell, lymphocyte, etc., of a regenerating organ exhibits telomerase activation even in a normal healthy cell (see Non-Patent Documents 6 and 7). It is also known that cell immortalization is not always induced by compulsive expression of TERT (telomerase enzyme component) in noncancerous cells (see Non-Patent Document 8); and that the healthy precursor cell or lymphocyte that expresses telomerase is not immortalized but has a prolonged life (see Non-Patent Documents 6 and 9). Accordingly, such exhibition of telomerase activation in healthy cells is obviously merely telomerase expression rather than cell immortalization. More specifically, though human cell immortalization substantially requires the activation of a telomerase enzyme that serves to extend the telomere in the chromosome terminus, the immortalization is not induced only by the telomerase activation, but also by the presence of specific molecules for immortalization. The molecules seem to function particularly in cancer cells. In fact, the inventors of the present invention confirmed that a gene that specifically undergoes expression and change in a clone, which has been immortalized by compulsive telomerase expression through the introduction of TERT into healthy cells, completely differs from the reported expression and change seen in cancer cells (see Non-Patent Document 10). This is another fact indicating that the gene involved in the immortalization of healthy cells differs from the gene involved in the immortalization of cancer cells. If the drug is directed to telomerase, it suppresses the division ability of the lymphocyte, blood precursor cell, gut mucosa crypt cell, etc., and thereby tends to damage immunity, hematogenous ability, and gastrointestinal function, which is known to be a fatal harm in cancer treatment. However, if the drug is directed to a immortalization-determining factor that works only in cancer cells, it becomes possible to block the specific infinite proliferation of cancer cells without damaging the healthy trunk/precursor cells having telomerase activities.

Based on this theory, the inventors of the present invention conducted an intensive study to discover genes that are universally involved in the immortalization of human cancer cells. As a result of the research, the inventors of the present invention eventually realized that those genes are not involved in the lifetime extension of cells resulting from telomerase activation in healthy cells, but is specifically involved in the constant proliferation of cancer cells. The inventors then identified thirteen immortalization-determining genes (Table 1) in the following manner. First, the genes that are universally expressed and strengthened in various types of cancers were extracted by microarray assay. Among the obtained genes, thirteen kinds of genes were selected on the basis of their expression in a clinical cancer organ consisting of immortalized cancer cells being stronger than the expression of a cancerous tissue comprised of non-immortalized cancer cells and that undergoing no expression or augmentation in healthy cells expressed by the introduction of telomerase. Then, the inventors confirmed that suppressing the expression of the thirteen genes using siRNA suppresses the proliferation of immortalized cancer cells.

The inventors of the present invention further conducted more detailed research based on the foregoing theory, and eventually completed the present invention. Specifically, the present invention includes the embodiments characterised in the claims. RNA prepared from a biological sample that is extracted from a test subject and that may contain a cancer cell, or a derivative of the RNA;
(2) measuring an amount of the RNA or the derivative bonded with the marker gene, using the marker gene as an index; and
(3) comparing the amount of RNA or the derivative thereof found in Step (2) (generically referred to as "an RNA amount", hereinafter) with an amount (generically referred to as "a comparative RNA amount", hereinafter) of a corresponding RNA or a derivative thereof in a non-immortalized healthy or cancer cell.

Item 4. The method for determining an immortalized cancer cell according to Item 3, further comprising the step of:
(4) determining that the cancer cell of the test subject is immortalized when the RNA amount found in Step (2) is higher than the comparative RNA amount, and determining that the cancer cell of the test subject is not immortalized when the RNA amount found in Step (2) is not higher than the comparative RNA amount.

Item 5. An antibody for recognizing a polypeptide having any one of amino acid sequences represented by SEQ ID Nos. 14 to 26. The antibody is suitable for use in determining an immortalized cancer cell.

Item 6. A method for determining an immortalized cancer cell, comprising the step of:
(1') bonding a protein-containing fraction containing a polypeptide prepared from a biological sample that is extracted from a test subject and that may contain a cancer cell with the antibody according to Item 5;
(2') measuring the amount of the polypeptide that is bonded with the antibody, Using the antibody as an index; and
(3') comparing the amount (generically referred to as "a polypeptide amount", hereinafter) of the polypeptide found in Step (2') with the amount (generically referred to as "a comparative polypeptide amount", hereinafter) of a corresponding polypeptide in a non-immortalized healthy or cancer cell.

Item 7. The method for determining an immortalized cancer cell according to Item 6, further comprising the step of:
(4') determining that the cancer cell of the test subject is immortalized when the polypeptide amount found in Step (2') is higher than the comparative polypeptide amount, and determining that the cancer cell of the test subject is not immortalized when the polypeptide amount found in Step (2') is not higher than the comparative polypeptide amount.

Item 8. An immortalized cancer cell determining reagent kit including at least one member selected from the group consisting of the marker gene according to Item 1 and the antibody according to Item 5.

Item 9. A method for screening a substance for suppressing proliferation of immortalized cancer cells, comprising the steps of:
(A) bringing a test material into contact with a cell that can express one of the immortalization-determining genes represented by SEQ ID Nos. 1 to 13;
(B) measuring an expression amount of the immortalization-determining gene in the cell brought into contact with the test material;
(C) comparing the expression amount of the immortalization-determining gene found in Step (B) with an expression amount (generically referred to as "a comparative expression amount", hereinafter) of an immortalization-determining gene in a cell not in contact with the test material; and
(D) selecting the test material as a candidate substance for suppressing proliferation of immortalized cancer cells when the expression amount found in Step (B) is lower than the comparative expression amount.

Item 10. A method for screening a substance for suppressing proliferation of immortalized cancer cells, comprising the steps of:
(A') bringing a test material into contact with a polypeptide having one of the amino acid sequences represented by SEQ ID Nos. 14 to 26;
(B') measuring activity of the polypeptide having one of the amino acid sequences represented by SEQ ID Nos. 14 to 26 brought into contact with the test material;
(C') determining the degree of the activity of the polypeptide found in Step (B') by comparing the activity of the polypeptide found in Step (B') with activity (generically referred to as "a comparative activity", hereinafter) of a polypeptide not in contact with the test material; and
(D') selecting the test material as a candidate substance for suppressing proliferation of immortalized cancer cells when the activity of the polypeptide found in Step (B') is lower than the comparative activity.

Item 11. An immortalized cancer cell growth inhibitor containing, as an active ingredient, a polynucleotide having a base sequence of at least 15 bases that specifically hybridizes with a continuous base sequence of at least 15 bases within any one of base sequences represented by SEQ ID Nos. 1 to 13.

. ' Item 12. The immortalized cancer cell growth inhibitor according to Item 11, wherein the polynucleotides has one of base sequences represented by SEQ ID No. 27 to 76.

Item 13. A method of anticancer treatment comprising the step of administering the immortalized cancer cell growth inhibitor according to Item 11 or 12 to cancer cells of a patient.

Item 14. Use of a polynucleotide having a base sequence of at

The present invention discovered genes that universally determine the immortalization of many different cancer cells. The genes specifically exhibit greater expression in immortalized cancer cells in a plurality of cancers. The present invention provides a method for discriminating immortalized cancer cells in cancer cells, and the use of a reagent for the discrimination method, which allows for measuring the expression level of the immortalization-determining genes. Referring to the measured level, it becomes possible to distinguish the immortalized cancer cells from finite cells. This differentiation was never possible in conventional general pathological diagnoses. The differentiation thus has great potential as a clinical application that is useful for early diagnosis, new treatment options, prognostification, and the like of various cancers.

Further, with a screening process targeting an immortalization-determining gene of the present invention, an active ingredient of an anticancer drug that specifically suppresses the proliferation of immortalized cancer cells is obtained.

### (I) Immortalization-determining gene

In the present invention, the term "non-immortalized cancer cells" refers to, among general cancer cells, a finite cell having (1) negative telomerase-activation, (2) a reduced telomere length, and (3) undetectable expression of a telomerase protein. Further, in the present invention, the term "immortalized cancer cell" refers to a cell that lacks at least one of the aforementioned conditions; more specifically, a cell having telomerase activation, a cell having an extended telomere length, and/or a cell detectable expression of a telomerase protein. Such an "immortalized cancer cell" is capable of limitless cell proliferation.

In the present invention, the term "immortalization-determining gene" refers to a gene that determines the immortalization of cancer cells.

The present invention does not particularly limit the type of cancer cell as a target, and includes cells that derive from, for example, lung cancer, breast cancer, esophagus cancer, head and neck cancer, gastric cancer, colon cancer, rectal cancer, liver cancer, gallbladder and bile duct cancer, pancreatic cancer, bladder cancer, prostatic cancer, or uterine cervix carcinoma.

Generally the immortalization-determining genes are, namely, (1) UBE2S gene, (2) RFC4 gene, (3) PTGES2 gene, (4) MAF1 gene, (5) ACVR2B gene, (6) FAM119A gene, (7) LTB4DH gene, (8) DPM2 gene, (9) SEPX1 gene, (10) PSMA3 gene, (11) CHCHD3 gene, (12) LSM3 gene, and (13) GTSE1 gene. Table 1 shows the GenBank accession number, formal gene name, and gene symbol of the foregoing genes, and the SEQ ID No.s indicating the base sequences of the genes. In Table 1, the gene name, gene symbol, etc., are based on NCBI data disclosed on the Internet (http://www.ncbi.nlm.nih.gov/).

**Table 1**

| SEQ ID No. | GenBank Accession No. | Gene Name | Gene Symbol |
|---|---|---|---|
| 1 | NM_014501 | ubiquitin-conjugating enzyme E2S | *UBE2S* |
| 2 | NM_M2916 | replication factor C (activator 1) 4, 37kDa | *RFC4* |
| 3 | NM_025072 | prostaglandin E synthase 2 | *PIGES2* |
| 4 | NM_032272 | MAF1 homolog (S. cerevisiae) | *MAF1* |
| 5 | NM_001106 | activin A receptor, type IIB | *ACVR2B* |
| 6 | NM_145280 | family with sequence similarity 119, member A | *FAM119A* |
| 7 | NM_012212 (D49387, BQ214856) | leukotriene B4 12-hydroxydehydrogenase | *LTB4LDH* |
| 8 | NM_003863 | dolichyl-phosphate mannosyltransferase polypeptide 2, regulatory subunit | *DPM2* |
| 9 | NM_016332 | selenoprotein X, 1 | *SEPX1* |
| 10 | NM_002788 | proteasome (prosome, macropain) subunit, alpha type, 3 | *PSMA3* |
| 11 | NM_017812 | coiled-coil-helix-coiled-coil-helix domain containing 3 | *CHCHD3* |
| 12 | NM_014463 | LSM3 homolog, U6 small nuclear RNA associated (S. cerevisiae) | *LSM3* |
| 13 | NM_016426 | G-2 and S-phase expressed 1 | *GTSE1* |

As shown in Example 1, the above-listed immortalization-determining genes are all highly expressed in immortalized cancer cells derived from a plurality of cancers, including lung cancer, esophagus cancer, and breast cancer. As shown in Example 2, when the expression of these thirteen genes was suppressed by siRNA in the immortalized cancer cells, in every case, the proliferation of immortalized cancer cells was suppressed. This shows that the thirteen immortalization-determining genes determine the immortalization of cancer cells (immortalization-determining gene).

### (II) Reagent for judging the immortalization of cancer cells (marker gene)

As described above, the immortalization-determining genes ((1) to (13)) having base sequences represented by SEQ ID No.s 1 to 13 are all highly expressed specifically in immortalized cancer cells. Therefore, using the thirteen genes as marker genes, the immortality/mortality of cancer cells can be judged.

The present invention provides the use of an immortalization-determining marker gene (abbreviated as "marker gene" in the present invention) as a tool (reagent) that is suitably used to judge the immortalization of cancer cells. The marker gene is designed as a polynucleotide of at least 15 bases that specifically hybridizes with a continuous base sequence of at least 15 bases among the base sequence of the immortalization-determining gene represented by the SEQ ID No. 13. The marker gene of the present invention may have an arbitrary form according to the object, and may be any of single-strand DNA, single-strand RNA, double-strand DNA, double-strand RNA, and DNA:RNA hybrid.

The marker gene contains a probe or primer useful for detecting the occurrence of expression of an immortalization-determining gene and/or the level (expression level) in cancer cells to allow for the discrimination of immortalized cancer cells. Further, the probe or primer is also useful as a tool (detection reagent) for detecting the expression variation of an immortalization-determining gene in the screening of a material that suppresses the proliferation of the immortalized cancer cells.

### (II-1) Probe

Generally, the immortalization of cancer cells is discriminated by detecting at least one of the above-mentioned immortalization-determining genes: (1) UBE2S gene, (2) RFC4 gene, (3) PTGES2 gene, and (4) MAF1 gene, (5) ACVR2B gene, (6) FAM119A gene, (7) LTB4DH gene, (8) DPM2 gene, (9) SEPX1 gene, (10) PSMA3 gene, (11) CHCHD3 gene, (12) LSM3 gene, and (13) GTSE1 gene, which are highly expressed specifically in the immortalized cancer cells. .

According to the present invention, the GTSE1 gene is detected.

The detection of the immortalization-determining genes uses, as probes, polynucleotides, each of which specifically hybridizes with the base sequence of one of the aforementioned immortalization-determining genes. In the present invention, an oligonucleotide having plural base sequences is included in the range of "polynucleotide".

Each polynucleotide is designed to specifically hybridize with a continuous base sequence of one of the immortalization-determining genes. More specifically, for each of the aforementioned immortalization-determining genes, a corresponding polynucleotide specifically hybridizes with a continuous base of at least 15 bases, preferably 20 bases, and more preferably 30 bases to the total base length of the immortalization-determining gene. Accordingly, each polynucleotide has a base length corresponding to the defined base length.

Throughout the present specification and claims, "specific hybridization" refers to a hybridization that forms only a specific hybrid and does not form a nonspecific hybrid under a stringent hybridization condition. The stringent hybridization condition is determined in the usual manner, for example, based on the melting temperature (Tm) of the nucleic acid at which the hybrid is formed. A typical cleaning condition to ensure the hybridization condition is approximately "1xSSC, 0.1%SDS, 37°C", more strictly "0.5xSSC, 0.1%SDS, 42°C", and yet more strictly "0.1xSSC, 0.1%SDS, 65°C".

The polynucleotide (probe) preferably has a base sequence that is complementary to a continuous base sequence of at least 15 bases of the immortalization-determining gene; however, the probe is not always required to be fully complementary to said continuous base sequence as long as the specific hybridization is possible. The polynucleotide has an identity of at least 70%, preferably not less than 80%, more preferably not less than 90%, yet more preferably not less than 95%, and still more preferably not less than 98%, with respect to either the polynucleotide having a continuous base of at least 15 bases within a base sequence of the immortalization-determining gene or the complementary polynucleotide thereof. The identity of each base sequence can be calculated by way of an identity search, alignment program, BLAST, FASTA, ClustalW, or the like.

Examples of probes suitable for the present invention include oligonucleotides and polynucleotides having a continuous base sequence of 15 bases, preferably 20 bases, and more preferably 30 bases to the total base length of the immortalization-determining gene that hybridizes with the polynucleotide (1) (however, when the polynucleotide is RNA, the base "t" in the sequence is replaced with "u").

A polynucleotide with a continuous base sequence having at least 15 bases from the base sequence (SEQ ID No.13) of the GTSE1 gene.

This polynucleotide (probe) can be produced by a known method, for example, using a commercially available nucleotide synthesizer, based on the target base sequence of the immortalization-determining gene. The polynucleotide (probe) can also be produced by the PCR method using the target base sequence of the immortalization-determining gene as a template.

More preferably, to simplify detection of the immortalization-determining genes, each probe may be labeled with a radioactive material, a fluorescent material, a chemical luminescent substance or an enzyme (described later in greater detail).

Further, each probe may be immobilized in an arbitrary solid phase before use.

Accordingly, the probes suitable for the present invention include a probe in which an aforementioned polynucleotide is immobilized in a solid phase (for example, an immobilized probe, including a gene chip, a cDNA microarray, an oligo DNA array, or a membrane filter.). This probe is suitable for a DNA chip for use in immortalization-determining gene detection, or more specifically, for use in immortalized cancer cell detection.

The solid phase of the immobilized probe (polynucleotide) is not particularly limited as long as it immobilizes the polynucleotide. Examples of solid phases include a glass plate, a nylon membrane, micro beads, a silicon chip, a capillary, and other substrates. The polynucleotide can be immobilized in the solid phase by disposing a synthetic polynucleotide in a solid phase, or by synthesizing a target polynucleotide in a solid phase. The immobilization is a well known technology in the present field, and any suitable method can be used according to the type of immobilized probe. For example, a commercially available spotter (manufactured by Cosmobio Co., Ltd., etc.) is often used to form a DNA microarray. (For example, polynucleotide in situ synthesis using photolithographic technology (AFFYMETRIX CO.), or inkjet technology (ROSETTA INPHARMATICS CO.)

### (II-2) Primer

The present invention provides a polynucleotide as a primer that serves as a marker gene to specifically amplify a base sequence region of each immortalization-determining gene.

The polynucleotide is typically a polynucleotide that has a continuous base sequence of at least 15 bases, preferably 15 to 100 bases, more preferably 15 to 50 bases, yet more preferably 15 to 35 bases, and that specifically hybridizes with a part of the polynucleotide (1) (however, when the polynucleotide is RNA, the base "t" in the sequence is replaced with "u"), so as to amplify part or all of the polynucleotide.

A polynucleotide with a continuous base sequence having at least 15 bases from the base sequence (SEQ ID No.13) of the GTSE1 gene.

As with the probe, the defined polynucleotide (primer) preferably has a base sequence complementary to the continuous base sequence of at least 15 bases in the immortalization-determining gene; however, as long as a specific hybridization is possible, it is not required to be totally complementary. The polynucleotide preferably has an identity of at least 70%, preferably not less than 80%, more preferably not less than 90%, yet more preferably not less than 95%, and still more preferably not less than 98%, with respect to either the polynucleotide having a continuous base of at least 15 bases within a sequence of the immortalization-determining gene or the complementary polynucleotide thereof.

As with the probe, this polynucleotide (primer) can be produced by a known method, for example, using a commercially available nucleotide synthesizer, based on the target base sequence of the immortalization-determining gene.

### (II-3) Marker

The marker gene (probe or primer) according to the present invention may be a product obtained by adding a marker suitable for detecting an immortalization-determining gene, such as a fluorescent coloring material, an enzyme, a protein, a radioisotope, a chemical luminescent substance, or a biotin, to the aforementioned polynucleotide.

For example, a suitable example of the marker of the present invention made of a fluorescent coloring material, a radioisotope, or a chemical luminescent substance is a general marker capable of labeling a nucleotide to detect or determine the quantity of nucleic acids. Examples of fluorescent coloring materials include, but are not limited to, HEX (4, 7, 2', 4', 5', 7'-hexachloro-6-carboxylfluorescein, green fluorescent coloring material), fluoresceine, NED (product name; Applied Biosystems Inc., yellow fluorescent coloring material), 6-FAM (product name; Applied Biosystems Inc, yellowish green fluorescent coloring material), and rhodamine or its derivative (e.g., tetra methyl rhodamine (TMR)). Fluorescent color labeling on a nucleotide is performed by a suitable method among the known labeling methods (see Nature Biotechnology, 14, 303-308 (1996)). A commercially available fluorescent label kit (for example, oligonucleotide ECL 3'-oligo labeling system produced by Amersham Pharmacia Biotech) can also be used.

The above-described marker gene (an unlabeled or labeled probe or primer) may be used as an immortalization-determining gene detection reagent, in other words, an immortalized cancer cell detection reagent.

### (II-4) Immortalized cancer cell detecting reagent kit

The present invention can be used in the context of an immortalized cancer cell detecting reagent (marker gene (an unlabeled or labeled probe or primer)) kit. The kit includes at least one of the above mentioned unlabeled or labeled polynucleotides used as probes or primers (the polynucleotide may be immobilized in a solid phase). In addition to the marker gene (probe or primer), the reagent kit of the present invention may include other reagents or tools as required during the actual performance (described later) of the method of the present invention, such as a hybridization reagent, a probe labeling material, a label detecting agent, a buffer solution etc.

### (III) Determination method for immortalized cancer cells

(III-1) The marker gene (probe or primer) of the present invention enables measurement of the expression amount of the immortalization-determining gene (marker gene) in the cancer cells obtained from a test subject; further, the measured expression amount enables judgment as to whether the cancer cells are "immortalized cancer cells" or "non-immortalized cancer cells". Existing pathological diagnoses are incapable of determining immortalized cancer cells; however, the immortalized cancer cell determination method of the present invention is capable of detecting the early-stage of incipient immortalized cancer cells even before they develop, thereby allowing for rapid provision of an appropriate cancer treatment for each individual.

In this case, the primer and the probe of the present invention are used as a primer for specifically recognizing and amplifying an RNA resulting from the expression of an immortalization-determining gene or a polynucleotide produced from the RNA (e.g., cDNA), and as a probe for specifically detecting an RNA, or a polynucleotide (e.g., cDNA, DNA amplified from RNA or cDNA) that derives from an RNA, respectively.

More specifically, the marker gene (primer or probe) of the present invention may be used as a primer and/or a probe for specifically detecting an immortalization-determining gene, using a known method for specifically detecting a specific gene, such as the northern blotting method, an RT-PCR method, an in situ hybridization method, or the like. Further, detection and/or quantity determination of the expression amount of the immortalization-determining gene in the cancer cell may be performed using a DNA chip. In this case, the probe of the present invention can be used as a probe for the DNA chip.

For example, the measurement of the expression amount of the immortalization-determining gene by way of the northern blotting method may be performed with the probe of the present invention, or more preferably, by the probe labeled with a radioisotope, a fluorescent material, or a chemical luminescent substance. More specifically, RNA extracted from a cancer cell of a test subject is transferred to a nylon membrane or the like, and the labeled probe is hybridized with the RNA. Then, the amount of the resulting double-strand of the probe and the RNA is measured based on a signal given by the marker of the probe. The detection of the signal may be carried out by an appropriate known tool according to the marker of the probe, such as a radiation detector, or a fluorescence detector. Detection may also be performed with a commercially available northern blotting reagent kit in accordance with the protocol of the kit.

Measurement of the immortalization-determining gene expression amount using the RT-PCR method may also be performed using a primer of the present invention, preferably, a primer labeled with a radioisotope, a fluorescent material, a chemical luminescent substance, or the like. More specifically, a pair of marked primers is hybridized with a template cDNA prepared from RNA deriving from a cancer cell of a test subject, and PCR is performed in the general manner. Then, the amount of the resulting amplified double-strand DNA is measured based on the signal given by the marker of the primer. The detection of the signal may be carried out by an appropriate known tool, such as a radiation detector, or a fluorescence detector. The detection may also be performed with a commercially available RT-PCR reagent kit in accordance with the protocol of the kit.

Measurement of the expression amount of an immortalization-determining gene using a DNA chip assay may also be carried out using a probe of the present invention, preferably, a probe labeled with a radioisotope, a fluorescent material, etc. More specifically, a DNA chip, in which a probe labeled with a radioisotope, a fluorescent material, a chemical luminescent substance, or the like, is fixed to an appropriate carrier. Then, the DNA chip is hybridized with a marked DNA or RNA that is prepared from RNA deriving from a cancer cell of a test subject. Then, the amount of the resulting double-strand of the probe and marked DNA or RNA is measured based on the signal given by the marker of the probe. Detection of the signal may be carried out by an appropriate known tool such as a radiation detector or a fluorescence detector. This method may be performed with a commercially available DNA chip in which DNA corresponding to a probe of the present invention is fixed.

Measurement of the expression amount of immortalization-determining genes includes the following two steps (1) and (2).
(1) a step of bonding a probe or primer of the present invention to RNA prepared from a biological sample that may contain a cancer cell obtained from a test subject, or a derivative of the RNA; and
(2) a step of measuring the amount of the RNA or derivative thereof bonded with the probe or primer, using the probe or primer as an index.

In Step (1), the biological sample can be any sample extracted from a test subject, as long as it has the potential to contain a cancer cell. Examples of the biological sample include body fluid (blood, urine, etc.), tissues, or the extracts thereof, and cultures of the obtained tissues. Extraction of the biological sample from the test object is performed by an appropriate method according to the type of biological sample or type of cancer. Preparation of the RNA from the biological sample is performed by a known method.

Throughout the specification and claims, the term "an RNA derivative" refers to a sample produced by RNA extracted from a biological sample. Examples of an RNA derivative include a complementary polynucleotide (cDNA) prepared by RNA transcription, and DNA amplified from the RNA or cDNA using PCR. The cDNA may be prepared using a known method, and the above-mentioned DNA is prepared by PCR using the primer of the present invention for the immortalization-determining gene. In addition, the probe or primer of the present invention used in Step (1) is preferably labeled with a marker, such as a radioisotope, a fluorescent material, or a chemical luminescent substance.

The amount of the RNA or the RNA derivative found in Step (2) depends on the expression amount of the immortalization-determining gene in the test subject. Accordingly, this amount of RNA or RNA derivative (hereinafter comprehensively referred to as "RNA amount") found in Step (2) is compared with the amount of the corresponding RNA or RNA derivative (hereinafter comprehensively referred to as "comparative RNA amount") in non-immortalized healthy or cancer cells to judge the immortalization of the cancer cells of the test subject. More specifically, if the RNA amount of the test subject found in Step (2) is greater than the comparative RNA amount, it is judged that the cancer cells of the test subject are immortalized. In the reverse case, it is judged that the cancer cells of the test subject are not immortalized.

Therefore, in addition to Steps (1) and (2), the immortalized cancer cell determination method of the present invention further includes the following Step (3), and preferably, also Step (4).
(3) a step of comparing the amount of RNA or the RNA derivative (RNA amount) found in Step (2) with the amount of the corresponding RNA or RNA derivative (comparative RNA amount) in a non-immortalized healthy or cancer cell; and
(4) a step of judging that the extracted cancer cell of the test subject is immortalized when the RNA amount in Step (2) is higher than the comparative RNA amount, and judging that the extracted cancer cell of the test subject is not immortalized when the RNA amount in Step (2) is not higher than the comparative RNA amount.

The RNA amount (comparative RNA amount) of the immortalization-determining gene in a non-immortalized healthy or cancer cell is calculated by first measuring the RNA amount of the immortalization-determining gene in a plurality of non-immortalized healthy or cancer cells under the same condition, and then finding a mean or intermediate value of the calculated values.

(III-2) Further, the immortalization of cancer cells can be judged based on the quantity of the expression product of the immortalization-determining gene of the present invention, in other words, based on the production quantity of a polypeptide (immortalization-determining polypeptide, hereinafter) encoded by the aforementioned gene. The production quantity of the immortalization-determining polypeptide can be measured using an antibody that recognizes the polypeptide.

The following are immortalization-determining polypeptides that can be generally used that correspond to the aforementioned immortalization-determining genes (1) to (13).
(14) UBE2S polypeptide: a polypeptide encoded by a UBE2S gene (1) having a base sequence represented by the SEQ ID No.1, namely, a polypeptide having an amino acid sequence represented by the SEQ ID No.14.
(15) RFC4 polypeptide: a polypeptide encoded by a RFC4 gene (2) having a base sequence represented by the SEQ ID No.2, namely, a polypeptide having an amino acid sequence represented by the SEQ ID No.15.
(16) PTGES2 polypeptide: a polypeptide encoded by a PTGES2 gene (3) having a base sequence represented by the SEQ ID No.3, namely, a polypeptide having an amino acid sequence represented by the SEQ ID No.16.
(17) MAF1 polypeptide: a polypeptide encoded by a MAF1 gene (4) having a base sequence represented by the SEQ ID No.4, namely, a polypeptide having an amino acid sequence represented by the SEQ ID No.17.
(18) ACVR2B polypeptide: a polypeptide encoded by an ACVR2B gene (5) having a base sequence represented by the SEQ ID No.5, namely, a polypeptide having an amino acid sequence represented by the SEQ ID No.18.
(19) FAM119A polypeptide: a polypeptide encoded by a FAM119A gene (6) having a base sequence represented by the SEQ ID No.6, namely, a polypeptide having an amino acid sequence represented by the SEQ ID No.19.
(20) LTB4DH polypeptide: a polypeptide encoded by a LTB4DH gene (7) having a base sequence represented by the SEQ ID No.7, namely, a polypeptide having an amino acid sequence represented by the SEQ ID No.20.
(21) DPM2 polypeptide: a polypeptide encoded by a DPM2 gene (8) having a base sequence represented by the SEQ ID No.8, namely, a polypeptide having an amino acid sequence represented by the SEQ ID No.21.
(22) SEPX1 polypeptide: a polypeptide encoded by a SEPX1 gene (9) having a base sequence represented by the SEQ ID No.9, namely, a polypeptide having an amino acid sequence represented by the SEQ ID No.22.
(23) PSMA3 polypeptide: a polypeptide encoded by a PSMA3 gene (10) having a base sequence represented by the SEQ ID No.10, namely, a polypeptide having an amino acid sequence represented by the SEQ ID No.23.
(24) CHCHD3 polypeptide: a polypeptide encoded by a CHCHD3 gene (11) having a base sequence represented by the SEQ ID No.11, namely, a polypeptide having an amino acid sequence represented by the SEQ ID No.24.
(25) LSM3 polypeptide: a polypeptide encoded by a LSM3 gene (12) having a base sequence represented by the SEQ ID No.12, namely, a polypeptide having an amino acid sequence represented by the SEQ ID No.25.
(26) GTSE1 polypeptide: a polypeptide encoded by a GTSE1 gene (13) having a base sequence represented by the SEQ ID No.13, namely, a polypeptide having an amino acid sequence represented by the SEQ ID No.26.

According to the present invention, the GTSE1 polypeptide is used.

The (14) UBE2S polypeptide is a publicly known polypeptide, and its production is also known, as disclosed in J. Biol. Chem. 267 (22), 15829-15835 (1992). The (15) RFC4 polypeptide is also publicly known as disclosed in Proc. Natl. Acad. Sci. U.S.A. 89 (12), 5211-5215 (1992). Similarly, the polypeptides are publicly known polypeptides, as the (16) PTGES2 polypeptide is disclosed in Biochem. Biophys. Res. Commun. 291 (4), 884-889 (2002); the (18) ACVR2B polypeptide is disclosed in Mol. Cell Biol. 16 (3), 1066-1073 (1996); the (20) LTB4DH polypeptide is disclosed in J. Biol. Chem. 271 (5), 2844-2850 (1996); the (21) DPM2 polypeptide is disclosed in EMBO J. 19 (11), 2475-2482 (2000); the (22) SEPX1 polypeptide is disclosed in J. Biol. Chem. 274 (48), 33888-33897 (1999); the (23) PSMA3 polypeptide is disclosed in Biochem. Biophys. Res. Commun. 207 (1), 318-323 (1995); the (25) LSM3 polypeptide is disclosed in EMBO J. 18 (12), 3451-3462 (1999); and the (26) GTSE1 polypeptide is disclosed in Gene 254 (1-2), 229-236 (2000).

The immortalization-determining polypeptides (14) to (26) may also be prepared by a process comprising cloning the corresponding immortalization-determining genes (1) to (13), ligating the genes into a vector plasmid, transforming the genes into host cells, such as E.coli, culturing the transformed cells, and isolating the polypeptides from the culture.

An antibody that can be used in the context of the present invention is not limited as long as it recognizes the immortalization-determining polypeptide, and may be a monoclonal antibody or a polyclonal antibody. The antibody may be an antibody prepared as an antibody against the immortalization-determining polypeptide as an immunizing antigen, or an antigen binding antibody with respect to a polypeptide having at least continuous 8 amino acids, preferably 15 amino acids, and more preferably 20 amino acids out of the amino acid sequences forming the immortalization-determining polypeptide. The polypeptide can be generally synthesized by a publicly known method based on the amino acid sequence of the immortalization-determining polypeptide of the present invention or the base sequence that encodes the amino acid. For example, chemical synthesis using an amino acid synthesizer or gene industrial synthesis can be employed.

The antibody can be produced by a general method (see, for example, Current protocols in Molecular Biology, edit. Ausubel et al. (1987), Publish. John Wiley and Sons. Section 11.12-11.13). For example, a polyclonal antibody can be produced by using a polypeptide obtained by expressing a polypeptide in E.coli or a synthetic polypeptide that is given the amino acid sequence portions by a general method, immunizing a test animal with the aforementioned polypeptid, and extracting the antibody from a serum obtained from an immune animal. A monoclonal antibody can also be produced from the polypeptide expressed by E.coli or a synthetic polypeptide that is given the amino acid sequence portions by a general method, immunizing a test animal with the aforementioned polypeptide, fusing the spleen cells and myeloma cells extracted from the test animal to synthesize a hybridoma cell, and extracting the antibody from the hybridoma cell.

These antibodies can also be included as a component of the aforementioned cancer cell immortalization determining reagent kit for use in judging the immortalization of cancer cells.

Specifically, judgment of immortalized cancer cells is carried out with the following Step (1') and Step (2').
(1') a step of mixing a protain-containing fraction containing a polypeptide prepared from a biological sample that has the potential to contain cancer cells obtained from a test subject, with an antibody that recognizes the immortalization-determining polypeptide of the present invention; and
(2') a step of measuring the amount of polypeptide bonded with the antibody, using the antibody as an index.
The amount of polypeptide found in Step (2') depends on the production quantity of the immortalization-determining polypeptide.

In Step (2'), the biological sample can be any sample extracted from the test subject, as long as it has the potential to contain cancer cells. Examples of biological samples include body fluid (blood, urine, etc.), tissues, or extracts thereof, and cultures of the obtained tissues. Extraction of the biological sample from the test subject is performed by an appropriate method according to the type of biological sample or type of cancer. Preparation of the protein-containing fraction (including polypeptide) from the biological sample is performed by an appropriate combination of known differentiation and purification methods. The immortalization-determining polypeptide can be detected by way of various immunological detection methods, such as western blotting, enzyme immunoassay (EIA), radioisotope immunoassay (RIA), luminescence immunoassay, or the like, using the antibody as a probe.

The following explains Steps (1') and (2') for measuring the amount of polypeptide (production quantity of the immortalization-determining polypeptide) by way of western blotting. First, an antibody against the immortalization-determining polypeptide is mixed as a primary antibody with a protein-containing fraction prepared from a biological sample of the test subject to be bonded with the immortalization-determining polypeptide contained in the protein-containing fraction. Next, a secondary antibody labeled with a radioisotope, a fluorescent material, or a chemical luminescent substance is bonded with the primary antibody. Then, the amount of immortalization-determining polypeptide is measured based on the signal that derives from the marker of the secondary antibody. Detection of the signal can be carried out by an appropriate known method using, for example, a radiation detector, or a fluorescence detector.

The immortalization of the cancer cells of the test object can be judged by comparing the obtained amount of polypeptide (production quantity of the immortalization-determining polypeptide) in the test subject in Step (2') with the amount of comparative immortalization-determining polypeptide (comparative production quantity) in a non-immortalized healthy or cancer cell, and finding the difference level. More specifically, if the obtained amount of polypeptide (production quantity of the immortalization-determining polypeptide) in the test subject in Step (2') is greater than the amount of comparative polypeptide (comparative production quantity), it is judged that the cancer cells of the test subject are immortalized. In the reverse case, it is judged that the cancer cells of the test subject are not immortalized.

Accordingly, in addition to the aforementioned Steps (1') and (2'), the immortalized cancer cell determination method of the present invention includes the following Step (3'), and preferably, also Step (4').
(3') a step of comparing the amount of polypeptide found in Step (2') with the amount (comparative polypeptide amount) of the corresponding polypeptide in a non-immortalized healthy or cancer cell; and
(4') a step of judging that the extracted cancer cell of the test subject is immortalized when the amount of polypeptide found in Step (2') is higher than the comparative polypeptide amount, and judging that the extracted cancer cell of the test subject is not immortalized when the polypeptide amount is not higher than the comparative polypeptide amount.

The production quantity of the immortalization-determining polypeptide in a non-immortalized healthy or cancer cell is calculated by first measuring the production quantity of the immortalization-determining polypeptide in a plurality of non-immortalized healthy or cancer cells under the same condition, and then finding a mean or intermediate value of the calculated values.

### (IV) A method for screening immortalized cancer cell growth inhibitor

(IV-1) As shown in the Examples, the immortalization-determining genes ((1) to (13)) exhibit high expression specifically in immortalized cancer cells. Further, by suppressing the expression using siRNA of the immortalization-determining genes, proliferation of the immortalized cancer cells is suppressed. This indicates that the substance that suppresses the expression of the immortalization-determining genes ((1) to (13)) can serve as an immortalized cancer cell growth inhibitor (anticancer drug). Therefore, by using the expression suppression of the immortalization-determining genes ((1) to (13)) as an index, the substance that suppresses proliferation of the immortalized cancer cells can be screened.

Accordingly, described herein is a method for screening immortalized cancer cell growth inhibitors using the expression suppression of the immortalization-determining genes ((1) to (13)) as an index.

The present method may include the following Steps (A) to (D).
(A) a step of bringing a test material into contact with a cell that can express one of the immortalization-determining genes (1) to (13);
(B) a step of measuring the expression amount of the immortalization-determining gene in the cell brought into contact with the test material;
(C) a step of comparing the expression amount of the immortalization-determining gene found in Step (B) with the expression amount (comparative expression amount) of the immortalization-determining gene in a cell not in contact with the test material; and
(D) a step of selecting the test material as a substance for inhibiting the proliferation of the immortalized cancer cells when the expression amount found in Step (B) is lower than the comparative expression amount.

The cell used can derive from any kind of cell as long as the cell can express at least one of the immortalization-determining genes (1) to (13). Examples of organs from which the cell used may derive include lung, stomach, colon, rectum, liver, gallbladder, bile duct, pancreas, kidney, bladder, prostate gland, womb, marrow, lymph gland and blood. The cell may derive from mammals or birds, preferably mammals, and more preferably humans. The expression of the immortalization-determining gene may be endogenous or exogenous.

The test material is not limited and may be nucleic acid (including polynucleotides), a peptide (including polypeptides), an organic compound, or an inorganic compound. The screening is carried out by bringing a test material or a sample containing the test material (test sample) into contact with a cell that can express the immortalization-determining gene. Examples of the test sample include cell extracts, expression products from a gene library, and extracts of natural vegetable or animal-origin products.

In the screening, the process of bringing the test material into contact with the cell is not limited as long as it does not kill the cell and does not inhibit the expression of the immortalization-determining gene in the cell.

In Steps (B) and (C), the expression amount of the immortalization-determining gene can be found by measuring the amount of mRNA of the immortalization-determining gene or its derivative cDNA or double-strand DNA by northern blotting or RT-PCR, using the aforementioned probe or primer of the present invention. Further, the amount of the immortalization-determining polypeptide as an expression product of the immortalization-determining gene may be used to find the expression amount of the immortalization-determining gene in Steps (B) and (C). The amount of the immortalization-determining polypeptide can be found through various immunological detection methods, such as western blotting, enzyme immunoassay (EIA), radioisotope immunoassay (RIA), luminescence immunoassay, or the like, using an antibody against the immortalization-determining polypeptide.

In Step (C), the substance that inhibits proliferation of the immortalized cancer cells can be screened by comparing the expression amount of the immortalization-determining gene measured in Step (B) with the expression amount of the immortalization-determining gene (comparative expression amount) in a cell not in contact with the test material. More specifically, when the expression amount of the immortalization-determining gene found in Step (B) is lower than the comparative expression amount, the test material can be selected as a substance for inhibiting the proliferation of the immortalized cancer cell.

(IV-2) Activity inhibition of the immortalization-determining polypeptides ((14) to (26)), which are the expression products of the immortalization-determining genes ((1) to (13)) also constitutes an index to screen the immortalized cancer cell growth inhibitor. For example, the (14) UBE2S polypeptide is known to have a ubiquitin activator (E2) (J. Biol. Chem. 267 (22), 15829-15835 (1992)); The (16) PTGES2 polypeptide is known to have an activity to convert prostaglandin H2 to prostaglandin E2 (Biochem. Biophys. Res. Commun. 291 (4), 884-889 (2002)); the (18) ACVR2B polypeptide is a transmembrane receptor, and has a Ser/Thr kinase activity in the intercellular domain (Mol. Cell Biol. 16 (3), 1066-1073 (1996)); the (20) LTB4DH polypeptide has an activity to convert leukotriene B4 to 12-oxo-leukotriene B4 (J. Biol. Chem. 271 (5), 2844-2850 (1996)); and the (22) SEPX1 polypeptide has a methionine sulfoxide reducing activity (Mol. Biol. Cell 15 (3), 1055-1064 (2004)); therefore, inhibitions of these activities of the immortalization-determining polypeptides is particularly useful as indices to screen the immortalized cancer cell growth inhibitor.

More specifically, by carrying out the following Steps (A') to (D'), a substance for suppressing the proliferation of the immortalized cancer cells can be screened.
(A') a step of bringing a test material into contact with one of the immortalization-determining polypeptides (14) to (26);
(B') a step of measuring the activity of the immortalization-determining polypeptide in contact with the test material;
(C') a step of comparing the activity of the immortalization-determining polypeptide measured in Step (B') with the activity (comparative activity) of the immortalization-determining polypeptide in an aqueous solution, a cell, or a cell fraction that is not in contact with the test material; and
(D') a step of selecting the test material as a substance for inhibiting the proliferation of the immortalized cancer cells when the activity found in Step (B') is lower than the comparative activity.

The screening uses the activity of the immortalization-determining polypeptide as an index, and may be carried out with respect to any object that contains or may contain one of the immortalization-determining polypeptides (14) to (26) (preferably (14), (16), (18), (20) or (22)). An appropriate object is selected according to the desired function (activity) of the immortalization-determining polypeptide. Examples of objects subjected to screening include an aqueous solution containing one of the immortalization-determining polypeptides (14) to (26) (preferably (14), (16), (18), (20) or (22)); a cell that can express one of the immortalization-determining genes (1) to (13) (preferably (1), (3), (5), (7) or (9)); or a cell fraction prepared from the aforementioned cell. The aqueous solution is not particularly limited as long as it contains an immortalization-determining polypeptide. In addition to a general aqueous solution, a cell solution, a nuclear extraction, or a liquid culture supernatant may be used. The cell may be endogenous or exogenous, and may be any cell that can express one of the immortalization-determining genes. The cell fraction designates a fraction that derives from the aforementioned cell, such as a cell membrane fraction, a cytoplasmic fraction, a cell nucleus fraction, or the like.

The same cell and test material as those for the previously described screening can be used for this screening process.

Measurement of the ubiquitin activation capability of the UBE2S polypeptide (14) in Step (B') can be carried out by a known method. For example, "J. Biol. Chem. 267 (22), 15829-15835 (1992)" discloses a method of performing ubiquitin activation reaction of the UBE2S polypeptide in a system containing ubiquitin, a ubiquitin activating enzyme (E1), and UBE2S polypeptide, and measuring the amount of ubiquitin bonded to the UBE2S polypeptide. Measurement of the ubiquitin amount may be performed by a known method. For example, the reaction is performed using ubiquitin labeled with a radioactive material or enzyme, and the amount of the marker is measured (J. Biol. Chem. 267 (22), 15829-15835 (1992), J. Biol. Chem. 279 (51), 52970-52977 (2004)). Alternatively, measurement can be performed by detecting ubiquitin using an anti-ubiquitin antibody (J. Biol. Chem. 279 (51), 52970-52977 (2004)). The ubiquitin and ubiquitin activating enzyme (E1) may be a marketed commodity, or may be obtained by a known protein synthesis process.

Measurement of the prostaglandin E2 synthesis activity of the PTGES2 polypeptide (16) in Step (B') may be performed using a known method. For example, as disclosed in "Biochem. Biophys. Res. Commun. 291 (4), 884-889 (2002)", the production amount of the prostaglandin E2 can be measured by performing a prostaglandin E2 conversion reaction using PTGES2 polypeptide in a system containing PTGES2 polypeptide and prostaglandin H2. The amount of the prostaglandin E2 can be measured using a known method. For example, according to "Proc. Natl. Acad. Sci. U.S.A. 96 (13), 7220-7225 (1999))", the reaction is performed using prostaglandin H2 labeled with a radioactive material or enzyme, and then the amount of the marker is measured to find the production quantity of the prostaglandin E2. Alternatively, the measurement can be performed by using an anti-prostaglandin E2 antibody (J. Dairy. Sci. 87 (7), 2197-2210 (2004)). The prostaglandin H2 may be a marketed commodity, or may be obtained by a known synthesis process.

Measurement of the Ser/Thr kinase activity of the ACVR2B polypeptide (18) in Step (B') may be performed using a known method. For example, as disclosed in "Mol. Cell Biol. 16 (3), 1066-1073 (1996)", the Ser/Thr kinase activity can be measured by performing a kinase reaction in a system containing ACVR2B polypeptide in the presence of a substrate and ATP, and then measuring the phosphorylation quantity of the substrate. The phosphorylation quantity of the substrate may be measured by a known method. For example, the phosphorylation quantity may be measured by measuring radioactivity using γ³²P-ATP or γ³³P-ATP as a tracer (Mol. Cell Biol. 16 (3), 1066-1073 (1996)). Alternatively, the phosphorylation quantity may be measured by performing a phosphorylation reaction using a substrate labeled with a fluorescent material or biotin, and measuring the quantity of the phosphorylation marker. Examples of substrates include ACVR2B polypeptide, and specific substrates and peptides.

Measurement of the leukotriene B4 conversion activity of the LTB4DH polypeptide (20) in Step (B') may be performed using a known method. For example, as disclosed in "J. Biol. Chem. 271 (5), 2844-2850 (1996)", the leukotriene B4 conversion activity can be measured by performing a leukotriene B4 conversion reaction using LTB4DH polypeptide in a system containing LTB4DH polypeptide and leukotriene B4, and then measuring the production quantity of the 12-oxo-leukotriene B4. The production quantity of the 12-oxo-leukotriene B4 may be measured by a known method, such as HPLC. The leukotriene B4 may be a marketed commodity, or may be obtained by a known chemical synthesis process.

Measurement of the methionine sulfoxide reducing activity of the SEPX1 polypeptide (22) in Step (B') may be performed using a known method. For example, as disclosed in "Mol. Biol. Cell 15 (3), 1055-1064 (2004)", the methionine sulfoxide reducing activity can be measured by performing a methionine sulfoxide reducing reaction using SEPX1 polypeptide in a system containing SEPX1 polypeptide and methionine sulfoxide, and then measuring the production quantity of the methionine. The production quantity of the methionine may be measured by a known method, such as HPLC. The methionine sulfoxide may be a marketed commodity, or may be obtained by a known chemical synthesis process.

In Steps (C') and (D'), the substance that inhibits the proliferation of the immortalized cancer cells can be screened by comparing the measured activity of the immortalization-determining polypeptide in Step (B') with the comparative activity. More specifically, when the measured activity of the immortalization-determining polypeptide is lower than the comparative activity, the test material is determined to be a substance for inhibiting the proliferation of the immortalized cancer cell.

As explained in the Examples, each of the antisence polynucleotides (siRNA) (SEQ ID No.s 27 to 76) shown in Tables 2 and 3 hybridizes with one of the immortalization-determining genes in a cancer cell to suppress the expression of the immortalization-determining gene, thereby suppressing the proliferation of the immortalized cancer cells. Therefore, the antisence polynucleotide (siRNA) can be used as an immortalized cancer cell growth inhibitor. Accordingly, described herein is an immortalized cancer cell growth inhibitor containing the antisence polynucleotide as an active ingredient.

The antisence polynucleotide is formed of a polynucleotide of at least 15 bases that specifically hybridizes with a polynucleotide having a continuous base sequence of at least 15 bases within a base sequence of the immortalization-determining genes (1) to (13). The polynucleotide preferably has a base sequence complementary to the continuous base sequence of at least 15 bases in one of the immortalization-determining genes (1) to (13); however, as long as a specific hybridization is possible and thereby the expression of the immortalization-determining gene is suppressed, it is not required to be totally complementary. For example, the base sequence of the polynucleotide may have an identity of 70%, preferably not less than 80%, more preferably not less than 90%, yet more preferably not less than 95%, and still more preferably not less than 98%, with respect to the complementary sequence of the immortalization-determining gene; and the polynucleotide may hybridize with RNA of the immortalization-determining gene to suppress the expression of the immortalization-determining gene. The antisence polynucleotide of the present invention preferably has 15 to 1,000 bases, more preferably 15 to 500 bases, and yet more preferably 16 to 30 bases.

The antisence polynucleotide preferably has a base sequence represented by one of SEQ ID No.s 27 to 76, and more preferably a double-strand RNA having one of the base sequences.

The antisence polynucleotide may be modified with a known technique to improve stability and cell permeability. For example, a phosphoric residue of each nucleotide may be substituted with a chemically modified phosphoric residue, such as phosphorothioate, methyl phosphonate, or phosphorodithioate, to prevent decomposition by a hydrolase such as nuclease.

The form of the antisence polynucleotide is not limited and may be single-strand DNA, single-strand RNA, double-strand DNA, double-strand RNA or DNA:RNA hybrid.

Generally, the antisence polynucleotide may be synthesized using a known method, such as chemical synthesis using a synthesizer.

The antisence polynucleotide is introduceable into cancer cells of a patient using a virus vector, such as a retrovirus vector, adenovirus vector, or adeno-associated virus vector, or a non-virus vector such as liposome, through an ex vivo process or an in vivo process, thereby suitably serving as an anticancer treatment. Accordingly, described herein is an anticancer treatment method comprising the step of administering an immortalized cancer cell growth inhibitor containing the antisence polynucleotide as an active ingredient to a patient.

The immortalized cancer cell growth inhibitor contains the aforementioned antisence polynucleotide as an active ingredient, and may also contain a stabilizer, a suspension, a freezing mixture, a buffer solution, a solvent, or the like insofar as the cell proliferation suppression effect of the inhibitor is not impaired. The dosage and administration schedule of the immortalized cancer cell growth inhibitor to a cancer patient can be suitably determined by a person having skill in the art according to the type of disease, patient's age, weight, etc. Examples of administration schedules include a 3-week cycle of intravenous administration in which a consecutive 2 to 3-week administration of the antisence polynucleotide is carried out with a dosage of 2 to 10 mg/kg per day; a 1-week cycle of intravenous administration in which consecutive 5-day administration of the antisence polynucleotide is carried out with a dosage of 80 to 120 mg/m² per day; and a 4-week cycle of intravenous administration in which a consecutive 3-week administration of the antisence polynucleotide is carried out with a dosage of 80 to 120 mg/m² per day.

### EXAMPLE

The present invention is more specifically explained below in reference to examples. The present invention is, however, not limited to those examples.

### Example 1

Identification of immortalization-determining gene 1. Preparation of total RNA from human tissue sample, human cancer cell strain, and human nonneoplastic culture cell Using an RNeasy^{™} Mini kit (Qiagen), total RNAs were extracted from nine kinds of lung cancer cell strains, 21 kinds of esophagus cancer cell strains, nine kinds of digestive system cancers, various other cancer cell strains (gastric cancer, colon cancer, head neck cancer, leukemia), two kinds of nonneoplastic esophagus epithelial cells, and two kinds of normal respiratory epithelia, according to the attached protocol. The extracted total RNAs were preserved at -80°C.

Further, total RNAs were also extracted from eleven kinds of breast cancer cell strains, ten kinds of ovary cancer cell strains, ten kinds of pancreas cancer cell strains, one kind of nonneoplastic immortalization mammary gland epithelia, one kind of nonneoplastic mammary gland epithelia, ten sets of pancreatic cancer tissue and non-cancer pancreas tissue of the same pancreatic cancer patient, the primary focus and three metastasis portions of lung cancer tissue of the same non-small cell lung cancer patient. The obtained total RNAs were preserved. The total RNAs were examined to ensure high quality, with clear 18S and 28S rRNA peaks using a 2100 Bioanalyzer (Agilent Technologies) and RNA LabChip (Agilent Technologies), before they were subjected to a microarray assay.

### 2. Microarray assay

Using the extracted total RNAs, an exhaustive gene expression assay was carried out by way of a microarray. The microarray used a CodeLink UniSet Human 20K I Bioarray (19881 probe), and comprised (1) a synthesis of cDNA from total RNA, (2) a synthesis of labeled cRNA from the cDNA, (3) a fragmentation process of the labeled cRNA, (4) a hybridization of fragment cRNA and microarray, (5) staining of a microarray, (6) scanning of a microarray, and (7) a gene expression assay.

### (1) Synthesis of cDNA from total RNA

Using a CodeLink Expression Assay Reagent Kit (GE Healthcare Bio Science), a first-strand cDNA was synthesized according to the protocol. More specifically, 1 µg each of the total RNAs obtained in section 1 were adjusted to 10 µl with nuclease-free water, mixed with 1 µl of a bacterial control mRNA diluent solution and 1 µl of T7 oligo (dT) primer, both contained in the kit. The total 12 µl of liquid was heated at 70°C for 10 minutes, followed by 3 minutes rapid cooling on ice. On the ice, the liquid was mixed with 2 µl of 10 x first-strand buffer, 4 µl of 5-mM dNTP Mix, 1 µl of RNase inhibitor, and 1 µl of reverse transcriptase (200 U/µl), all contained in the kit. The total 20 µl of liquid was heated at 42°C for two hours.

Subsequently, according to the protocol, the RNA in the RNA-DNA hybrid was decomposed, and the RNA strand was substituted with a DNA strand, thereby synthesizing a second-strand cDNA (double-strand cDNA). More specifically, 63 µl of nuclease-free water, 10 × second-strand buffer (10 µl), 5-mM dNTP mix (4 µl), DNA polymerase mix (2 µl:10 U/µl), 1 µl of RNase H, all contained in the kit, were added to the 20 µl of first-strand cDNA reaction solution. The total 100 µl of liquid was heated at 16°C for two hours.

After the reaction, the synthesized double-strand cDNA was purified using a QIA quick PCR purification Kit (QIAGEN) according to the attached protocol. More specifically, 100 µl of the cDNA solution was mixed with 500 µl of buffer PB contained in the kit, and the mixed liquid was placed in a QIA quick spin column set in a 2-ml centrifugal tube and subjected to centrifugation for 50 seconds at 13,000 rpm. After the elution liquid was removed, the QIA quick spin column was set again, 700 µl of buffer PE contained in the kit was added thereto, and 13,000 rpm centrifugation was performed for 1 minute. The elution liquid was removed, the QIA quick spin column was set again, and still another centrifugation was performed at 13,000 rpm for 1 minute so as to completely remove the buffer PE. The QIA quick spin column was set in a new 1.5-ml tube, and 30 µl of nuclease-free water was directly added onto the membrane in the QIA quick spin column, allowed to stand for 1 minute, and followed by 13,000-rpm centrifugation for 1 minute. This process was performed twice (60 µl was used in total). Then, the cDNA solution was concentrated to less than 9.5 µl using a vacuum dryer, and adjusted by adding nuclease-free water to 9.5 µl.

### (2) Synthesis of labeled-cRNA from cDNA

Subsequently, according to the protocol of a CodeLink Expression Assay Reagent Kit (GE Healthcare Bio Science), an in vitro transcription (IVT) reaction was performed in the presence of a biotin-labeled nucleotide to synthesize a cRNA. More specifically, 4.0 µl of a 10 × T7 reaction buffer, 4.0 µl of a T7 ATP solution, 4.0 µl of a T7 GTP solution, 4.0 µl of a T7 CTP solution, and 3.0 µl of a T7 UTP solution, all contained in the kit, were mixed. After further adding 7.5 µl of 10-mM biotin-11-UTP (Perkin Elmer Corporation), the total 26.5 µl of liquid was added to the 9.5 µl of cDNA solution prepared in the foregoing process (1). Further, 4.0 µl of a 10 x T7 Enzyme Mix contained in the kit was added, and the total 40.0 µl of liquid was mixed well and heated in a vapor phase at 37°C for 14 hours. After the reaction, the synthesized cRNA was purified using an RNeasy Mini Kit(QIAGEN) according to the attached protocol. More specifically, 60 µl of nuclease-free water was added to the IVT reaction solution (40 µl), and 350 µl of buffer RLT contained in the kit and 250 µl of 100% ethanol was added and mixed well, and the total 700 µl of mixed liquid was placed in an RNeasy mini spin column to be subjected to centrifugation for 15 seconds at 12,000 rpm. The RNeasy mini spin column was set in a new 2-ml centrifugal tube, 500 µl of buffer RPE contained in the kit was added to the column, and another 12,000-rpm centrifugation was performed for 15 seconds. This process was performed twice. After the elution liquid was removed, the RNeasy mini spin column was set in the 2-ml centrifugal tube, and 12,000 rpm centrifugation was performed for 2 minutes to dry the membrane in the column. Thereafter, the RNeasy mini spin column was set in a new 1.5-ml tube, and 50 µl of nuclease-free water was directly added onto the membrane, allowed to stand for 10 minute at room temperature, and followed by 12,000-rpm centrifugation for 1 minute. This process was performed twice (100 µl was used in total).

The sample was mixed well, and dispensed into a 2-µl portion for cRNA quality check using an Agilent 2100 Bioanalyzer and a 2-µl portion diluted 50-fold with sterile distilled water for cRNA quantitative determination; the rest was preserved at - 80°C. For the quantitative determination, the absorbency of the 50-fold diluted solution was measured with respect to 260 nm and 280 nm, and the cRNA concentration was determined. The absorbency ratio of 260 nm to 280 nm was confirmed as 1.8 or greater. When the RNA concentration was 0.5 µg/µl or less, the sample was concentrated using a vacuum dryer. The cRNA quality check was performed by electrophoresis using an Agilent 2100 Bioanalyzer and RNA LabChip according to the attached protocol, confirming that the smear peak had at least 500 bases.

### (3) Fragmentation of labeled cRNA

Subsequently, according to the protocol of the CodeLink Expression Assay Reagent Kit (GE Healthcare Bio Science), the cRNA was fragmented into about 100 to 200 bases. More specifically, nuclease-free water was added so that the 10 µg of cRNA became 20 µl; then, 5 µl of 5 × fragmentation buffer contained in the kit was added. After heating at 94°C for 20. minutes, the liquid was rapidly cooled on ice. 78 µl of hybridization buffer A, 130 µl of hybridization buffer B, 27 µl of nuclease-free water, all contained in the kit, were added to the 25 µl of solution containing 10 µg of cRNA fragments, preparing a hybridization solution of 260 µl in total. After vortexing at maximum speed for 5 seconds and spinning-down, the sample was heated at 90°C for 5 minutes to denaturate the cRNA by heat, followed by cooling on ice.

### (4) Hybridization of cRNA fragment and microarray

Using a CodeLink Shaker Kit (GE Healthcare Bio Science) and a CodeLink Innova shaker, hybridization was performed according to the protocol. More specifically, a CodeLink uniSet human 20K I bioarray was set in the 12-slide shaker tray contained in the kit. The hybridization solution prepared in the foregoing process (3)-was vortexed at maximum speed for 5 seconds and spinned down. The 250 µl of hybridization solution was injected to a right-bottom hole of the sealing chamber of the array, and the hole was covered with a sealing strip (sticker) attached to the array. The shaker tray having an array thereon was placed in the CodeLink Innova shaker, and heated at 37°C for 18 hours while being rotated at 300 rpm.

### (5) Staining of microarray

Using a CodeLink Parallel Processing Kit (GE Healthcare Bio Science), the array was stained and washed according to the protocol. More specifically, the sealing chamber was removed from the hybridized array, 13 ml of 0.75 × TNT buffer (0.1M Tris-HCl (pH 7.6), 0.15M NaCl, 0.05% Tween 20) was added at room temperature, and the resulting liquid was divided into the slots of a medium reagent reservoir with a bioarray rack contained in the kit. The rack was moved up and down several times to shake off extra hybridization solution. The bioarray rack was then shifted to a large reagent reservoir filled with 240 ml of 0.75 x TNT buffer heated to 46°C. The bioarray rack was heated to 46°C for an hour.

As a staining fluid, for each array, 6.8 µl of Streptavidin-Cy5 liquid dissolved by nuclease-free water to 1 mg/ml was diluted 500-fold using 3393.2 µl of TNB buffer (0.1 M Tris-HCl(pH 7.6), 0.15 M NaCl, 0.5 % TSA Blocking Reagent (Perkin Elmer Corporation)) at room temperature. The total 3.4 ml was supplied to fill up the slots of a small reagent reservoir contained in the kit. The bioarray rack holding the array was shifted to the small reagent reservoir, blocked from light with aluminum or the like, and subjected to staining for 30 minutes at room temperature (23°C ±2°C).

After staining, the bioarray rack was shifted to a large reagent reservoir filled with 240 ml of TNT buffer (room temperature), moved up and down several times and allowed to stand for 5 minutes at room temperature. The rack was then shifted to a large reagent reservoir filled with new TNT buffer (room temperature), moved up and down several times and allowed to stand for 5 minutes at room temperature. This washing process was repeated four times. Finally, the rack was washed with 240 ml of 0.05% Tween 20 solution by being moved up and down for five seconds. This process was repeated twice. The bioarray rack was set in a dried medium reagent reservoir, followed by 3 minutes of centrifugation at room temperature, 600 × g.

### (6) Scanning of microarray

Each of the stained arrays was scanned by an Agilent G2565 scanner (Agilent), to be saved a staining pattern as TIFF image. Each TIFF image was processed by CodeLink expression analysis software, thereby digitizing a signal strength for each gene spot on the array.

### (7) Gene expression assay

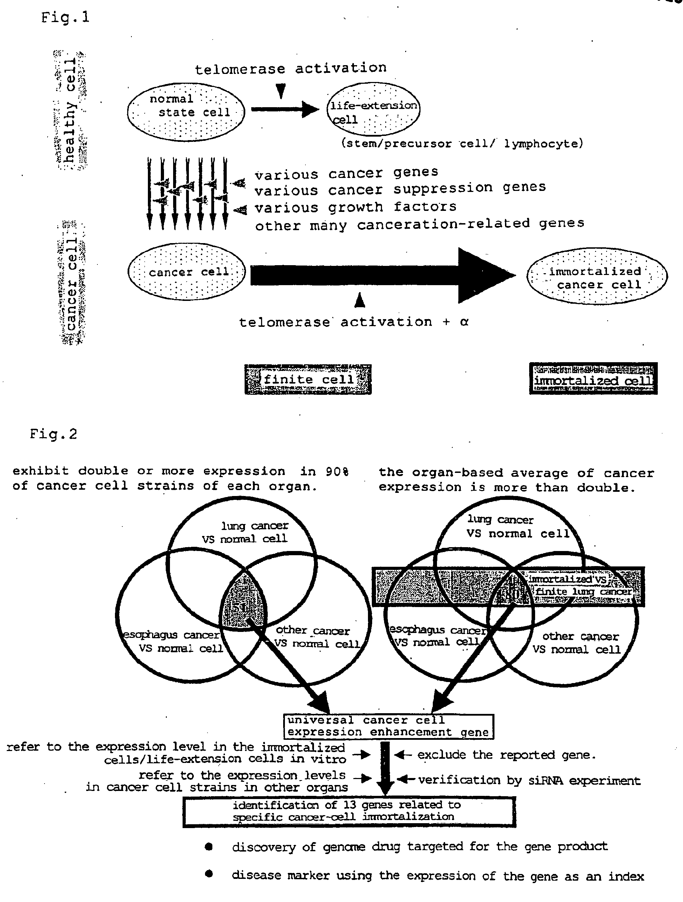

The signal strength data thus obtained was normalized using GeneSpring GX (Agilent Technologies) microarray gene expression assay software, and the data was then assayed. Specifically, the background signal was subtracted from the spot signal. Values less than 0.01 were equalized to 0.01, and each value was divided by the intermediate value of all spot signals so as to find a standardized relative expression amount of the gene. Genes whose relative expression amount was higher universally in the immortalized cancer cell strains (FIG. 1, lower right) than a healthy cell (FIG. 1, upper left) were discriminated in the following manner. Genes that exhibited enhanced expression universally in various organs appeared to be genes involved in cancer cell immortalization (FIG. 1, large arrow pointing to the right), rather than genes (FIG. 1, downward arrow) involved in canceration of healthy cells (FIG. 1, upper left).

Screened as the first group was any gene in which at least 8 out of 9 kinds of lung cancer cell strains exhibited expression in a quantity of two times or higher than the intermediate value of two normal respiratory epithelia, any gene in which at least 19 out of 21 kinds of esophagus cancer cell strains exhibited expression in a quantity of two times or higher than the intermediate value of two nonneoplastic esophagus epithelia, and any gene in which at least 8 out of 9 digestive cancers, or various other cancer cell strains (gastric cancer, colorectal cancer, head and neck cancer, leukemia) exhibited expression in a quantity of two times or higher than the intermediate value of the four kinds of nonneoplastic epithelia (two kinds of respiratory epithelia and two kinds of esophagus epithelia). As a result, fifty-one genes were screened (FIG. 2, upper left). As the second group, screened was any gene in which the mean value of the expression levels of the three groups of cancer cell strains of different organs was two times or higher than the mean value of the corresponding epithelia (note that, the esophagus cancer cell strains used for this screening based on the mean value correspond to the seven kinds asseyed first), and in which the expression level of the metastasis lung cancer tissue consisting of immortalization lung cancer cells was two times or higher than the mean value of the primary focus tissue and metastasis tissue consisting of non-immortalization lung cancer cells of the same patient. As a result, 80 genes were extracted (FIG. 2, upper right). Among the two screened groups, seven genes were common.

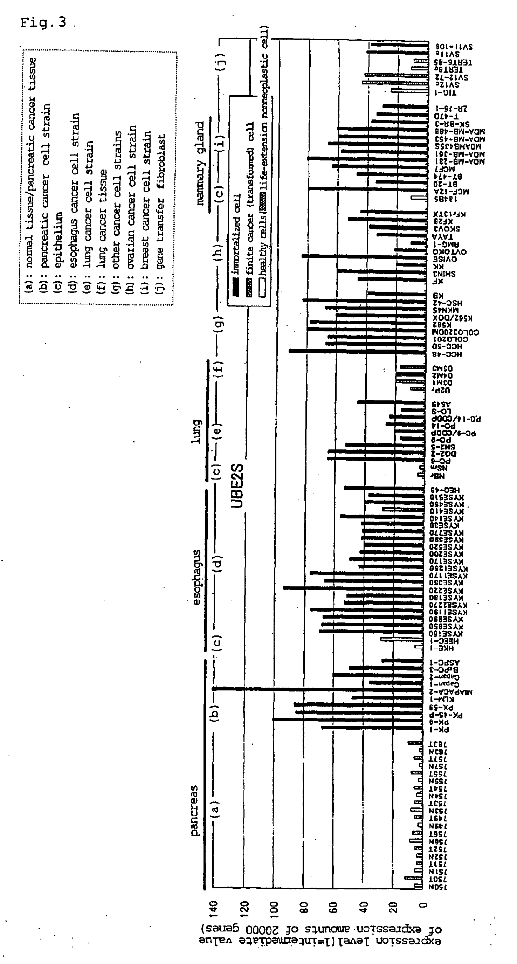

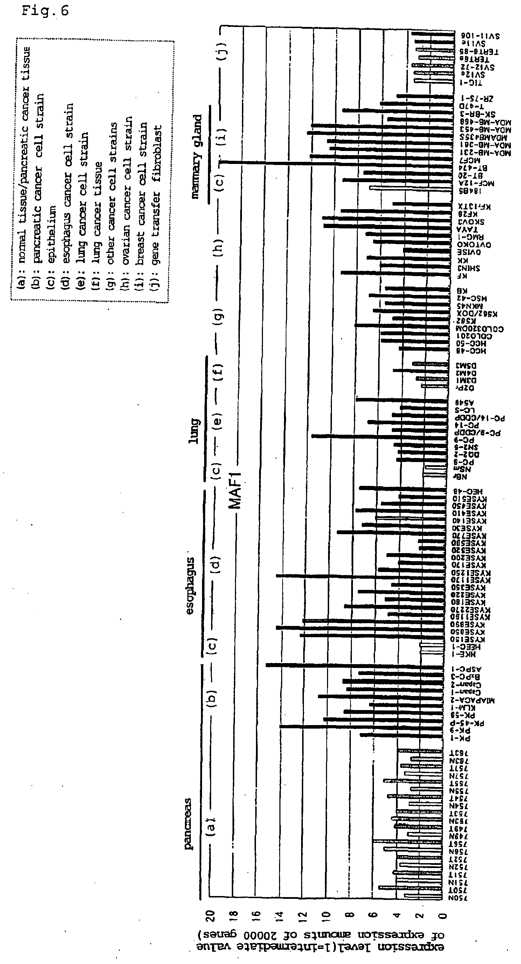

It is a known fact that cancer cell strains are all immortalized. For each of the immortalization/non-immortalization lung cancer tissues used in the experiments, it was confirmed that the primary focus (FIG. 3, "D2Pr") and hepatic metastasis focus (FIG. 3, "D5M3") of the same patient were not immortalized (telomerase activitation: negative; telomere length: reduced; hTERT protein expression: not observed), and that one of the lymph node metastasis focuses (FIG. 3, "D4M2") substantially consisted only of immortalized cells (high telomerase activation, extended telomere length, hTERT protein expression was observed in almost all cells) and the other metastasis focus (FIG. 3, "D3M1") consisted of both immortalized cells and non-immortalization cells (low telomerase activation; hTERT protein expression cells were observed among the non-expression cells). These characteristics were confirmed by assaying telomerase activation (Hiyama K et al., J Natl Cancer Inst 87: 895-902, 1995, Case G), telomere length (Hiyama K et al., Oncogene 10: 937-44, 1995, Case 92-D), and hTERT protein in situ expression (Hiyama E et al., Neoplasia 3: 17-26, 2001, Fig 6A, B).

For further refinement, a gene that exhibits high expression universally in 11 cell strains of breast cancer, 10 cell strains of ovarian cancer, and 10 cell strains of pancreatic cancer was screened. Finally, in total, the thirteen genes: (1) ubiquitin-conjugating enzyme E2S (UBE2S), (2) replication factor C (activator 1) 4, 37kDa(RFC4), (3) prostaglandin E synthase 2, (PTGES2), (4) MAF1 homolog (S. cerevisiae) (MAF1), (5) activin A receptor, type IIB(ACVR2B), (6) family with sequence similarity 119, member A (FAM119A), (7) leukotriene B4 12-hydroxydehydrogenase (LTB4DH), (8) dolichyl-phosphate mannosyltransferase polypeptide 2, regulatory subunit (DPM2), (9) selenoprotein X, 1(SEPX1), (10) proteasome (prosome, macropain) subunit, alpha type, 3 (PSMA3), (11) coiled-coil-helix-coiled-coil-helix domain containing 3 (CHCHD3), (12) LSM3 homolog, U6 small nuclear RNA associated (S. cerevisiae) (LSM3), and (13) G-2 and S-phase expressed 1(GTSE1), were found as genes that exhibit higher expression universally in most lung cancer cell strains, esophagus cancer cell strains, digestive cancer cell strains, breast cancer cell strains, ovarian cancer cell strains, pancreatic cancer cell strains and various other cancer cell strains than mortal nonneoplastic cells; exhibit higher expression in a telomerase-positive lung cancer metastasis tissue (immortalized cancer cells) than in a telomerase-negative lung cancer metastasis tissue and primary tissue (non-immortalized cancer cells) of the same patient; and exhibit higher expression in pancreatic cancer cell strains consists only of immortalized cells than in a pancreatic cancer tissue containing both immortalized cells and non-immortalized cells (FIG. 3). The pancreatic cancer tissue used in this experiment showed a higher expression of full length mRNA of the telomerase-coding gene TERT than in the healthy pancreatic tissue of the same patient. However, since the expression was evidently lower compared with the immortalized pancreatic cancer cell strains, it is assumed that the tissue contained non-immortalized cells.

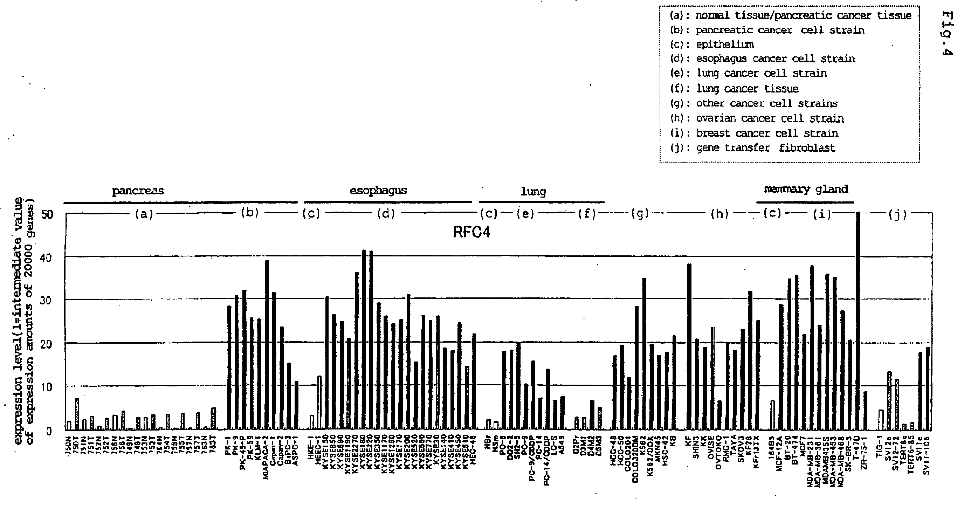

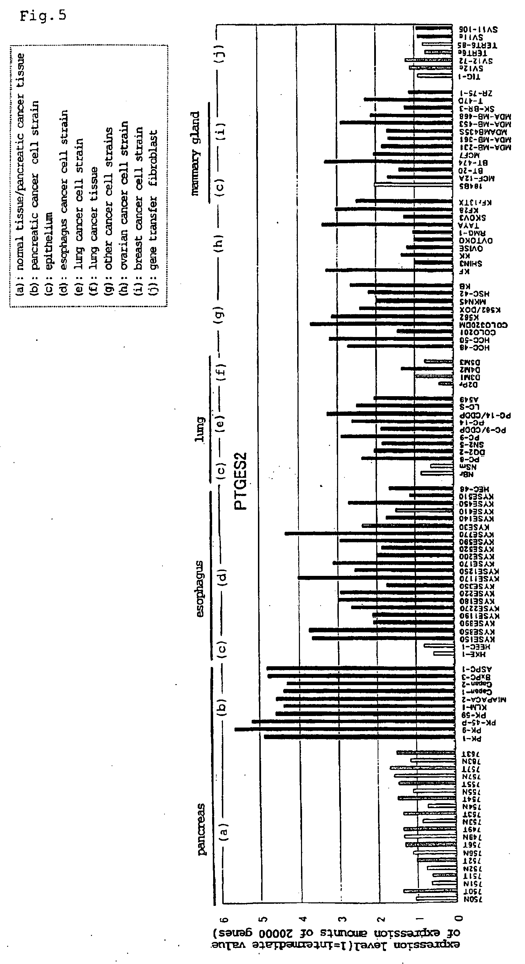

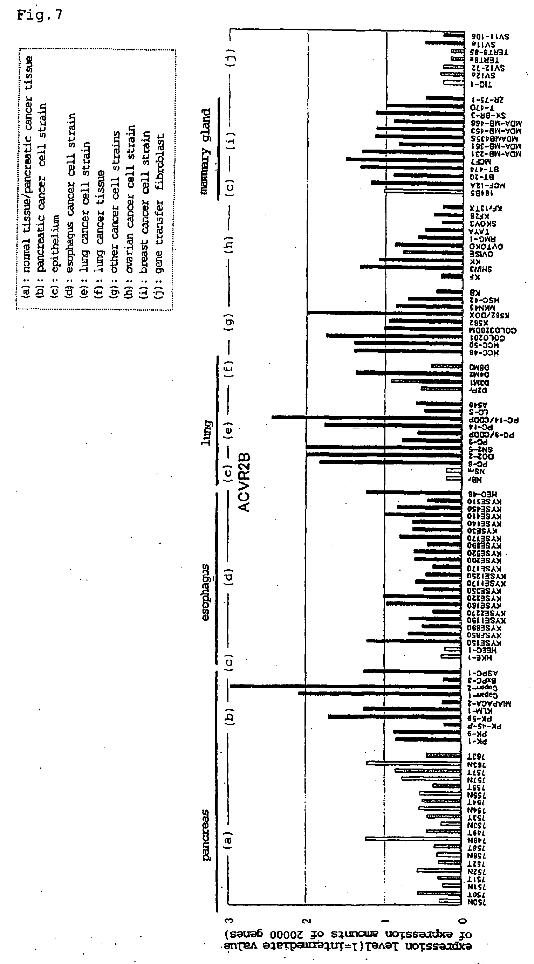

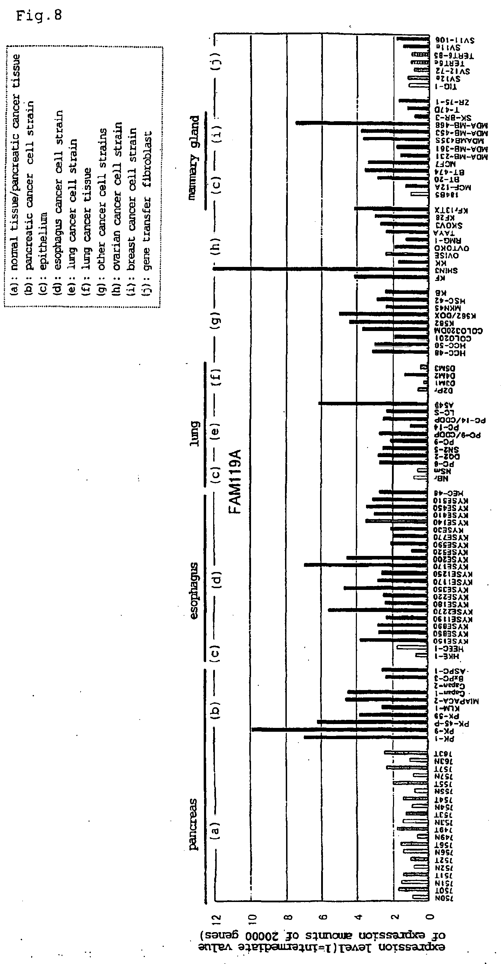

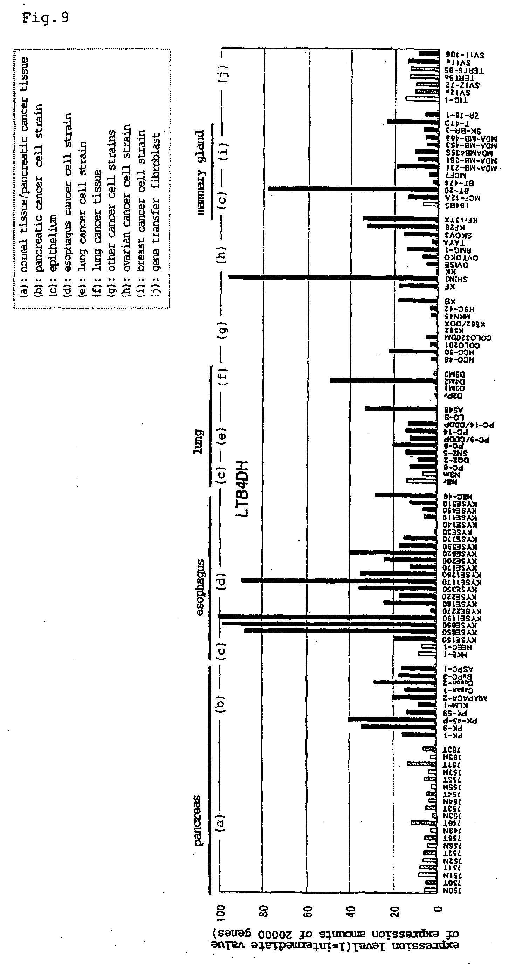

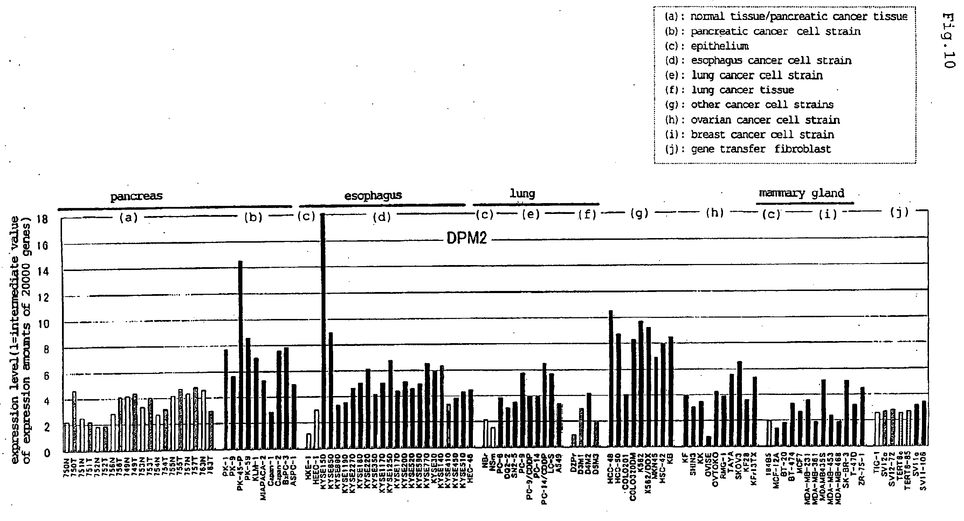

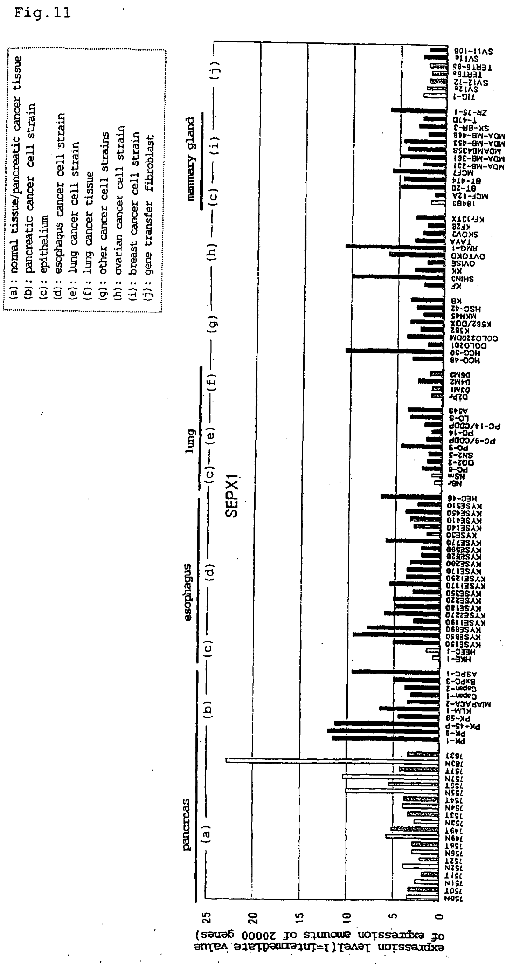

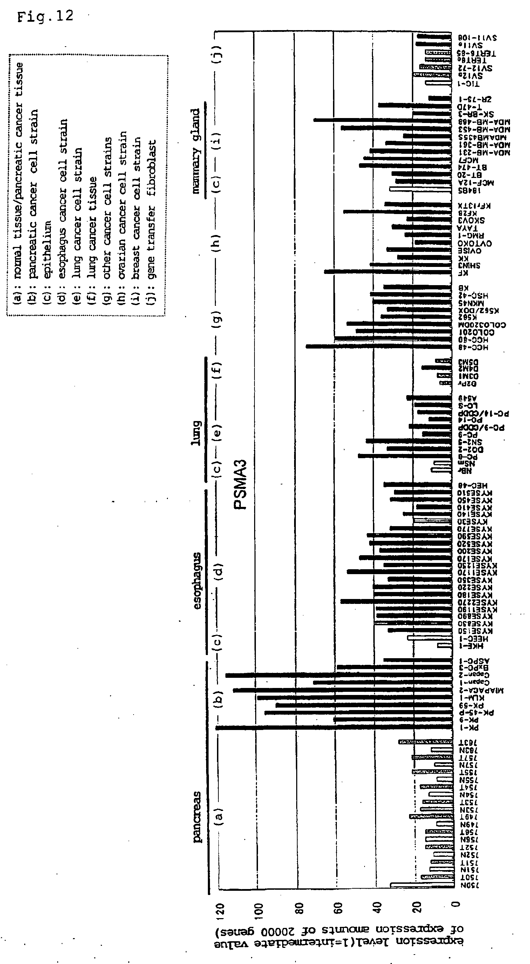

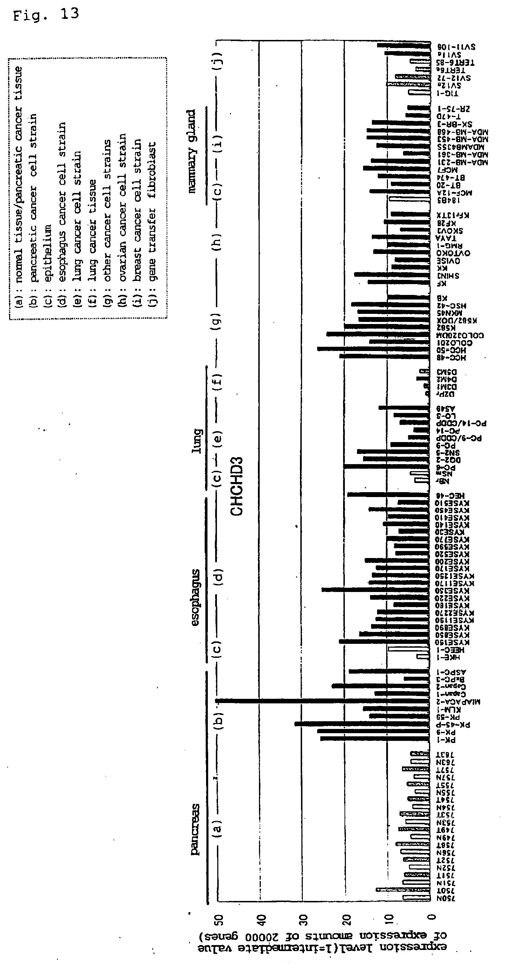

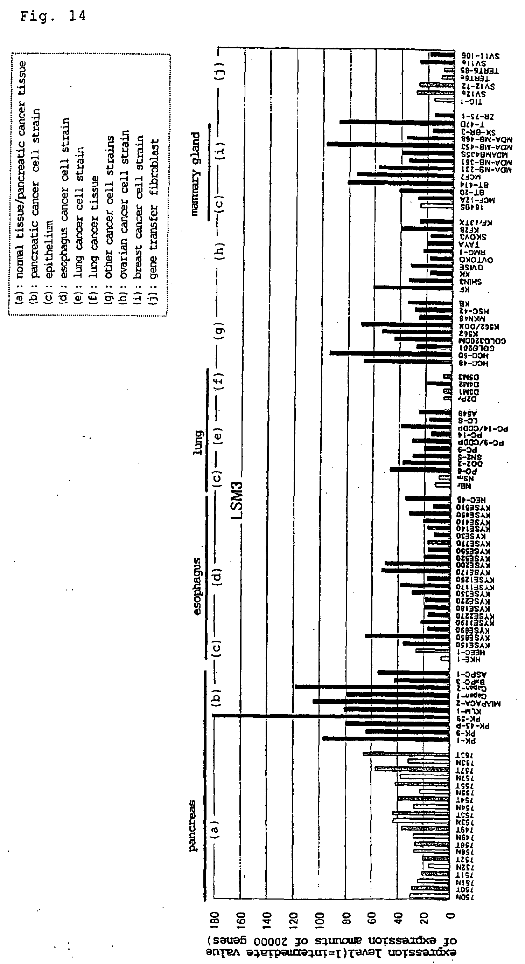

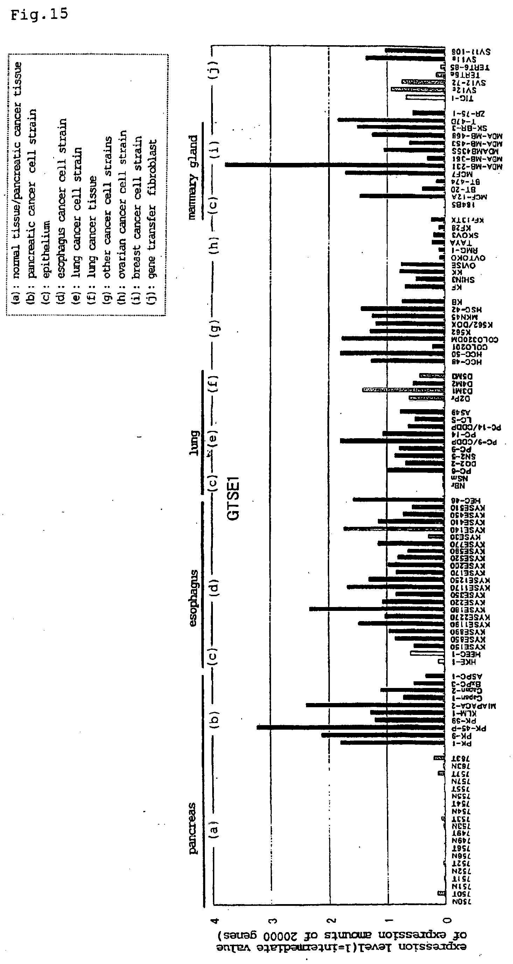

As shown in FIGS. 3 to 15, the foregoing thirteen genes have the following characteristics.
(1) each gene exhibits more enhanced expression in immortalization cells (solid bar in FIGS. 3 to 15, human cancer derived cell strains except for MCF-12A, SV11e, SV11-106) than in nonneoplastic mortal cells (open bar in FIGS. 3 to 15);
(2) each gene exhibits more enhanced expression in immortalized cancer cells derived human cancer (solid bar in FIGS. 3 to 15, except for MCF-12A, SV11e SV11-106) than finite life cancer cells (diagonal lined bar in FIGS. 3 to 15, except for SV12e, SV12-72); and
(3) its expression is less enhanced in nonneoplastic telomerase expression life-extension cells obtained by introducing a telomerase-coding gene TERT into healthy lung fibroblasts (diamond-shaped dotted bars in FIGS. 3 to 15; TERT6e, TERT6-85) than in the parent cells (TIG-1). These genes are highly expressed specifically in telomerase-positive immortalized cancer cells. This shows that these genes serve to determine the immortalization of cancer cells (immortalization-determining gene).

Moreover, in the immortalization transformed cells obtained by introducing genes encoding a telomerase-coding gene TERT and SV40 early antigen into healthy lung fibroblasts (FIGS. 3 to 15, SV11e SV11-106: colony formation and 300 PDL or greater passage capability of passage were confirmed using colony formation assay with soft agar), the expression was enhanced except for MAF1, LTB4DH, than in the parent cells (TIG-1) or nonneoplastic life-extension cells (TERT6e, TERT6-85: absence of colony formation and cessation of cell proliferation at 100PDL or less were confirmed using colony formation assay with soft agar). This is presumably because the cells transformed in vitro are not always equal to human cancer cells.

### Example 2

Suppression of cancer cell proliferation by siRNA (small interfering RNA) of immortalization-determining gene To analyze how the thirteen kinds of immortalization-determining genes identified in Example 1 relate to cancer cell proliferation, siRNAs specified according to the respective gene sequences were designed and created. They were introduced into three kinds of cancer cell strains (HeLa: uterine cervix cancer, KYSE150: esophagus cancer, and HCC50: colon cancer). With these samples, the suppression of mRNA expression of an immortalization-determining gene, and the effect on cell proliferation at 96 hours after introduction were observed.

### (1) Designing and creation of immortalization-determining gene sequence specific siRNA (small interfering RNA)

In each of the thirteen immortalization-determining genes, two or four kinds of specific siRNA sequences were determined, and Double-strand siRNAs each formed of a sense strand and an antisense strand was purchased from Qiagen and Japan Bio Service. Among the created siRNAs sequences, only the sense strands are shown in Tables 2 and 3. The siRNA-1 of each gene was an equimolar mixture of two kinds.

**Table 2**

| Gene Symbol | siRNA Name | Position | Sequence | SEQ ID No. |
|---|---|---|---|---|
| *UBE2S* | siRNA-1 | 44-64 | CCGGCCGGCCGCAGCCAUGAA | 27 |
| | | 146-166 | UGGCAUCAAGGUCUUUOOCAA | 28 |
| | siRNA-2 | 473-491 | GGAGAACUAOGAGGAGUAU | 29 |
| | siRNA-3 | 659-677 | UGGOGAGOGOGAUAAGAAG | 30 |
| *RFC4* | siRNA-1 | 897-917 | AGGGAAUAGCUUAUCUUGUUA | 31 |
| | | 189-209 | CUGCAOGAGAAGOCAGGCUAA | 32 |
| | siRNA-2 | 745-763 | COGAUUCUGUCUUAUCUGU | 33 |
| | siRNA-3 | 1023-1041 | GGGUAAUACCAGCUGAGAA | 34 |
| *PTGES2* | siRNA-1 | 2003-2023 | CUGGGACAUGUUUGCAAUAAA | 35 |
| | | 1105-1125 | CUGGUCAUGCUCAAOGAGAA | 36 |
| | siRNA-2 | 943-961 | AGGAGAAAGCUOGCAACAA | 37 |
| | siRNA-3 | 1086-1104 | CCGAGUUOGGCAAUAAGUA | 38 |
| *MAF1* | siRNA-1 | 1538-1558 | CAGCUGGAOOGCAGAGUUUAU | 39 |
| | | 1031-1051 | UAGOCUCUGGUOCUUCAACUA | 40 |
| | siRNA-2 | 604-622 | AOGACAAACACAUGUUCAA | 41 |

**Table 3**

| | | | | |
|---|---|---|---|---|
| | siRNA-3 | 856-874 | GCCUUAGCUGGGUGGUGAA | 42 |
| *ACVR2B* | siRNA-1 | 626-646 | CAGCUCAUGAAUGACUUUGUA | 43 |
| | | 208-228 | CAOCAUOGAGCUOGUGAAGAA | 44 |
| | siRNA-2 | 684-702 | GGCAGAGUGAACGGGAGAU | 45 |
| | siRNA-3 | 840-858 | GGAACAUCAUCACAUGGAA | 46 |
| *FAM1194* | siRNA-1 | 754-774 | AACGUUCACUACGAUOCUGAA | 47 |
| | | 1068-1088 | UOGAUUUAUGCUAUUUGUGUA | 48 |
| | siRNA-2 | 201-219 | GGAAUUUGGGUUGCAGAAA | 49 |
| | siRNA-3 | 810-828 | CCAGAAGGAGGACUUAUAA | 50 |
| *LTB4DH* | siRNA-1 | 775-795 | CACUGUUAUOGGOCAGAUGAA | 51 |
| | | 658-678 | UGGAUUUGAUGUCGUCUUUAA | 52 |
| | siRNA-2 | 263-281 | GOCAAAAGAUUGAAGGAAG | 53 |
| | siRNA-3 | 725-743 | OCUGAUGGUUAUGAUUGUU | 54 |
| *DPM2* | siRNA-1 | 145-165 | CACCAUGUCAUCCACAAGUAU | 55 |
| | | 84-104 | UAGCCUGAUCAUCUUCACCUA | 56 |
| | siRNA-2 | 116-134 | GGGUGAUUCUCUUGCCAUU | 57 |
| | siRNA-3 | 224-242 | UGUUUGUGGGACUGUUCAU | 58 |
| *SEPX1* | siRNA-1 | 870-890 | CAGACUCUOGCCUCACOGAA | 59 |
| | | 794-814 | CUGAAUGAOGUUACAOCCUCA | 60 |
| | siRNA-2 | 161-179 | GGGOGAGGUUUUOCAGAAU | 61 |
| | siRNA-3 | 179-197 | UCACUUUGAAOCUGGOGUU | 62 |
| *PSMA3* | siRNA-1 | 853-873 | CCAGUCCAAUGUAACUAUUUA | 63 |
| | | 686-706 | CUCAGCUGGGUUGGUGAAUUA | 64 |
| | siRNA-2 | 291-309 | UGGCAGAUGCUOGUUCUUU | 65 |
| | siRNA-3 | 512-530 | GGUGUUUCAUACGGUUAUU | 66 |
| *CHCHD3* | siRNA-1 | 546-566 | CACGAUGCAUUCUACAAAGAA | 67 |
| | | 1450-1470 | CUGGAAUAAUGUUUAUCAUUA | 68 |
| | siRNA-2 | 374-392 | CGAAGAUCAGAAACGACUA | 69 |
| | siRNA-3 | 522-540 | GAGAAAGAOOGAGUGCUAA | 70 |
| *LSM3* | siRNA-1 | 540-560 | UOCAAUAAAUAUGACCACCAA | 71 |
| | | 37-57 | ACGACGUAGACCAGCAACAAA | 72 |
| | siRNA-2 | 39-57 | GACGUAGACCAGCAACAAA | 73 |
| | siRNA-3 | 261-279 | ACGAAACGGAAUAUUOCAA | 74 |
| *GTSE1* | siRNA-2 | 1082-1100 | GGGCAAAGCUAAAUCAAGU | 75 |
| | siRNA-3 | 2116-2134 | UGACAAACACUOCAGACAU | 76 |

### (2) Culture of human cancer cell strains

The three kinds of cancer cell strains (HeLa: uterine cervix cancer, KYSE150: esophagus cancer, and HCC50: colon cancer) were cultured under the following conditions. HeLa was cultured in a DMEM culture medium (Nacalai Tesque Inc.) containing 10% fetal bovine serum (Sigma Corporation) and gentamycin (Sigma Corporation). KYSE150 and HCC50 were cultured in RPMI 1640 culture media (Nacalai Tesque Inc.) containing 10% Fetal Bovine Serum and gentamycin.

### (3) Gene introduction of immortalization-determining gene sequence specific siRNA into cancer cell strain

On the day before the transfection, cancer cells previously cultured were isolated using trypsin. After being suspended in the culture medium detailed in (2) above, HeLa =1.2 × 10⁴, KYSE150 = 2 × 10⁴, and HCC50 = 1.5 × 10⁴ were seeded for each well of a 24-well plate. On the day of transfection, the media were replaced with serum-free media Opti-MEM (Invitrogen), and the siRNA shown in Table 2 and 3 were subjected to transfection using Oligofectamine (Invitrogen) or Lipofectamine 2000 (Invitrogen). The concentration of siRNA upon transfection was 40 nM per well. A control (non-silencing) siRNA (Qiagen) was used as a negative control.

### (4) Assay of the suppression of relative mRNA expression of immortalization-determining gene

One day after the transfection detailed in (3), total RNA was extracted from each cancer cell strain using RNeasy Mini (Qiagen) according to the instruction book. Using a QuantiTect probe RT-PCR (Qiagen) and an immortalization-determining gene sequence specific TaqMan probe (ABI), quantitative RT-PCR (ABI PRISM 7000 sequence detection system (produced by ABI) was used) was performed under the following conditions.
i) 50°C, 30 minutes,
ii) 95°C, 15 minutes,
iii) 94°C, 15 seconds → 60°C, 1 minutes x 35 to 45 cycles.

Using β-actin as an internal standard, the expression amount was digitalized based on each amplification curve. As a control, the value of a cell into which NS-sequence siRNA was introduced was used. Measurement of the relative mRNA expression of the immortalization-determining gene was performed twice with the same target gene and the same cancer cell strains under the same conditions.

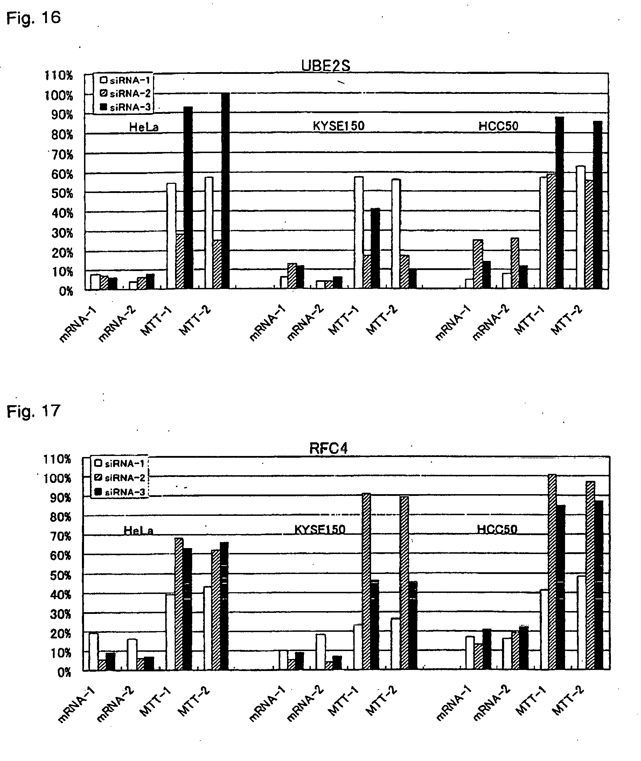

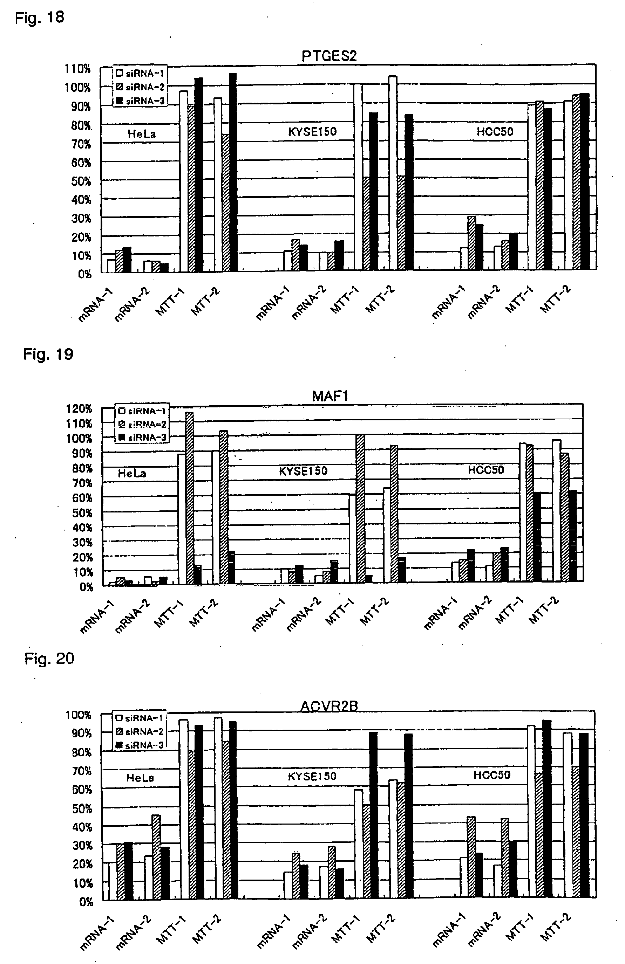

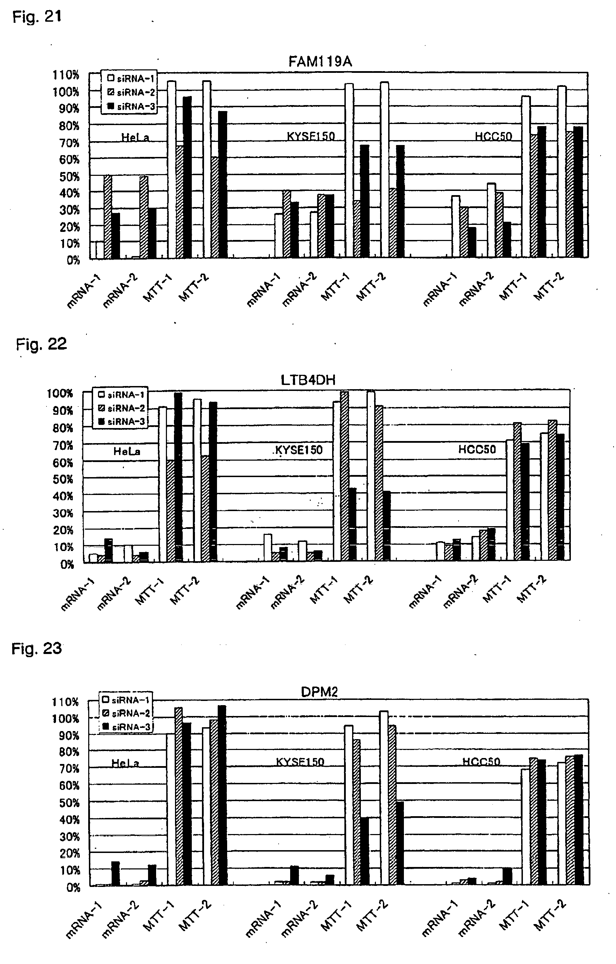

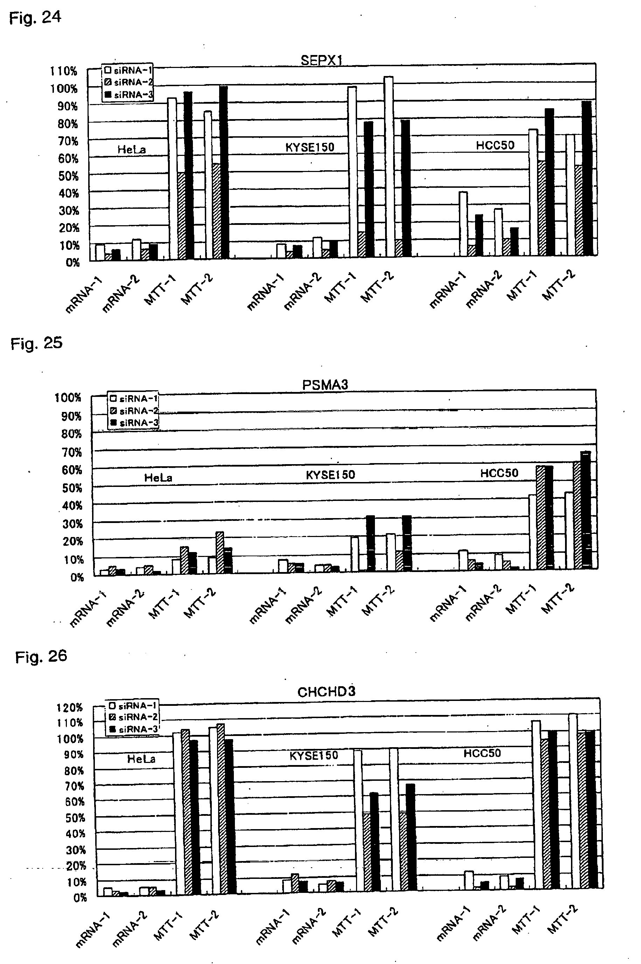

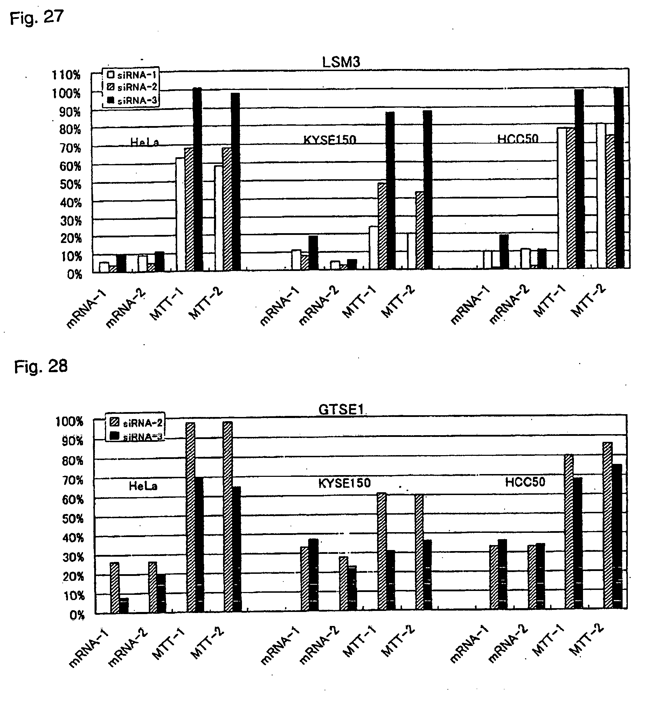

FIGS. 16 to 28 show the percentages of relative mRNA amounts of immortalization-determining genes in the cells to which each of siRNAs was introduced, when the immortalization-determining gene mRNA percentage in the control is assumed to be 100%. The measurements are shown as mRNA-1 and mRNA-2, respectively, in FIGS. 16 to 28. The suppression of mRNA expression by any one of siRNAs (siRNA-1 to siRNA-3) was observed in all target genes.

### (5) Measurement of the proliferation of cancer cell strains

The proliferation of cancer cell strains was measured using viable cell count measurement reagent SF (Nacalai Tesque Inc.). At 96 hours after the transfection detailed in (3), 10 µl of WST reagent was added to each well. After 3-hour incubation at 37°C, absorbencies at 450 nm and 595 nm (reference wavelengths) were measured using a plate reader (Wallac ARVO MX1420 Multilabel Counter: produced by Perkin Elmer). According to: absorbency at 450 nm - absorbency at 595 nm - absorbency at BG (only culture medium), the data was digitalized, and a mean value of three wells was found. Then, the value was shown as a percentage when the percentage of NS sequence siRNA introduction cell is assumed to be 100%. Measurement of proliferation of cancer cell strains was performed twice with the same target gene and same cancer cell strains under the same conditions.

The respective cell counts upon siRNA introduction are shown as MTT-1 and MTT-2, respectively, in FIGS. 16 to 28, when the percentage of cell count upon the NS sequence siRNA introduction is assumed to be 100%. In all target genes, the suppression of proliferation by any one of the siRNAs (siRNA-1 to siRNA-3) was seen in three kinds of cancer cells, indicating that those thirteen kinds of immortalization-determining genes can be regarded as being target molecules to attain universal suppression of immortalized cancer cell proliferation.

The discrimination of immortalized cancer cells from mortal cancer cells has significance in functioning as a malum marker, as well as in contributing to remedy development. General cancer diagnosis is based on pathological findings, with which the diagnosis of differentiation, atypism, etc., is also possible. However, general pathologic assays do not allow judgment as to whether the cells are immortalized, in other words, whether the cells have infinite lives. Human telomerase activation has attracted attention to date as an immortalization marker. However, as shown above, telomerase activation has also been seen in some healthy cells; therefore, the new idea that telomerase activation does not always immediately immortalize cells has become knowledge. Further, since the telomerase expression amount is very low, even though immune system staining was established, only a few laboratories have reported their achievements (Hiyama E et al., Neoplasia 3: 17-26, 2001). The use of the marker of the present invention to detect the expression of an immortalization-determining gene allows immortalized cancer cells to be discriminated from finite life cells. Such discrimination was not possible by general conventional pathological diagnosis. The present invention thus has great potential as a clinical application useful for early diagnosis, new treatment options, prognostification, and the like.
FIG. 1 is a schematic view illustrating a mechanism of human cell canceration and immortalization.
FIG. 2 is a schematic view illustrating a target gene extraction process according to the present invention.
FIG. 3 is a graph showing expression levels of UBE2S gene in the various immortalized cancer cells, using an oligo array.
FIG. 4 is a graph showing expression levels of RFC4 gene in the various immortalized cancer cells, using an oligo array.
FIG. 5 is a graph showing expression levels of PTGES2 gene in the various immortalized cancer cells, using an oligo array.
FIG. 6 is a graph showing expression levels of MAF1 gene in the various immortalized cancer cells, using an oligo array.
FIG. 7 is a graph showing expression levels of ACVR2B gene in the various immortalized cancer cells, using an oligo array.
FIG. 8 is a graph showing expression levels of FAM119A gene in the various immortalized cancer cells, using an oligo array.
FIG. 9 is a graph showing expression levels of LTB4DH gene in the various immortalized cancer cells, using an oligo array.
FIG. 10 is a graph showing expression levels of DPM2 gene in the various immortalized cancer cells, using an oligo array.
FIG. 11 is a graph showing expression levels of SEPX1 gene in the various immortalized cancer cells, using an oligo array.
FIG. 12 is a graph showing expression levels of PSMA3 gene in the various immortalized cancer cells, using an oligo array.
FIG. 13 is a graph showing expression levels of CHCHD3 gene in the various immortalized cancer cells, using an oligo array.
FIG. 14 is a graph showing expression levels of LSM3 gene in the various immortalized cancer cells, using an oligo array.
FIG. 15 is a graph showing expression levels of GTSE1 gene in the various immortalized cancer cells, using an oligo array.
FIG. 16 is a graph showing an effect of expression suppression of UBE2S gene and suppression of cell proliferation given by siRNA in cancer cell strains (Example 2).
FIG. 17 is a graph showing an effect of expression suppression of RFC4 gene and suppression of cell proliferation given by siRNA in cancer cell strains (Example 2).
FIG. 18 is a graph showing an effect of expression suppression of PTGES2 gene and suppression of cell proliferation given by siRNA in cancer cell strains (Example 2).
FIG. 19 is a graph showing an effect of expression suppression of MAF1 gene and suppression of cell proliferation given by siRNA in cancer cell strains (Example 2).
FIG. 20 is a graph showing an effect of expression suppression of ACVR2B gene and suppression of cell proliferation given by siRNA in cancer cell strains (Example 2).
FIG. 21 is a graph showing an effect of expression suppression of FAM119A gene and suppression of cell proliferation given by siRNA in cancer cell strains (Example 2).
FIG. 22 is a graph showing an effect of expression suppression of LTB4DH gene and suppression of cell proliferation given by siRNA in cancer cell strains (Example 2).
FIG. 23 is a graph showing an effect of expression suppression of DPM2 gene and suppression of cell proliferation given by siRNA in cancer cell strains (Example 2).
FIG. 24 is a graph showing an effect of expression suppression of SEPX1 gene and suppression of cell proliferation given by siRNA in cancer cell strains (Example 2).
FIG. 25 is a graph showing an effect of expression suppression of PSMA3 gene and suppression of cell proliferation given by siRNA in cancer cell strains (Example 2).
FIG. 26 is a graph showing an effect of expression suppression of CHCHD3 gene and suppression of cell proliferation given by siRNA in cancer cell strains (Example 2).
FIG. 27 is a graph showing an effect of expression suppression of LSM3 gene and suppression of cell proliferation given by siRNA in cancer cell strains (Example 2).
FIG. 28 is a graph showing an effect of expression suppression of GTSE1 gene and suppression of cell proliferation given by siRNA in cancer cell strains (Example 2).

### SEQUENCE LISTING FREE TEXT

SEQ ID No.27 represents the base sequence of the sense strand of siRNA-1 (44-64), of the UBE2S gene.
SEQ ID No.28 represents the base sequence of the sense strand of siRNA-1 (146-166), of the UBE2S gene.
SEQ ID No.29 represents the base sequence of the sense strand of siRNA-2, of the UBE2S gene.
SEQ ID No.30 represents the base sequence of the sense strand of siRNA-3, of the UBE2S gene.
SEQ ID No.31 represents the base sequence of the sense strand of siRNA-1 (897-917), of the RFC4 gene.
SEQ.ID No.32 represents the base sequence of the sense strand of siRNA-1 (189-209), of the RFC4 gene.
SEQ ID No.33 represents the base sequence of the sense strand of siRNA-2, of the the RFC4 gene.
SEQ ID No.34 represents the base sequence of the sense strand of siRNA-3, of the RFC4 gene.
SEQ ID No.35 represents the base sequence of the sense strand of siRNA-1 (2003-2023), of the PTGES2 gene.
SEQ ID No.36 represents the base sequence of the sense strand of siRNA-1 (1105-1125), of the PTGES2 gene.
SEQ ID No.37 represents the base sequence of the sense strand of siRNA-2, of the PTGES2 gene.
SEQ ID No.38 represents the base sequence of the sense strand of siRNA-3, of the PTGES2 gene.
SEQ ID No.39 represents the base sequence of the sense strand of siRNA-1 (1538-1558), of the MAF1 gene.
SEQ ID No.40 represents the base sequence of the sense strand of siRNA-1 (1031-1051), of the MAF1 gene.
SEQ ID No.41 represents the base sequence of the sense strand of siRNA-2, of MAF1 gene.
SEQ ID No.42 represents the base sequence of the sense strand of siRNA-3, of the MAF1 gene.
SEQ ID No.43 represents the base sequence of the sense strand of siRNA-1 (626-646), of the ACVR2B gene.
SEQ ID No.44 represents the base sequence of the sense strand of siRNA-1 (208-228), of the ACVR2B gene.
SEQ ID No.45 represents the base sequence of the sense strand of siRNA-2, of the ACVR2B gene.
SEQ ID No.46 represents the base sequence of the sense strand of siRNA-3, of the ACVR2B gene.
SEQ ID No.47 represents the base sequence of the sense strand of siRNA-1(754-774), of the FAM119A gene.
SEQ ID No.48 represents the base sequence of the sense strand of siRNA-1(1068-1088), of the FAM119A gene.
SEQ ID No.49 represents the base sequence of the sense strand of siRNA-2, of the FAM119A gene.
SEQ ID No.50 represents the base sequence of the sense strand of siRNA-3, of the FAM119A gene.
SEQ ID No.51 represents the base sequence of the sense strand of siRNA-1(775-795), of the LTB4DH gene.
SEQ ID No.52 represents the base sequence of the sense strand of siRNA-1(658-678), of the LTB4DH gene.
SEQ ID No.53 represents the base sequence of the sense strand of siRNA-2, of the LTB4DH gene.
SEQ ID No.54 represents the base sequence of the sense strand of siRNA-3, of the LTB4DH gene.
SEQ ID No.55 represents the base sequence of the sense strand of siRNA-1(145-165), of the DPM2 gene.
SEQ ID No.56 represents the base sequence of the sense strand of siRNA-1(84-104), of the DPM2 gene.
SEQ ID No.57 represents the base sequence of the sense strand of siRNA-2, of the DPM2 gene.
SEQ ID No.58 represents the base sequence of the sense strand of siRNA-3, of the DPM2 gene.
SEQ ID No.59 represents the base sequence of the sense strand of siRNA-1(870-890), of the SEPX1 gene.
SEQ ID No.60 represents the base sequence of the sense strand of siRNA-1(794-814), of the SEPX1 gene.
SEQ ID No.61 represents the base sequence of the sense strand of siRNA-2, of the SEPX1 gene.
SEQ ID No.62 represents the base sequence of the sense strand of siRNA-3, of the SEPX1 gene.
SEQ ID No.63 represents the base sequence of the sense strand of siRNA-1(853-873), of the PSMA3 gene.
SEQ ID No.64 represents the base sequence of the sense strand of siRNA-1(686-706), of the PSMA3 gene.
SEQ ID No.65 represents the base sequence of the sense strand of siRNA-2, of the PSMA3 gene.
SEQ ID No.66 represents the base sequence of the sense strand of siRNA-3, of the PSMA3 gene.
SEQ ID No.67 represents the base sequence of the sense strand of siRNA-1(546-566), of the CHCHD3 gene.
SEQ ID No.68 represents the base sequence of the sense strand of siRNA-1(1450-1470), of the CHCHD3 gene.
SEQ ID No.69 represents the base sequence of the sense strand of siRNA-2, of the CHCHD3 gene.
SEQ ID No.70 represents the base sequence of the sense strand of siRNA-3, of the CHCHD3 gene.
SEQ ID No.71 represents the base sequence of the sense strand of siRNA-1(540-560), of the LSM3 gene.
SEQ ID No.72 represents the base sequence of the sense strand of siRNA-1 (37-57), of the LSM3 gene.
SEQ ID No.73 represents the base sequence of the sense strand of siRNA-2, of the LSM3 gene.
SEQ ID No.74 represents the base sequence of the sense strand of siRNA-3, of the LSM3 gene.
SEQ ID No.75 represents the base sequence of the sense strand of siRNA-2, of the GTSE1 gene.
SEQ ID No.76 represents the base sequence of the sense strand of siRNA-3, of the GTSE1 gene.

## Claims

1. Use of a polynucleotide as determinant of an immortalized cancer cell in cancer cells obtained from a test subject, the polynucleotide comprising a polynucleotide having a base sequence of at least 15 bases that specifically hybridizes with a continuous base sequence of at least 15 bases within the base sequence represented by SEQ ID NO. 13.

2. The use according to claim 1, wherein the polynucleotide is a probe or primer.

3. A method for determining an immortalized cancer cell in cancer cells comprising the steps of:
(1) bonding the polynucleotide according to claim 1 or 2 with RNA prepared from a biological sample that is extracted from a test subject and that contains a cancer cell, or a derivative of the RNA;
(2) measuring an amount of the RNA or the derivative thereof bonded with the polynucleotide, using the polynucleotide as an index; and
(3) comparing the amount of RNA or the derivative thereof found in Step (2) (generically referred to as "an RNA amount", hereinafter) with an amount (generically referred to as "a comparative RNA amount", hereinafter) of a corresponding RNA or a derivative thereof in a non-immortalized cancer cell.

4. The method for determining an immortalized cancer cell in cancer cells according to claim 3, further comprising the step of:
(4) determining that the cancer cell of the test subject is immortalized when the RNA amount found in Step (2) is higher than the comparative RNA amount, and determining that the cancer cell of the test subject is not immortalized when the RNA amount found in Step (2) is not higher than the comparative RNA amount.

5. A method for determining an immortalized cancer cell in cancer cells, comprising the steps of:
(1') bonding a protein-containing fraction containing a polypeptide prepared from a biological sample that is extracted from a test subject and that contains a cancer cell with an antibody for recognizing a polypeptide having the amino acid sequence represented by SEQ ID NO: 26;
(2') measuring the amount of the polypeptide that is bonded with the antibody, using the antibody as an index; and
(3') comparing the amount (generically referred to as "a polypeptide amount", hereinafter) of the polypeptide found in Step (2') with the amount (generically referred to as "a comparative polypeptide amount", hereinafter) of a corresponding polypeptide in a non-immortalized cancer cell.

6. The method for determining an immortalized cancer cell in cancer cells according to claim 5, further comprising the step of:
(4') determining that the cancer cell of the test subject is immortalized when the polypeptide amount found in Step (2') is higher than the comparative polypeptide amount, and determining that the cancer cell of the test subject is not immortalized when the polypeptide amount found in Step (2') is not higher than the comparative polypeptide amount.

7. Use of a reagent kit as determinant of an immortalized cancer cell in cancer cells obtained from a test subject, the reagent kit including at least one member selected from the group consisting of the polynucleotide according to claim 1 and an antibody for recognizing a polypeptide having the amino acid sequence represented by SEQ ID NO: 26.

## Patentansprüche

1. Verwendung eines Polynukleotids als Bestimmungsgröße einer immortalisierten Krebszelle in Krebszellen, die von einem Testsubjekt erhalten wurden, wobei das Polynukleotid ein Polynukleotid umfaßt, das eine Basensequenz von mindestens 15 Basen aufweist, welche spezifisch mit einer kontinuierlichen Basensequenz von mindestens 15 Basen innerhalb der durch. SEQ ID NO: 13 dargestellten Basensequenz hybridisiert.

2. Verwendung nach Anspruch 1, wobei das Polynukleotid eine Sonde oder ein Primer ist.

3. Verfahren zum Bestimmen einer immortalisierten Krebszelle in Krebszellen, umfassend die Schritte:
(1) Binden des Polynukleotids nach Anspruch 1 oder 2 mit RNA, die aus einer biologischen Probe präpariert wurde, welche von einem Testsubjekt entnommen wurde und eine Krebszelle enthält, oder mit einem Derivat der RNA,
(2) Messen einer Menge der RNA oder des Derivats davon, welche(s) mit dem Polynukleotid gebunden ist, unter Verwendung des Polynukleotids als eine Kenngröße, und
(3) Vergleichen der in Schritt (2) gefundenen Menge der RNA oder des Derivats davon (nachstehend allgemein bezeichnet als "eine RNA-Menge") mit einer Menge (nachstehend allgemein bezeichnet als "Vergleichs-RNA-Menge") einer entsprechenden RNA oder eines Derivats davon in einer nicht-immortalisierten Krebszelle.

4. Verfahren zum Bestimmen einer immortalisierten Krebszelle in Krebszellen nach Anspruch 3, weiter umfassend den Schritt:
(4) Bestimmen, daß die Krebszelle des Testsubjekts immortalisiert ist, wenn die in Schritt (2) gefundene RNA-Menge größer ist als die Vergleichs-RNA-Menge, und Bestimmen, daß die Krebszelle des Testsubjekts nicht immortalisiert ist, wenn die in Schritt (2) gefundene RNA-Menge nicht größer ist als die Vergleichs-RNA-Menge.

5. Verfahren zum Bestimmen einer immortalisierten Krebszelle in Krebszellen, umfassend die Schritte:
(1') Binden einer Protein-enthaltenden Fraktion, enthaltend ein Polypeptid, das aus einer biologischen Probe präpariert wurde, welche von einem Testsubjekt entnommen wurde und eine Krebszelle enthält, mit einem Antikörper zur Erkennung eines Polypeptids, das die in SEQ ID NO: 26 dargestellte Aminosäuresequenz aufweist,
(2') Messen der Menge des Polypeptids, das mit dem Antikörper gebunden ist, unter Verwendung des Antikörpers als eine Kenngröße, und
(3') Vergleichen der Menge (nachstehend allgemein bezeichnet als "Polypeptidmenge") des in Schritt (2') gefundenen Polypeptids mit der Menge (nachstehend allgemein bezeichnet als "Vergleichs-Polypeptidmenge") eines entsprechenden Polypeptids in einer nichtimmortalisierten Krebszelle.

6. Verfahren zum Bestimmen einer immortalisierten Krebszelle in Krebszellen nach Anspruch 5, weiter umfassend den Schritt:
(4') Bestimmen, daß die Krebszelle des Testsubjekts immortalisiert ist, wenn die in Schritt (2) gefundene Polypeptidmenge größer ist als die Vergleichs-Polypeptidmenge, und Bestimmen, daß die Krebszelle des Testsubjekts nicht immortalisiert ist, wenn die in Schritt (2) gefundene Polypeptidmenge nicht größer ist als die Vergleichs-Polypeptidmenge.

7. Verwendung eines Reagenzienkits als Bestimmungsgröße einer immortalisierten Krebszelle in Krebszellen, die von einem Testsubjekt erhalten wurden, wobei das Reagenzienkit mindestens ein Mitglied enthält, ausgewählt aus der Gruppe, bestehend aus dem Polynukleotid nach Anspruch 1 und einem Antikörper zur Erkennung eines Polypeptids, das die in SEQ ID NO: 26 dargestellte Aminosäuresequenz aufweist.

## Revendications

1. Utilisation d'un polynucléotide en tant que déterminant d'une cellule cancéreuse immortalisée dans des cellules cancéreuses obtenues d'un sujet à tester, le polynucléotide comprenant un polynucléotide ayant une séquence de bases d'au moins 15 bases qui s'hybride spécifiquement à une séquence de bases continues d'au moins 15 bases au sein de la séquence de bases représentée par SEQ ID NO : 13.

2. Utilisation selon la revendication 1, où le polynucléotide est une sonde ou une amorce.

3. Procédé de détermination d'une cellule cancéreuse immortalisée dans des cellules cancéreuses, comprenant les étapes suivantes :
(1) la liaison du polynucléotide selon la revendication 1 ou 2, avec de l'ARN préparé à partir d'un échantillon biologique qui est extrait d'un sujet à tester et qui contient une cellule cancéreuse ou un dérivé de l'ARN ;
(2) la mesure d'une quantité de l'ARN ou du dérivé de celui-ci lié au polynucléotide, en utilisant le polynucléotide comme index ; et
(3) la comparaison de la quantité d'ARN ou du dérivé de celui-ci trouvée dans l'étape (2) (appelée de manière générique « une quantité d'ARN », ci-après dans ce document) à une quantité (appelée de manière générique « une quantité d'ARN comparative », ci-après dans ce document) d'un ARN correspondant ou d'un dérivé de celui-ci dans une cellule cancéreuse non immortalisée .

4. Procédé de détermination d'une cellule cancéreuse immortalisée dans des cellules cancéreuses selon la revendication 3, comprenant en outre l'étape suivante :
(4) la détermination que la cellule cancéreuse du sujet à tester est immortalisée lorsque la quantité d'ARN trouvée dans l'étape (2) est supérieure à la quantité d'ARN comparative, et la détermination que la cellule cancéreuse du sujet à tester n'est pas immortalisée lorsque la quantité d'ARN trouvée dans l'étape (2) n'est pas supérieure à la quantité d'ARN comparative.

5. Procédé de détermination d'une cellule cancéreuse immortalisée dans des cellules cancéreuses, comprenant les étapes suivantes :
(1') la liaison d'une fraction contenant une protéine contenant un polypeptide préparée à partir d'un échantillon biologique qui est extrait d'un sujet à tester et qui contient une cellule cancéreuse à un anticorps pour reconnaître un polypeptide ayant la séquence d'acides aminés représentée par SEQ ID NO : 26 ;
(2') la mesure de la quantité du polypeptide qui est lié à l'anticorps, en utilisant l'anticorps comme index ; et
(3') la comparaison de la quantité (appelée de manière générique « une quantité de polypeptide », ci-après dans ce document) du polypeptide trouvée dans l'étape (2') à la quantité (appelée de manière générique « une quantité de polypeptide comparative », ci-après dans ce document) d'un polypeptide correspondant dans une cellule cancéreuse non immortalisée.

6. Procédé de détermination d'une cellule cancéreuse immortalisée dans des cellules cancéreuses selon la revendication 5, comprenant en outre l'étape suivante :
(4') la détermination que la cellule cancéreuse du sujet à tester est immortalisée lorsque la quantité de polypeptide trouvée dans l'étape (2') est supérieure à la quantité de polypeptide comparative, et la détermination que la cellule cancéreuse du sujet à tester n'est pas immortalisée lorsque la quantité de polypeptide trouvée dans l'étape (2') n'est pas supérieure à la quantité de polypeptide comparative.

7. Utilisation d'un kit de réactifs en tant que déterminant d'une cellule cancéreuse immortalisée dans des cellules cancéreuses obtenues d'un sujet à tester, le kit de réactifs comprenant au moins un membre choisi dans le groupe constitué du polynucléotide selon la revendication 1 et d'un anticorps pour reconnaître un polypeptide ayant la séquence d'acides aminés représentée par SEQ ID NO : 26.
